# EUROPEAN PATENT APPLICATION

(11) **EP 4 371 575 A1**
(43) Date of publication of application: **22.05.2024**
(21) Application number: 23020497.6
(22) Date of filing: 08.11.2023
(51) Int. Cl.: A61K 41/00

(54) **COMBINING SPATIAL AND TEMPORAL SEQUENCES OF IRRADIATION USING A SPECIFIC NANO-SYSTEM FOR AN IMPROVED TREATMENT OF DISEASES SUCH AS CANCER**

(30) Priority: 15.11.2022 EP 22020561
(71) Applicant: Nanobacterie, 75116 Paris (FR)
(72) Inventor: Alphandery, Edouard, 75116 PARIS (FR); Chebbi, Imene, 91140 Villebon Sur Yvette (FR)

(57) **Abstract**

The invention relates to nanoparticle for use in a method, for increasing the production of free radicals or amplifying radiation during a sonodynamic, photodynamic or radiation therapy or exposure of a body part to a radiation, comprising the steps of:
1) Preferentially introducing or reintroducing in a body part to be treated at least one nanoparticle comprising:
- Preferentially a first center of a first center activity, C_{A1}, such as a first center of free radical production or radiation amplification, C_{1FRP}, comprised in its core,
and
- Preferentially a second center of activity, C_{A2}, such as a second center of free radical production or radiation amplification, C_{2FRP}, comprised in its coating,

2) Preferentially applying an external radiation on the body part comprising the nanoparticle(s) during a time t₁;
3) Preferentially not applying the external radiation on the body part comprising the nanoparticle(s) during a time t₂ or applying a radiation of lower intensity, energy, power or power density during the time t₂ than during the time t₁;
4) Preferentially re-applying the external radiation on the body part comprising the nanoparticle(s) during a time t₃ or applying an external radiation of larger intensity, energy, power or power density during t₃ than during t₂;

## Description

### FIELD OF THE INVENTION

The field of the invention is that of a pathological body part, for example a tumor, comprising nanoparticle or at least one constituent of a composition comprising nanoparticles preferentially with at least two centers of activity or free radical production in the core and coating of the nanoparticle or at least one constituent of the compositions, respectively, which are preferentially irradiated preferentially resulting in an improved treatment efficacy compared with standard irradiation conditions performed in the absence of such composition.

### TECHNICAL BACKGROUND

Nanoparticle or at least one constituent of the compositions can be associated with a photosensitizing molecule, for example Protoporphyrin IX was associated with gold nanoparticle or at least one constituent of the compositions to yield efficient photosensitizing effect in cervical cancer therapy, (H. Eshghi et al, Photodiagnosis and Photodynamic Therapy, V. 10, P. 304-312 (2013)). Here, we introduce the new feature of preferentially inserting at least two centers activity or of free radical production or radiation amplification, where preferentially one of these centers is inserted in the core of the nanoparticle or at least one constituent of the composition and the other center is inserted in the coating of the nanoparticle or at least one constituent of the composition. Such configuration preferentially presents the advantage of increasing the quantity of free radicals that is produced or of amplifying the radiation compared with a nanoparticle or at least one constituent of the composition comprising only one center of activity or free radical production or radiation amplification. The presence of two centers located in two different parts of the nanoparticle or at least one constituent of the composition preferentially enables applying the treatment through a spatial sequence, *i.e.* with free radical production or radiation amplification occurring at two different locations, where these two locations are preferentially separated by a nanometric distance or 1 or 5 or 10 nm, hence potentially enabling to reach a large concentration of these centers in the treated body part, hence potentially enabling to achieve a synergic effect of these two centers. The arrangement in chains of the nanoparticles or at least one constituent of the composition can also be a way to increase the number of centers activity or of free radical production or radiation amplification in the treated body part, given that the available surface into which the centers can be introduced is enhanced both by the presence of the nanoparticle or at least one constituent of the composition with a high surface/volume ratio and of the chains with an increased external surface /coating available compared with a single nanoparticle or at least one constituent of the composition. In addition to this spatial sequence or arrangement or assembly, we propose to preferentially use a temporal sequence, *i.e.* to apply an external radiation to the composition or nano-system in an intermittent or sequential manner in order to reduce potential side effects associated with too long and/or too intense continuous radiation application.

### DESCRIPTION OF THE INVENTION

The invention relates to nanoparticle or at least one constituent of a composition for use in a method, preferentially method A, for at least one of the following purposes: i) increasing the production of free radicals ii) amplifying radiation, iii) amplifying the effect of radiation, iv) increasing the destruction of the body part, preferentially pathological or tumor cells or a tumor or cancer or virus or a pathological part of the body part, v) destroying or inactivating at least 1, 1.1, 2, 5 or 10 times more pathological or tumor cells or virus preferentially when the pathological cell or pathological body part is exposed to radiation in the presence of the composition than when the pathological cell or pathological body part is exposed to radiation in the absence of the composition, vi) reducing side effects of radiation or preserving the body part, preferentially non-pathological or healthy cells or healthy body part, preferentially surrounding the pathological body part, vii) preserving or maintaining activated or alive at least 1, 1.1, 2, 5 or 10 times more healthy cells preferentially when the healthy cells are exposed to radiation or subjected to an indirect such as an immune reaction that occurs after the application of radiation on the body part in the presence of the composition than when the healthy cells are exposed to radiation or subjected to an indirect such as an immune reaction that occurs after the application of radiation on the body part in the presence of the composition in the absence of the composition, viii) sonodynamic therapy, ix) photodynamic therapy, x) radiation therapy, xi) a diagnosis, and x) a treatment,
wherein the method preferentially comprises at least one step of:
   1) Preferentially introducing or reintroducing in a body part to be treated at least one nanoparticle or at least one constituent of the composition comprising a first center of activity or free radical production or radiation amplification, C_{1FRP}, in its core, and a second center of activity or free radical production or radiation amplification, C_{2FRP}, in its coating;
   2) Preferentially applying an external radiation on the body part comprising the nanoparticle or at least one constituent of the composition(s) during a time t₁;
   3) Preferentially not applying the external radiation on the body part comprising the nanoparticle or at least one constituent of the composition(s) during a time t₂ or applying a radiation of lower intensity, energy, power or power density during the time t₂ than during the time t₁;
   4) Preferentially re-applying the external radiation on the body part comprising the nanoparticle or at least one constituent of the composition(s) during a time t₃ or applying an external radiation of larger intensity, energy, power or power density during t₃ than during t₂;
wherein C_{1FRP} and C_{2FRP} are preferentially two different compounds C and D preferentially selected in the group consisting of a photo-sensitizer, a sono-sensitizer, and a radio-sensitizer.

In one embodiment of the invention, at least one method according to the invention comprises at least 1, 2, 3 or 4 step(s), which is repeated at least 1, 2, 3, 4, 5 or 10 time(s).

In another embodiment of the invention, at least one method according to the invention comprises at least 1, 2, 3 or 4 step(s), which is repeated less than 10, 5, 2 or 1 time(s).

In one embodiment of the invention, during step 2, the quantity of free radicals produced by the radiation is preferentially increased by compounds C and D combined or by compound D alone, wherein during step 3, compound D is preferentially detaching or disassembling from the coating of the nanoparticle or at least one constituent of the composition and is either destroyed/inactivated or diffuses away from the treated body part, wherein during step 4, the quantity of free radicals produced by the radiation is preferentially increased by compound C alone.

In some cases, free radicals can be oxygen or nitrogen species.

The invention also relates to a nanoparticle or at least one constituent of the composition for use in a method, preferentially method B, for at least one of the following purposes: i) decreasing the production of free radicals, ii) reducing radiation, iii) reducing the effect of radiation, iv) increasing the destruction of the body part, preferentially pathological or tumor cells or a tumor or cancer or virus or a pathological part of the body part, v) destroying or inactivating at least 1, 1.1, 2, 5 or 10 times more pathological or tumor cells or virus preferentially when the pathological cell or pathological body part is exposed to radiation in the presence of the composition than when the pathological cell or pathological body part is exposed to radiation in the absence of the composition, vi) reducing side effects of radiation or preserving the body part, preferentially non-pathological or healthy cells or healthy body part, preferentially surrounding the pathological body part, vii) preserving or maintaining activated or alive at least 1, 1.1, 2, 5 or 10 times more healthy cells preferentially when the healthy cells are exposed to radiation or subjected to an indirect such as an immune reaction that occurs after the application of radiation on the body part in the presence of the composition than when the healthy cells are exposed to radiation or subjected to an indirect such as an immune reaction that occurs after the application of radiation on the body part in the presence of the composition in the absence of the composition, viii) sonodynamic therapy, ix) photodynamic therapy, x) radiation therapy, xi) a diagnosis, and x) a treatment,
wherein the method preferentially comprises at least one step of:
   1') Preferentially introducing in a body part to be treated at least one nanoparticle or at least one constituent of the composition comprising a first center of activity or free radical capture C_{1FRC} in its core, and a second center of free capture C_{2FRC} in its coating (Step 1'),
   2') Preferentially applying a radiation on the body part comprising the nanoparticle or at least one constituent of the composition(s) during a time t₄ (Step 2');
   3') Preferentially not applying the radiation on the body part comprising the nanoparticle or at least one constituent of the composition(s) during a time t₅ or applying a radiation of lower intensity, energy, power or power density during the time t₅ than during the time t₄ (Step 3');
   4') Preferentially re-applying the radiation on the body part comprising the nanoparticle or at least one constituent of the composition(s) during a time t₆ or applying a radiation of larger intensity, energy, power or power density during t₆ than during t₅ (Step 4');
wherein C_{1FRC} and C_{2FRC} are preferentially two different anti-oxidant compounds E and F.

In one embodiment of the invention, during step 2', the quantity of free radicals or oxygen or nitrogen species produced by the radiation is preferentially decreased by compounds E and F combined or by compound F alone, wherein during step 3', compound F is preferentially detaching or disassembling from the coating of the nanoparticle or at least one constituent of the composition and is either destroyed/inactivated or diffuses away from the treated body part, wherein during step 4', the quantity of free radicals or oxygen or nitrogen species produced by the radiation is preferentially decreased by compound E alone.

The invention also relates to nanoparticle or at least one constituent of the composition for sequentially applying radiation on a body part of an individual, comprising a spatial sequence I and/or a temporal sequence II,
wherein the spatial sequence I preferentially consists in applying the radiation on two different centers of activity or free radical production, C_{1FRP} and C_{2FRP},
wherein C_{1FRP} and C_{2FRP} are preferentially comprised in the core and coating of the nanoparticle or at least one constituent of the composition, respectively,
wherein C_{1FRP} and C_{2FRR} are preferentially spatially separated by more than 0.1 nm,
wherein the temporal sequence II preferentially consists in at least one of the following steps:
   1) Preferentially introducing in a body part to be treated at least one nanoparticle or at least one constituent of the composition comprising a first center of activity or free radical production C_{1FRP} in its core and a second center of activity or free radical production C_{2FRR} in its coating, (Step 1);
   2) Preferentially applying for a first time a radiation on the body part comprising the nanoparticle or at least one constituent of the composition(s) during a time t₁ (Step 2);
   3) Preferentially not applying the radiation on the body part comprising the nanoparticle or at least one constituent of the composition(s) during a time t₂ or applying a radiation of lower intensity, energy, power or power density during the time t₂ than during the time t₁ (Step 3);
   4) Preferentially re-applying for another time the radiation on the body part comprising the nanoparticle or at least one constituent of the composition(s) during a time t3 or applying a radiation of larger intensity, energy, power or power density during t₃ than during t₂ (Step 4);
wherein the method preferentially aims at increasing the quantity of free radicals produced by radiation preferentially during a limited time of exposure of a body part to radiation preferentially to avoid toxicity induced by a too long continuous exposure of a body part to radiation, preferentially when or after nanoparticles or at least one constituent of the composition is(are) introduced in a body part and the radiation is applied on the body part, preferentially sequentially.

The invention also relates to the nanoparticle or at least one constituent of the composition for sequentially applying radiation on a body part of an individual, comprising a spatial sequence III and/or a temporal sequence IV,
wherein the spatial sequence III preferentially consists in applying the radiation on two different centers of activity or free radical capture, C_{1FRC} and C_{2FRC},
wherein C_{1FRC} and C_{2FRC} are preferentially comprised in the core and coating of the nanoparticle or at least one constituent of the composition, respectively,
wherein C_{1FRC} and C_{2FRC} are preferentially spatially separated by more than 0.1, 1 or 10 nm,
wherein the temporal sequence IV preferentially consists in or comprises at least one of the following steps:
   1') preferentially introducing in a body part to be treated at least one nanoparticle or at least one constituent of the composition preferentially comprising a first center of activity or free radical capture C_{1FRC} in its core, and a second center of free capture C_{2FRC} in its coating(Step 1'),
   2') preferentially applying a radiation on the body part comprising the nanoparticle or at least one constituent of the composition(s) preferentially during a time 14(Step 2'),
   3') preferentially not applying the radiation on the body part preferentially comprising the nanoparticle or at least one constituent of the composition(s) preferentially during a time t₅ or applying a radiation of lower intensity, energy, power or power density during the time t₅ than during the time t₄ (Step 3'),
   4') preferentially re-applying the radiation on the body part comprising the nanoparticle or at least one constituent of the composition(s) preferentially during a time t₆ or applying a radiation of larger intensity, energy, power or power density during t₆ than during t₅ (Step 4'),
wherein the method preferentially aims at reducing the quantity of free radicals preferentially produced by radiation preferentially by using at least two one or centers free radical capture, preferentially C_{1FRC} and/or C_{2FRC}, and preferentially a limited time of exposure of a body part comprising the nanoparticle or at least one constituent of the composition to radiation.

In some cases, t₁, t₂, t₃, t₄, t₅, t₆, t₇ and/or t₈ can be larger than 10⁻¹⁰, 10⁻⁵, 10⁻³, 10⁻², 10 ', 0, 1, 5, 10 or 10³ second(s).

In some other cases, t₁, t₂, t₃, t₄, t₅, t₆, t₇ and/or t₈ can be smaller than 10¹⁰, 10⁵, 10³, 10², 10, 0 or 10⁻³ second(s).

The invention also relates to nanoparticle or at least one constituent of the compositions for use according to the invention, wherein C_{1FRP} and/or C_{2FRP} has/have at least one property selected in the group consisting of:
i) C_{1FRP} and C_{2FRR} are separated by more than 0.1, 5 or 10 nm; and
ii) C_{1FRP} and/or C_{2FRP} is/are comprised in the nanoparticle or at least one constituent of the composition(s) at a concentration ranging from 1 to 10⁴ C_{1FRP} and/or C_{2FRP} per nanoparticle or at least one constituent of the composition.

In some cases, C_{A1} can be C_{1FRP} or have at least one property in common with C_{1FRP}.

In some other cases, C_{A2} can be C_{2FRP} or have at least one property in common with C_{2FRP}.

In some cases, C_{1FRP} and C_{2FRP} can be separated by more than 0.1, 1, 5, 10 or 100 nm.

In some other cases, C_{1FRP} and C_{2FRR} can be separated by less than 10⁵, 100, 50, 10, 5, 2, 1 or 0.1 nm.

In some cases, C_{1FRP} and/or C_{2FRP} can be comprised in the nanoparticle or at least one constituent of the composition(s) at a concentration that is larger than 1, 5, 10, 10³ or 10⁵ C_{1FRP} and/or C_{2FRR} per nanoparticle or at least one constituent of the composition or per chain of nanoparticle or at least one constituent of the composition.

In some other cases, C_{1FRP} and/or C_{2FRR} can be comprised in the nanoparticle or at least one constituent of the composition(s) at a concentration that is smaller than 10⁵, 10³, 10, 5, 2 or 1 C_{1FRP} and/or C_{2FRP} per nanoparticle or at least one constituent of the composition or per chain of nanoparticle or at least one constituent of the composition.

The invention also relates to the nanoparticle or at least one constituent of the composition for use in the method according to the invention, wherein at least two steps among steps 1), 2), 3) and 4) follow each other preferentially in a specific or in any order, *i.e.* that preferentially: i) step 2 follows step 1), step 2) follows step 3), step 2) follows step 4), step 3) follows step 1), step 3) follows step 2), step 3) follows step 4), step 4) follows step 1), step 4) follows step 2), step 4) follows step 3), step 1) follows step 2), step 1) follows step 3), and/or step 1) follows step 4).

The invention also relates to nanoparticle or at least one constituent of the compositions for use in a method according to the invention, wherein the nanoparticle or at least one constituent of the composition comprises:
i) preferentially At least compound C and compound B, where compound C is another metal than iron such as Zinc and compound B is Oxygen, where the core is optionally composed of ZnO in this case,
ii) preferentially At least compound C, compound B, and compound A, where compound C is another metal than iron such as Zinc, compound B is Oxygen, and compound A is iron, where the core is optionally composed of ZnFe₂O₄ or ZnFeO₃ in this case, and/or
iii) preferentially At least compound C and/or compound D, wherein compound C and/or compound D is/are radioactive compound(s).

The invention also relates to nanoparticle or at least one constituent of the compositions for use in the method according to the invention, wherein at least two nanoparticle or at least one constituent of the compositions are organized in a chain.

The invention also relates to nanoparticle or at least one constituent of the compositions for use in the method according to the invention, which is characterized by at least one of the following properties:
i) t₂ is smaller than t₁ and/or t₃;
ii) the intensity, power, energy, or energy density of the radiation produced by C_{1FRP} and/or C_{2FRP} is smaller than the intensity, power, energy, or energy density of the radiation produced by the external radiation.

The invention also relates to nanoparticle or at least one constituent of the compositions for use in the method according to the invention, which is characterized by at least one of the following properties:
i) t₂ is larger than t₁ and/or t₃;
ii) the intensity, power, energy, or energy density of the radiation produced by C_{1FRP} and/or C_{2FRP} is larger than the intensity, power, energy, or energy density of the radiation produced by the external radiation.

The invention also relates to nanoparticle or at least one constituent of the composition for use in a method according to the invention, wherein the radiation is selected in the group consisting of: i) an undulating radiation, ii) a particulate radiation, iii) an acoustic wave, iv) a heating radiation, v) a non-heating radiation, vi) an ionizing radiation, vii) a non-ionizing radiation, viii) a laser, ix) a light, x) a sound radiation, xi) an ultrasound radiation, xii) a hyper-sound radiation, xiii) an infrasound radiation, xiv) an X-ray radiation, xv) an electromagnetic radiation, xvi) a radiation due to an electric, magnetic, electromagnetic field, a magnetic field, an alternating magnetic field, xvii) a thermal radiation, xviii) a radiation due to or producing by cold, xix) a radiation due to or producing heat, xx) a radiation produced by or associated with zero-mass, non-zero-mass, neutrons, photon protons, electrons, positron, alpha particle, beta, gamma, neutrinos or muon particle presence or emission, xxi) a radiation oscillating at least 1 frequency or wavelength, and xxii) a source producing any of the previously cited radiation(s). The invention also relates to nanoparticle or at least one constituent of the composition for use in a method according to the invention, wherein the nanoparticle or at least one constituent of the composition comprises a metallic core that is synthesized by a living organism or nanoparticle or at least one constituent of the composition producing cells, preferentially a magnetosome or magnetosome mineral, wherein the living organism or nanoparticle or at least one constituent of the composition producing cells is preferentially a magnetotactic bacterium.

The invention also relates to nanoparticle or at least one constituent of the composition for use in a method according to the invention, wherein C and/or D is/are photosensitizer(s), selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or at least one constituent of the composition or nanomaterial, 56) Natural products or compounds, 57) Non-Steroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66) Silicon phthalocyanine (PC4), 67) RLP068, 68) Porfimer sodium, 69) Porfins, 69) Porphyrins, such as 5,10,15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene.

The invention also relates to nanoparticle or at least one constituent of the composition for use in a method according to the invention, wherein C and/or D is/are sono-sensitizer(s), selected in the group consisting of: 1) ABS-FA, 2) Acrylonitrile Butadiene Styrene, 3) Styrene, 4) Folic acid, 5) AIMP NP, aminoacyl tRNA synthetase complex-interacting multifunctional protein, 6) Au Nanomaterial, 7) gold, 8) Au-MnO nanomaterial, 9) manganese oxide, 10) Antineoplastic drugs, 11) NSAIDs, 12) nonsteroidal anti-inflammatory drug, 13) Artemether, 14) 5-ALA (5-aminolevulinic acid), 15) Acridine, Acridine Orange, 16) Au-doped TiO2, 17) Carbon based nanomaterial, 18) carbon nanotube, 19) Chlorine, 20) Ce6, 21) PTX, Paclitaxel, 22) chemotherapeutic drug or compound, 23) infrared dye or IR783, 24) Curcumin, 25) Cyanine or Cu-Cyanine, 26) DHMS, 27) dimethylsulfure, 28) Docetaxel, 29) chemotherapeutic drug or compound, 30) DOX/Mn-TPPS@RBCS, 31) doxorubicin, 32) manganese, 33) blood cell, 34) red blood cell, cell, 35) polymer, 36) elastomer, 37) Erythosin or Erythosin B, 38) FA or FA-OI or FA-OI NP or folic acid, 39) F3-PLGA@MB/Gd NPs, 40) poly(lactic-co-glycolic acid), 41) gadolinium, 42) Fe-TiO2 or titanium oxide, 43) Fe-VS2, 44) iron, 45) vanadium disulfide, 46) FMSNs-DOX, 47) silica, 48) HCQ, 49) hydrochloroquine, 50) HP, 51) hematoporphyrin, 52) HMME, 53) hematoporphyrin monomethyl ether, 54) HSYA or Hydroxysafflor yellow A, 55) Hypocrellin,

Hypocrellin B, 56) IR780, 57) Levofloxacin, 58) LIP3 or Lithium phosphide, 59) Lithium, 60) Liposome or Liposomal nanomaterial, 61) Lomefoxacin" 62) MG@P NPs, 63) MnP or Manganese peroxidase, 64) MnTTP-HSAs, 65) HSA-wrapped metal-porphyrin complex, 66) albumin, 67) MnWOx, 68) MnWOx-PEG, 69), PEG, 70) bimetallic oxide, 71) Mn (III)-HFs, 72) managense, hemoporfin, 73) Nanoroads, 74) Noble metal nanomaterial, 75) OI NP or oxygen indyocyanine, 76) Phthalocyanines, 77) PIO or Pioglitazone, 78) Polymeric nanomaterial, 79) Porphyrin, 80) Pt-doped TiO2, 81) R837, 82) Rose Bengal, 83) Sparfloxacin,, 84) TAPP or 5,10,15,20-tetrakis (4-aminophenyl) porphyrin, 85) TiO2 or titanium dioxide nanomaterial, 86) TCPP, isomer, or Tris(1-chloro-2-propyl) phosphate 87) TPI or Thermoplastic Polyimide or thermoplastic polymer, 88) TPZ or Tirapazamine, 89) Transition metal oxide, 90) nanoparticle or at least one constituent of the composition or Janus nanoparticle or at least one constituent of the composition, and 91) Xanthones.

The invention also relates to nanoparticle or at least one constituent of the composition for use in a method according to the invention, wherein C and/or D is/are radio-sensitizer(s), selected in the group consisting of: : 1) AMG102, 2) AQ4N, 3) Apaziquone (E09), 4) Bromodeoxyuridine, 5) Carbogen, 6) Cetuximab, 7) Chemotherapeutic drug or compound, 8) Chlorpromazine, 9) C-reactive peptide, 10) Curcumin, 11) Diamide, 12) Diethylmaeate,, 13) Dihydroartemisinin, 14) Docetaxel, 15) ECI301, 16) Etanidazole, 17) Fludarabine, 18) 5-Fluorouracil, 19) Fluorodeoxyuridine, 20) Gadolynium, 21) Gemcitabine, 22) HER-3 ADC, 23) HSP, 24) Hydrogen peroxide, 25) Hydroxyurea, 26) Hyperbaric oxygen, 27) Hyperthermia, 28) Hypoxic cell cytotoxic agent, 29) Irinotecan, 30) lanthanide-doped radiosensitizer-based metal-phenolic network, 31) Lidocaine, 32) Lododeoxyuridine, 33) Metronidazole, 34) misonidazole, 35) etanidazole, 36) nimorazole, 37) N-Ethylmalemide, 38) malmeide, 39) ethylmalmeide, 40) Nanomaterial such as those consisting of or composed of at least partly or fully gold, silver, bismuth, gadolinium, polysiloxane matrix and gadolinium chelates, hafnium, Tantalum, Zinc, Gadolinium, Germanium, Chromium, Praseodymium, Silicon, iron, platinum, cobalt, manganese, magnesium, iron, Titanium, carbon nanotube, quantum dot, nanoroad, Triflate, or metal oxide, 41) Nelfinavir, 42) Nicotinamide, 43) Nimotuzumab, 44) RNA, or miRNA, or miR-201, or miR-205, or miR-144-5p, or miR-146a-5p, or miR-150, or miR-99a, or miR-139-5p, or miR-320a, 45) Membrane active agent, 46) Mitomycin-C or Mitomycin, 47) Motexafin, 48) NBTXR3, 49) Oligonucleotide, 50) Paclitaxel, 51) Papaverine or Papaverine hydrochloride, 52) Paraxonase-2, 53) Pocaine, 54) Porfiromycin (POR), 55) Protein, 56) Peptide, 57) Radiosensitizing nucleosides or compounds, 58) Resveratrol, 59) RRx-001, 60) SiRNa, 61) Suppressors of sulfhydral groups, 62) SYM004, 63) Texaphyrins, 64) TH-302, and 65) Tirapazamine.

The invention also relates to nanoparticle or at least one constituent of the composition for use in a method according to the invention, wherein C and/or D is/are oxidizing compound(s).

The invention also relates to nanoparticle or at least one constituent of the composition for use in a method according to the invention, wherein C and/or D is/are anti-oxidizing compound(s).

The inventions relates to a preparation comprising at least one multiply active nanoparticle or at least one constituent of the composition, where the nanoparticle or at least one constituent of the composition comprises:
- a metallic central part composed of at least three different compounds A, B, and C,
- a coating part surrounding the central part composed of at least one compound D,
   wherein optionally the metallic central part is crystallized,
   wherein optionally the coating part is amorphous,
   wherein A is a metal, preferentially iron,
   wherein B is a binding or coordinating material or is oxygen,
   wherein C is preferentially a metallic compound with at least property selected in the group consisting of:
      i) it is a photosensitizer,
      ii) it is a sonosensitizer,
      iii) it is a radio-sensitizer,
   wherein D is a non-metallic or organic compound with at least one property selected in the group consisting of:
      i) it is a photosensitizer,
      ii) it is a sonosensitizer,
      iii) it is a radio-sensitizer,
      iv) it is an anti-oxidant,

In some cases, the center of activity or free radical capture C_{FRC1} or C_{FRC2} comprises a compound E.

The invention relates to nanoparticle or at least one constituent of the compositions for use in the method according to the invention, wherein the coating of the nanoparticle or at least one constituent of the composition preferentially comprises holes, enabling the diffusion of free radicals produced by compound C and/or A preferentially during step 2) or the capture of free radicals by the compound E preferentially during step 2'), hence preferentially increasing the quantity of produced free radicals by compound(s) C and/or A or the quantity of free radicals captured by compound E.

The invention also relates to nanoparticles or at least one constituent of the composition for use in the method according to the invention to detach or disassemble the coating of the nanoparticle or at least one constituent of the composition, compound C or compound E, preferentially by increasing the duration t₂ or t₅, or by choosing a duration t₂ or t₅ that is longer than t₁, t₄, t₃, and/or t₆, or by introducing the nanoparticle or at least one constituent of the composition(s) in a degrading/altering medium/material such as acidic/basic pH media or liposomes.

The invention also relates to nanoparticle or at least one constituent of the compositions for use in the method according to the invention, wherein the nanoparticle or at least one constituent of the composition comprises:
a metallic central part or core comprising the compounds A, B, and C, or A, B, and E,
a coating part surrounding the central part or core comprising the compound D or the compound F,
wherein the central and coating parts are characterized by at least one property selected in the group consisting of:
   - the metallic part is crystallized, partly or fully,
   - the coating part is amorphous, partly or fully,
   - A is a metal, preferentially iron,
   - B is a binding or coordinating material or is oxygen,
   - C when present is a metallic compound different from iron,
   - E when present is a metallic compound different from iron,
   - D when present is a non-metallic or organic compound,
   - F when present is a non-metallic or organic compound,
   - C and D are separated by more than 0.1 or 1 nm,
   - E and F are separated by more than 0.1 or 1 nm,
   - C and A have a larger binding strength than D and A, and
   - E and A have a larger binding strength than F and A.

The invention also relates to nanoparticle or at least one constituent of the compositions for use according to the invention, where the central part or core of the nanoparticle or at least one constituent of the composition has at least one property selected in the group consisting of:
a. it is a magnetosome mineral synthesized by a magnetotactic bacterium,
b. it is ferrimagnetic,
c. it is composed of maghemite or magnetite or of an intermediate composition or at least one constituent of the composition between maghemite and magnetite,
d. it has a size comprised between 0.1 and 100 nm,
e. it comprises at least one crystallographic plane.

The invention also relates to nanoparticle or at least one constituent of the compositions for use in the method according to the invention, where the compound C is selected among zinc and aluminum.

The invention also relates to the nanoparticle or at least one constituent of the compositions for use in a method according to the invention, wherein the proportion of A, B, and C compounds in the central part or core of the nanoparticle or at least one constituent of the composition is such that:
i) the percentages in mass of A and B compounds in the nanoparticle or at least one constituent of the composition are larger than 50%, and/or
ii) the percentage in mass of C compound in the nanoparticle or at least one constituent of the composition is lower than 50%.

The invention also relates to the nanoparticle or at least one constituent of the composition for use in the method according to the invention, wherein the coating part surrounding the central part or core of the nanoparticle or at least one constituent of the composition has at least one property selected in the group consisting of:
i) it has a thickness that is lower than 10 µm,
ii) it comprises a number of crystallographic planes per unit surface that is lower than the number of crystallographic planes per unit surface of the central part,
iii) it is more rapidly degraded than the central part, and
iv) it has a non-neutral charge.

Nanoparticle or at least one constituent of the composition for use in a method according to any of the claims 1 to 9The preparation according to claim 8, where C and D compounds act synergistically, i.e. that the photo-sensitizing or sono-sensitizing or radio-sensitizing strength of the nanoparticle or at least one constituent of the composition comprising C and D is larger than the photo-sensitizing or sono-sensitizing or radio-sensitizing strength of the nanoparticle or at least one constituent of the composition comprising only C or only D.

The invention also relates to nanoparticle or at least one constituent of the compositions for use in the method according to the invention, where C and D are compounds selected in two different groups 1 and 2, where:
- group 1 consists of photosensitizers, sonosensitizers, and radio-sensitizers,
- group 2 consists of antioxidants,

The invention also relates to nanoparticle or at least one constituent of the composition for use in the method according to the invention, where C and D act anti-synergistically, *i.e.* that the photo-sensitizing or sono-sensitizing or radio-sensitizing strength of the nanoparticle or at least one constituent of the composition comprising C and D is lower than the photo-sensitizing or sono-sensitizing or radio-sensitizing strength of the nanoparticle or at least one constituent of the composition comprising only C or only D.

The invention also relates to nanoparticle or at least one constituent of the compositions for use in the method according to the invention, where the nanoparticle or at least one constituent of the composition first comprises the compounds C and D during a time t₇ and second comprises the compounds D during a time t₈, where t₈ preferentially follows t₇.

The invention also relates to nanoparticle or at least one constituent of the compositions for use in the method according to the invention being or comprising: i) a medical device, ii) a drug, iii) a pharmaceutical product or composition or at least one constituent of the composition, iv) a medical product or composition or at least one constituent of the composition, iv) a biological product or composition or at least one constituent of the composition, v) a composition or at least one constituent of the composition, vi) an adjuvant, vii) an excipient, viii) an active principle, ix) a vaccine or vaccine component, and/or vi) a product.

The invention also relates to the nanoparticle or at least one constituent of the compositions for use in a method according to the invention,.

The invention relates to the method according to the invention for use in photodynamic therapy, sonodynamic therapy, and/or radiotherapy.

The invention also relates to nanoparticle or at least one constituent of the composition for use in the method according to the invention, for the treatment of an infectious, viral, or cancerous disease.

The invention also relates to a method of fabrication of the nanoparticle or at least one constituent of the composition according to the invention, which comprises at least one of the following steps:
- **Step 1** of amplifying magnetotactic bacteria preferentially in a in at least one medium, preferentially selected among a pre-growth, growth and/or fed-batch medium(a), preferentially comprising:
   1) the compounds necessary for the growth of magnetotactic bacteria and/or the production of magnetosomes, which are preferentially selected in the group consisting of:
      - a source of carbon, which is preferentially selected from the group consisting of: at least one compound comprising at least one atom of carbon, lactic acid, Na lactate, acetate, glycolate, glucose, pyruvate, succinate, carbon dioxide, glycerol and combinations thereof, at a concentration preferentially comprised between 1 nM and 2 Mol/L;
      - a source of iron preferentially selected from the group consisting of: at least one compound comprising at least one atom of iron, iron citrate, iron quinate, iron chloride, iron sulfate, FeCl₃, and combinations thereof, at a concentration preferentially comprised between 1 nM and 2.10⁻³ Mol/L;
      - a source of nitrogen preferentially selected from the group consisting of: at least one compound comprising at least one atom of nitrogen, nitrate salt, nitrogen gas, ammonium, ammonia, ammonium salt, urea, an amino acid, ammonia gas, and combinations thereof, at a concentration preferentially comprised between 1 nM and 4 Mol/L;
      - a source of oxygen preferentially selected from the group consisting of: at least one compound comprising at least one atom of oxygen, oxygen or air or compressed air, preferentially in the form of a gas, the source of oxygen being in some cases bubbled or introduced to the growth medium, at a gas rate that is preferentially comprised between 5 mL of gas per minute and 50000 mL of gas per minute;
      - a source of phosphate preferentially consisting of at least one compound comprising at least one atom of phosphate, at a concentration preferentially comprised between 1 nM and 2.10⁻¹ Mol/L;
      - a source of potassium preferentially consisting of at least one compound comprising at least one atom of potassium, at a concentration preferentially comprised between 1 nM and 2.10⁻¹ Mol/L;
      - a source of sulfur or sulfate preferentially consisting of at least one compound comprising at least one atom of sulfur or sulfate, at a concentration preferentially comprised between 1 nM and 4.10⁻¹ Mol/L;
      - a source of manganese preferentially consisting of at least one compound comprising at least one atom of manganese, at a concentration preferentially comprised between 1 nM and 4.10⁻¹ Mol/L;
      - a source of vitamin preferentially selected from the group consisting of: at least one compound comprising at least one vitamin, Biotin, Calcium, pantothenate, Folic acid, Inositol, Nicotinic acid, p-Aminobenzoic acid, Pyridoxine HCl, Riboflavin, Thiamine, Thiamine HCL and derivatives thereof and combinations thereof, at a concentration preferentially comprised between 1 nM and 10⁻⁴ Mol/L, and
      - a source of calcium preferentially consisting of at least one compound comprising at least one atom of calcium, at a concentration preferentially comprised between 1 nM and 10⁻¹ Mol/L.
   2) at least one compound necessary for doping the magnetosomes with C_{1FRPC} or another metal than iron, preferentially zinc or aluminum, for example a source of zinc, preferentially zinc sulfate or zinc citrate or zinc chlorate or zinc quinate.
      - **Step** 2 of extracting magnetosomes from magnetotactic bacteria and/or of purifying the extracted magnetosomes preferentially by heating them to yield magnetosome minerals preferentially comprising a percentage in mass of organic material coming from magnetotactic bacteria that is lower than 100, 50 or 1%,
      - **Step** 3 of coating the magnetosome minerals with a coating material preferentially comprising the compound C_{2FRPC} by mixing the magnetosome minerals with the coating material, where the mixing is preferentially realized in at least one of the following conditions:
         i) under sonication,
         ii) under the application of radiation,

         i) under temperature variation,
         ii) under pH changes,
         iii) under oxidoreduction potential adjustment,
         preferentially using a ratio between the quantity or mass of magnetosome minerals and the quantity or mas of coating material, preferentially of compound D, that is preferentially adjusted or varied or larger than 1,
      - **Step 4** of adding at least one cryoprotectant or protectant compound to the coated magnetosome minerals preferentially obtained at the end of step 3,
      - **Step 5** of lyophilizing or dehydrating or drying or desiccating the composition or at least one constituent of the composition preferentially obtained at the end of step 4,
      - **Step 6** of re-suspending the lyophilized composition or at least one constituent of the composition preferentially obtained from step 5, preferentially in water or in another liquid.

The invention also relates to the method according to the invention, wherein the concentration of the source of zinc, preferentially zinc citrate or zinc sulfate, is comprised between 1 and 100 µM, preferentially 2 and 50 µM, most preferentially 5 and 20 µM.

The invention also relates to the method according to the invention, wherein the nanoparticle or at least one constituent of the compositions fabricated according to the method according to the invention comprise a quantity of metal other than iron, preferentially zinc or aluminum, which is incorporated in or comprised in the metallic core is: i) comprised between 0.1 and 100 mg of metal other than iron comprised in the core per gram of iron comprised in the core, ii) preferentially comprised between 0.5 and 10 mg of metal other than iron comprised in the core per gram of iron comprised in the core, iii) most preferentially comprised between 1 and 5 mg of metal other than iron comprised in the core per gram of iron comprised in the core.

The invention relates to a composition or at least one constituent of the composition comprising at least one nanoparticle or at least one constituent of the composition, wherein each nanoparticle or at least one constituent of the composition preferentially comprises an iron oxide mineral core or an iron oxide core or a metallic core or a core composed of a metal oxide, surrounded by a coating,
wherein the composition or at least one constituent of the composition further preferentially comprises a cryoprotectant or protectant compound,
wherein preferentially the dissociating energy between the coating and the core is larger than the dissociating energy between the cryoprotectant or protectant compound and the core,
wherein preferentially the core comprises a first center of activity or free radical production or capture, C_{1FRPC}, and/or
wherein preferentially the coating comprises a second center of activity or free radical production or capture, C_{2FRPC},
wherein C_{1FRPC} and/or C_{2FRPC} preferentially remain(s) comprised or is/are comprised in the core and/or coating, respectively, preferentially at a temperature that is lower than 0°C. In one embodiment of the invention, the composition or at least one constituent of the composition comprises the at least one nanoparticle or at least one constituent of the composition with the cryoprotectant or protectant compound and optionally a liquid such as water.

In some cases, the nanoparticle or at least one constituent of the composition comprises a core and/or a coating, wherein the coating preferentially stabilizes the core and/or prevents the core to aggregate and/or leads to the chain arrangement of at least two nanoparticle or at least one constituent of the compositions.

In some other case, the nanoparticle or at least one constituent of the composition is amorphous and/or crystallized.

In some cases, an iron oxide core is composed of at least one iron atom and at least one atom of oxygen. In some other cases, a core composed of a metal oxide is composed of at least one metal atom and at least one oxygen atom.

In still some other cases, a metallic core is composed of at least one metal atom.

In one embodiment, the center of activity or free radical production or capture is a compound that increases the quantity of free radicals produced or captured when: i) a radiation is applied on the center, preferentially designated as radio-sensitizer in this case, ii) light is applied on the center, preferentially designated as photo-sensitizer in this case, iii) thermal radiation or temperature increase or temperature decrease is applied on the center, preferentially designated as thermo-sensitizer in this case, iv) cryo treatment or protection applied on the center, preferentially designated as cryo-sensitizer in this case, v) a magnetic field is applied on the center, preferentially designated as magneto-sensitizer in this case, vi) ultrasound is applied on the center, preferentially designated as sonosensitizer.

In one embodiment, the center of activity or free radical production or capture can be a center of physico-chemical disturbance, such as radiation, amplification, where the amplification can correspond to an effect of the physico-chemical disturbance applied on the body part that is ore important in the presence than in the absence of the center, for example eradication or death of a tumor could be induced in the presence of the irradiated center while such effect would be absent or less pronounced in the absence of the center.

In one embodiment, the center of activity or free radical production or capture is a compound that amplifies or increases the physico-chemical disturbance or radiation or at least one parameter or property of the physico-chemical disturbance or radiation or at least one effect of a radiation or physico-chemical disturbance. When the center is exposed to radiation, light, thermal variation, cryo-treatment or cryo-protection, magnetic field, or ultrasound, it can be designated as radio-sensitizer, photo-sensitizer, thermo-sensitizer, cryo-sensitizer, magneto-sensitizer, or sonosensitizer, respectively.

In another embodiment of the invention, the composition or at least one constituent of the composition comprises the at least one nanoparticle or at least one constituent of the composition without the cryoprotectant or protectant compound and optionally a liquid such as water.

In another embodiment of the invention, the composition or at least one constituent of the composition comprises an excipient, an active principle, and/or a surfactant.

In another embodiment of the invention, the composition or at least one constituent of the composition is a preparation or a suspension or a powder, preferentially of or with the at least one nanoparticle or at least one constituent of the composition, optionally with the cryoprotectant or protectant compound. In one embodiment of the invention, the cryoprotectant or protectant compound is or comprises at least one substance or compound or chemical function or molecule or atom, which protects or maintains or keeps at least one property of the composition or at least one constituent of the composition, preferentially selected among: i) the chain arrangement or geometric figure of the at least two nanoparticle or at least one constituent of the composition(s), ii) the at least one center of activity or free radical capture or production, iii) the isotonicity or osmolality, preferentially of or in the composition or at least one constituent of the composition, preferentially at or below the temperature of 10⁵, 10³, 10², 50, 20, 10, 5, 2, 1, 0, -5, -10, -50, -77, -100, -200 or -273 °C.

In another embodiment of the invention, the at least one property of the composition or at least one constituent of the composition is maintained, or kept or protected when:
i) At least two nanoparticle or at least one constituent of the compositions preferentially remain arranged in chains or in a geometric figure,
ii) At least one center of activity or free radical production or capture preferentially remain comprised in the nanoparticle or at least one constituent of the composition or composition or at least one constituent of the composition or remain active, *i.e.* able to produce or capture free radical preferentially under the application of a radiation,
   and/or
iii) The isotonicity, osmolality, and/or at least one other property of the nanoparticle or at least one constituent of the composition or composition or at least one constituent of the composition preferentially does not change or vary by more than 10⁻³, 1, 5, 10, 25, 50, 75 or 100%, where this percentage is preferentially equal to (P2-P1)/P1, preferentially measured in absolute terms or values, where P1 and P2 are values of isotonicity, osmolality, and/or at least one other property of the nanoparticle or at least one constituent of the composition or composition or at least one constituent of the composition at a temperature T1 and T2, respectively,
preferentially for or when the temperature of the composition or at least one constituent of the composition or nanoparticle or at least one constituent of the composition is maintained at or decreased below or at the temperature of 10⁵, 10³, 10², 50, 20, 10, 5, 2, 1, 0, -5, -10, -50, -77, -100, -200 or -273 °C.

In some cases, T1 and T2 are two different temperatures.

In some cases, T2 is smaller than T1, preferentially by at least 10⁻⁵, 10⁻³, 10 ', 0, 1, 2, 5, 10 or 10³ °C. In some cases, T1 and/or T2 is/are smaller than 10⁵, 10³, 10², 50, 20, 10, 5, 2, 1, 0, -5, -10, -50, -77, - 100, -200 or -273 °C.

In some other cases, T1 and/or T2 is/are larger than -273, -200, -100, -77, -50, -10, -5, 0, 1, 2, 5, 10, 50, 100 or 10³ °C.

In one embodiment of the invention, the composition or at least one constituent of the composition can be cooled down, preferentially below or at 10, 5, 2, 1, 0, -5, -10, -50, -77, -90, -270, or -273 °C, or the composition or at least one constituent of the composition below or at 10, 5, 2, 1, 0, -5, -10, -50, -77, - 90, -270, or -273 °C, can be reached or obtained preferentially for more than 1 second, 1 minute or 1 hour, preferentially without destroying at least one chain or geometric figure or at least one center of activity or free radical/production, preferentially with at least one chain or geometric figure or at least one center of activity or free radical/production that is maintained or exists. This effect is preferentially due to the presence of the cryoprotectant or protectant compound.

In one embodiment of the invention, the composition or at least one constituent of the composition is lyophilized or desiccated, preferentially for more than 1 second, 1 minute or 1 hour, preferentially without destroying or removing at least one center of activity or free radical capture or production or at least one chain or geometric figure, preferentially with at least one chain or geometric figure at least one center of activity or free radical/production that is maintained or exists. This effect is preferentially due to the presence of the cryoprotectant or protectant compound.

In one embodiment of the invention, the composition or at least one constituent of the composition is prepared or used by following at least one of the following steps:
- First, the composition or at least one constituent of the composition is preferentially mixed in water to add the cryoprotectant or protectant compound to the at least one nanoparticle or at least one constituent of the composition or chain in liquid suspension.
- Second, the composition or at least one constituent of the composition is preferentially lyophilized to remove water from the composition or at least one constituent of the composition and keep the composition or at least one constituent of the composition in a powder form for storage.
- Third, the composition or at least one constituent of the composition is preferentially resuspended in water, ready for use and administration to humans.

In one embodiment, the at least one step mentioned above or in a method according to the invention is repeated.

In one embodiment of the invention, the dissociation energy between a chemical group of a molecule of the coating and a chemical group of the core of the nanoparticle or at least one constituent of the composition is preferentially larger than 10 Kcal/mol, 100 KJ/mol, or 1 eV/bond.

In one embodiment of the invention, the dissociation energy between: i) a chemical group of a molecule of the cryoprotectant or protectant compound and a chemical group of the core of the nanoparticle or at least one constituent of the composition or ii) a chemical group of a molecule of the cryoprotectant or protectant compound and a chemical group of the coating of the nanoparticle or at least one constituent of the composition, is preferentially lower than 10 Kcal/mol, 100 KJ/mol, or 1 eV/bond.

The invention relates to the composition or at least one constituent of the composition according to invention, wherein the chemical affinity between the coating and the core of the nanoparticle or at least one constituent of the composition is preferentially larger than: i) the chemical affinity between the cryoprotectant or protectant compound and the coating and/or ii) the chemical affinity between the cryoprotectant or protectant compound and the core of the nanoparticle or at least one constituent of the composition.

In one embodiment of the invention, the coating is more strongly bound to or is in stronger interaction with the nanoparticle or at least one constituent of the composition core than the cryoprotectant or protectant compound.

In one embodiment of the invention, the coating can't be separated or isolated from the nanoparticle or at least one constituent of the composition core or is inseparable or un-isolable from the nanoparticle or at least one constituent of the composition core, preferentially using a magnet or magnetic separation or centrifugation.

In another embodiment of the invention, the cryoprotectant or protectant compound can be separated or isolated from the nanoparticle or at least one constituent of the composition core or is separable or isolable from the nanoparticle or at least one constituent of the composition core, preferentially using a magnet or magnetic separation or centrifugation.

In some cases, this property can be highlighted by the fact that: i) the coating and core of the nanoparticle or at least one constituent of the composition can't be separated by a magnet of low intensity, *i.e.* preferentially of intensity smaller than 1 T or 1 mT, or of low magnetic field gradient, *i.e.* preferentially of magnetic field gradient smaller than 1 T or mT per cm preferentially of body part, whereas the cryoprotectant or protectant compound can preferentially be separated from the nanoparticle or at least one constituent of the composition by such magnet, ii) the coating and core of the nanoparticle or at least one constituent of the composition can't preferentially be separated by mixing the nanoparticle or at least one constituent of the composition core and coating in water followed by centrifugation whereas the cryoprotectant or protectant compound can preferentially be separated from the nanoparticle or at least one constituent of the composition by being mixed in water with the suspended nanoparticle or at least one constituent of the compositions followed by centrifugation.

In one embodiment, the coating comprises at least one compound or chemical function, which is able to establish interactions or weak interactions or weak bonds or covalent bonds with the core of the nanoparticle or at least one constituent of the composition or at least one chemical function or atom or ion of the core part of the nanoparticle or at least one constituent of the composition, in particular iron oxide or any combination or ionic state of iron and/or oxygen. In some cases, such interactions or bonds maintain the nanoparticle or at least one constituent of the composition coating attached or linked or associated with or to the nanoparticle or at least one constituent of the composition core, preferentially called associating or attaching or linking bonds or interactions.

In one embodiment of the invention, the nanoparticle or at least one constituent of the composition consists of a core, preferentially a surrounding coating and preferentially associating or linking or attaching bonds or interactions.

In one embodiment of the invention, the nanoparticle or at least one constituent of the composition consists of a core and preferentially a surrounding coating, which is preferentially associated or linked or attached to or with the core, preferentially in such a way or through sufficiently strong bonds or interactions that it does not detach or dissociates from the core or that it remains attached or bound to the core.

In some cases, the at least one center of activity or free radical capture or production or chain arrangement or geometric figure or assembly of the nanoparticle or at least one constituent of the compositions is maintained or exists or forms in the presence of the coating.

In some cases, in the absence of coating the at least one center of activity or free radical capture or production or chain arrangement or geometric figures does not exist or does not form or does not exist. In one embodiment of the invention, the composition or at least one constituent of the composition comprises a cryoprotectant or protectant compound, which is associated or linked or bound with or to the nanoparticle or at least one constituent of the composition, core, coating, preferentially in such a way or through sufficiently weak bonds or interactions that it detaches or dissociates from the nanoparticle or at least one constituent of the composition, core or coating, preferentially when the cryoprotectant or protectant compound is washed away or removed from the nanoparticle or at least one constituent of the compositions with water, by centrifugation or by using a magnet.

In some cases, the cryoprotectant or protectant compound can be dissociated or detached or disassembled or unlinked from the nanoparticle or at least one constituent of the composition, core or coating, preferentially in such a way that the at least one center of activity or free radical capture or production or chain arrangement or geometry of the nanoparticle or at least one constituent of the compositions is maintained optionally comprised in the nanoparticle or at least one constituent of the composition.

In some cases, the cryoprotectant or protectant compound can be associated or attached or assembled or linked to or with the nanoparticle or at least one constituent of the composition, core or coating, preferentially in such a way that the at least one center of activity or free radical capture or production or chain arrangement or geometry of the nanoparticle or at least one constituent of the compositions is maintained, optionally in the nanoparticle or at least one constituent of the composition, preferentially when the composition or at least one constituent of the composition is cooled down, preferentially below or at 100, 10, 5, 2, 1, 0, -10, -50, -77, -270, -273 °C, preferentially for more than 0.001, 0.1, 0, 1, 5, 10, 10³ or 10⁵ seconds, or when the composition or at least one constituent of the composition is lyophilized or desiccated or when water is removed from the composition or at least one constituent of the composition partly or fully or when the percentage in mass of water in the composition or at least one constituent of the composition is lower than 100, 50, 10, 5, 2, 1, 0, 10⁻³ or 10⁻⁵ % or when the composition or at least one constituent of the composition is in powder form.

In some cases, the at least one center of activity or free radical capture or production or chain arrangement or geometric figure of the nanoparticle or at least one constituent of the compositions can be observed by following at least one of the following step: i) removing the cryoprotectant or protectant compound from the composition or at least one constituent of the composition, ii) suspending or resuspending the composition or at least one constituent of the composition, preferentially lyophilized, preferentially in water, iii) by depositing a droplet of the composition or at least one constituent of the composition on top of a substrate, preferentially a carbon grid, iv) by waiting for water evaporation, and v) by observing the nanoparticle or at least one constituent of the composition arrangement under electron microscopy.

In some cases, preferentially in the absence of coating at least one center of activity or free radical capture or production the chains or geometric figures do not form or exist while in the presence of coating the chains or geometric figures or at least one center of activity or free radical/production preferentially form or exist.

In some cases, when the temperature of the at least one nanoparticle or at least one constituent of the composition is lower than or equal to 100, 10, 5, 2, 1, 0, -10, -50, -77, -270, -273 °C, preferentially for more than 0.001, 0.1, 0, 1, 5, 10, 10³ or 10⁵ seconds, or when the nanoparticle or at least one constituent of the composition is lyophilized or desiccated or dehydrated or dried or when water is removed from the composition or at least one constituent of the composition or from the at least one nanoparticle or at least one constituent of the composition partly or fully or when the percentage in mass of water in the composition or at least one constituent of the composition the at least one nanoparticle or at least one constituent of the composition is lower than 100, 50, 10, 5, 2, 1, 0, 10⁻³ or 10⁻⁵ %, at least one center of activity or free radical capture or production or the chain arrangement or the geometric figure or assembly preferentially does not exist or does not form or is destroyed in the absence of the cryoprotectant or protectant compound, and/or the at least one center of activity or free radical capture or production or chain arrangement or the geometric figure or assembly preferentially exists or forms or is maintained or is not destroyed in the presence of the cryoprotectant or protectant compound.

In one embodiment of the invention, the coating or at least one compound or chemical function comprised in the coating is or remains chemisorbed or physisorbed or adsorbed or attached to or linked to or associated with or bound to the core of the nanoparticle or at least one constituent of the composition, preferentially in the presence of the cryoprotectant or protectant compound, preferentially surrounding or embedding the nanoparticle or at least one constituent of the composition, preferentially for a duration of more than 0.001, 0.1, 0, 1, 5, 10, 10³ or 10⁵ seconds, preferentially for or when the composition or at least one constituent of the composition is in powder form, preferentially for or when the percentage in mass of water in the composition or at least one constituent of the composition is lower than 100, 50, 10, 5, 2, 1, 0, 10⁻³ or 10⁻⁵ %.

In one embodiment of the invention, the coating or at least one compound or chemical function comprised in the coating is not or does not remain chemisorbed or physisorbed or adsorbed or attached to or linked to or associated with or bound to the core of the nanoparticle or at least one constituent of the composition, preferentially in the absence of the cryoprotectant or protectant compound, preferentially surrounding or embedding the nanoparticle or at least one constituent of the composition, preferentially for a duration of more than 0.001, 0.1, 0, 1, 5, 10, 10³ or 10⁵ seconds, preferentially for or when the composition or at least one constituent of the composition is in powder form, preferentially for or when the percentage in mass of water in the composition or at least one constituent of the composition is lower than 100, 50, 10, 5, 2, 1,0, 10⁻³ or 10⁻⁵ %.

In one embodiment, the coating or at least one compound or chemical function comprised in the coating is linked through bonds or interactions with or to Fe²⁺ or Fe³⁺ ions, hydroxyls OH⁻, oxides O₂⁻, crystalline defects of the core, which may be in or at the surface of the core of the nanoparticle or at least one constituent of the composition.

In one embodiment, the coating comprises at least one compound, atom, ion, or chemical function such as an acid, carboxylic acid, phosphoric acid, or sulfonic acid function, wherein the compound, atom or ion contained in the coating is able to establish interactions or bonds with the core or with at least one atom of the core, a chemical function of the core, an ion of the core such as Fe²⁺, Fe³⁺, Hydroxyl OH⁻, oxide O2 or a crystalline defect of the core.

In one embodiment of the invention, the interactions or bonds exist or are maintained when the composition or at least one constituent of the composition is cooled down, preferentially below or at 100, 10, 5, 2, 1, 0, -10, -50, -77, -270, -273 °C, preferentially for more than 0.001, 0.1, 0, 1, 5, 10, 10³ or 10⁵ seconds, or when the composition or at least one constituent of the composition is lyophilized or desiccated or dehydrated or dried or when water is removed from the composition or at least one constituent of the composition partly or fully or when the percentage in mass of water in the composition or at least one constituent of the composition is lower than 100, 50, 10, 5, 2, 1, 0, 10⁻³ or 10⁻⁵ % or when the composition or at least one constituent of the composition is in powder form, preferentially in the presence of the cryoprotectant or protectant compound. In some cases, these interactions or bonds keep the at least one center of activity or free radical capture or production comprised in the nanoparticle or at least one constituent of the composition or nanoparticle or at least one constituent of the compositions organized in chain or in geometric figure, *i.e.* that preferentially in the absence of these interactions or bonds, the nanoparticle or at least one constituent of the compositions are not organized in chain or geometric figure or the at least one center of activity or free radical capture or production is not comprised in the nanoparticle or at least one constituent of the composition.

In some cases, a geometric figure is an assembly or aggregate of nanoparticle or at least one constituent of the compositions forming a geometric figure.

In some cases, a geometric figure is selected from the group consisting of:, a Balbis, Concave polygon, Constructible polygon, Convex polygon, Cyclic polygon, Equiangular polygon, Equilateral polygon, Penrose tile, Polyform, Regular polygon, Simple polygon, Tangential polygon, Polygons with specific numbers of sides, Henagon, Digon, Triangle, Acute triangle, Equilateral triangle, Heptagonal triangle, Isosceles triangle, Obtuse triangle, Rational triangle, Right triangle, Kepler triangle, Scalene triangle, Quadrilateral, Cyclic quadrilateral, Kite, Parallelogram, Rhombus, Lozenge, Rhomboid, Rectangle, Square, Tangential quadrilateral, Trapezoid, Isosceles trapezoid, Pentagon, Hexagon, Lemoine hexagon, Heptagon, Octagon, Nonagon, Decagon, Hendecagon, Dodecagon, Tridecagon, Tetradecagon, Pentadecagon, Hexadecagon, Heptadecagon, Octadecagon, Enneadecagon, Icosagon, Swastika, Star polygon, Pentagram - star polygon, Hexagram, Star of David, Heptagram, Octagram, Star of Lakshmi, Decagram - star polygon, Annulus, Arbelos, Circle, Archimedes' twin circles, Bankoff circle, Circumcircle, Disc, Incircle and excircles of a triangle, Nine-point circle, Circular sector, Circular segment, Crescent, Indalo, Lens, Lune, Reuleaux polygon, Reuleaux triangle, Salinon, Semicircle, Tomahawk, Triquetra, Heart, Archimedean spiral, Astroid, Cardioid, Deltoid, Ellipse, Heart, Heartagon, Various lemniscates, Oval, Cartesian oval, Cassini oval, Oval of Booth, Ovoid, Superellipse, Taijitu, Tomoe, and/or Magatama.

In one embodiment of the invention, the nanoparticle or at least one constituent of the composition, the suspension, composition or at least one constituent of the composition, or assembly of nanoparticle or at least one constituent of the composition is stable, preferentially during a lapse of time, preferentially being its stability duration, which is larger than 10⁻¹⁰, 5, 10, 10⁵⁰ or 10¹⁰⁰ minute(s), 1, 2, 3, 4, 5, 6, 10, 12, 24 or 36 months.

In some cases, the nanoparticle or at least one constituent of the compositions, the suspension of nanoparticle or at least one constituent of the compositions, composition or at least one constituent of the composition, or assembly of nanoparticle or at least one constituent of the compositions can be stable preferentially at a concentration of nanoparticle or at least one constituent of the compositions larger than 1, 5, 10, 50, 100, 200, 500 or 1000 mg of nanoparticle or at least one constituent of the compositions per mL of solvent, water, matrix, or body part surrounding or comprising or embedding the or nanoparticle or at least one constituent of the compositions or composition or at least one constituent of the composition.

In one embodiment of the invention, the body part is the body part of an individual, animal, or human. In an embodiment of the invention, the body part comprises more than or at least 1, 2, 5, 10, or 100 similar or different organism(s), apparatus, organ(s), tissue(s), cell(s), or biomolecule(s).

In some cases, the body part can be all or part of the head, neck, shoulder, arm, leg, knee, foot, hand, ankle, elbow, trunk, inferior members, or superior members.

In some other cases, the body part can be or belong to an organ, the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, or integumentary system, reproductive organ (internal or external), sensory organ, endocrine glands. The organ or body part can be human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, or skin.

In some cases, the body part or organ can belong to the blood circulation or circulatory system.

In some cases, the body part can be or comprise at least one tumor, cancer, virus, bacterium, or pathological cell.

In one embodiment of the invention, the body part is or comprises water, an excipient, a solution, a suspension, at least one chemical element, organic material, or gel, which can be synthetic or produced by a living organism.

Preferably, the body part of an individual, also designated as the body part, represents or is part of an individual or a whole individual, where the individual is preferentially a human, an animal, or an organism, preferentially a living or inactivated or dead organism, comprising at least one prokaryotic or eukaryotic cell.

In one embodiment of the invention, the body part is alive (or not), is any tissue, water, medium, substance, cell, organelle, organ protein, lipid, DNA, RNA, biological material, preferentially localized in a specific region of an individual, preferentially originating or extracted from such region.

In an embodiment of the invention, the body part comprises a pathological site, a healthy site, and/or a nanoparticle or at least one constituent of the composition or composition or at least one constituent of the composition region.

In one embodiment of the invention, the body part is or comprises a pathological site or pathological cells.

In some cases, the pathological site can be defined as an unhealthy site, or a site that is in a different condition from a site of a healthy individual, or the site of an unhealthy individual. It can comprise pathological cells, such as tumor cells, bacteria, eukaryotic or prokaryotic cells, as well as viruses or other pathological material. Pathological cells can be cells that are: i) not arranged or working as they usual do in a healthy individual, ii) dividing more quickly than healthy cells, iii) healthy cells having undergone a transformation or modification, iv) dead, sometimes due to the presence of a virus or to other organisms, or v), in contact, in interaction, with foreign material not belonging to the individual, such as viruses, where viruses can possibly penetrate, colonize, or replicate in these cells. In some cases, pathological cells can be assimilated to viruses or to other organisms or entities that colonize cells or target cells or destroy cells or use cells or enter in interaction with cells, preferentially to enable their own reproduction, multiplication, survival, or death. In some cases, a pathological site can comprise healthy cells, preferentially with a lower number, activity or proliferation, than that of pathological cells. In one embodiment of the invention, the body part is or comprises a healthy site or healthy cells. In some cases, the healthy site can be defined as a site or region that comprises healthy cell(s), where a healthy cell can be defined as a cell that belongs to a healthy individual or to the body part of a healthy individual. In some cases, the healthy site can surround the pathological site when it is preferentially located at a distance of less than 1 or 10⁻⁹ m from the pathological site.

In some cases, the composition or at least one constituent of the composition or body part can be exposed to radiation.

Preferably, the radiation is selected from the group consisting of: i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave. In one embodiment of the invention, the radiation has at least one of the following properties:
i) it has a power or power density lower than 1000, 10 or 1 W (Watt), preferentially per cm, W per cm², or W per cm³, preferentially of body part or composition or at least one constituent of the composition, or preferentially per gram or milligram preferentially, preferentially of body part or composition or at least one constituent of the composition,
ii1) it has an energy or energy density lower than 10⁵, 10³, 100 or 1 W.sec per cm, W.sec per cm², or W.sec per cm³, preferentially of body part or composition or at least one constituent of the composition,
ii2) it has an energy or energy density lower than 10⁵, 10³, 100 or 1 W.sec per gram or milligram, preferentially of body part or composition or at least one constituent of the composition,
ii3) it has an energy or energy density lower than 10⁵, 10³, 100 or 1 (Joule), J, preferentially per cm, per cm², or per cm³, preferentially of body part or composition or at least one constituent of the composition, preferentially per gram of milligram, preferentially of composition or at least one constituent of the composition or body part,
iii) it has a frequency lower than 10⁵ 100, 10, 1, 10⁻¹ or 10⁻³ MHz,
iv) it has a penetration depth in the body part lower than 10¹⁰, 10⁵, 10³, 10, 1, 0, or 10⁻⁵ cm,
v) it has a wavelength lower than 10¹⁰, 10⁵, 10³, 100, 50, 10, 10, 5, 2, 1, 0.1 or 0 nm,

In another embodiment of the invention, the radiation has at least one of the following properties:
i) it has a power or power density larger than 10⁻¹⁰, 10⁻⁵, 10⁻³, 10 ', 0, 1, 5, 10, 100, 10³ or 10⁵ W (Watt), preferentially per cm, W per cm², or W per cm³, preferentially of body part or composition or at least one constituent of the composition, or preferentially per gram or milligram preferentially, preferentially of body part or composition or at least one constituent of the composition,
ii1) it has an energy or energy density larger than 10⁻¹⁰, 10⁻⁵, 10⁻³, 10 ', 0, 1, 5, 10, 100, 10³ or 10⁵ W.sec per cm, W.sec per cm², or W.sec per cm³, preferentially of body part or composition or at least one constituent of the composition,
ii2) it has an energy or energy density larger than 10⁻¹⁰, 10⁻⁵, 10⁻³, 10 ', 0, 1, 5, 10, 100, 10³ or 10⁵ W.sec per gram or milligram, preferentially of body part or composition or at least one constituent of the composition,
ii3) it has an energy or energy density larger than 10⁻¹⁰, 10⁻⁵, 10⁻³, 10 ', 0, 1, 5, 10, 100, 10³ or 10⁵ (Joule), J, preferentially per cm, per cm², or per cm³, preferentially of body part or composition or at least one constituent of the composition, preferentially per gram of milligram, preferentially of composition or at least one constituent of the composition or body part,
iii) it has a frequency lower than 10⁻¹⁰, 10⁻⁵, 10⁻³, 10⁻¹, 0, 1, 5, 10, 100, 10³ or 10⁵ MHz,
iv) it has a penetration depth in the body part lower than 10⁻¹⁰, 10⁻⁵, 10⁻³, 10 ', 0, 1, 5, 10, 100, 10³ or 10⁵ cm,
v) it has a wavelength lower than 10⁻¹⁰, 10⁻⁵, 10⁻³, 10⁻¹, 0, 1, 5, 10, 100, 10³ or 10⁵ nm,

In one embodiment of the invention, the nanoparticle or at least one constituent of the composition or composition or at least one constituent of the composition is administered to or in the body part.

In another embodiment of the invention, the nanoparticle or at least one constituent of the composition or composition or at least one constituent of the composition is administered at a distance of less than 1 or 10⁻⁹ m away from the body part.

In still another embodiment of the invention, the nanoparticle or at least one constituent of the composition or composition or at least one constituent of the composition is administered at a distance of more than 1 or 10⁻⁹ m away from the body part.

In another embodiment of the invention, the nanoparticle or at least one constituent of the composition or composition or at least one constituent of the composition is administered to or in the body part preferentially following at least one of the following administration routes: local, enteral, gastrointestinal, parenteral, topical, oral, inhalation, intramuscular, subcutaneous, intra-tumor, in an organ, in a vein, in arteries, in blood, or in tissue.

In some cases, the nanoparticle or at least one constituent of the compositions, the suspension of nanoparticle or at least one constituent of the compositions, the composition or at least one constituent of the composition, or assembly of nanoparticle or at least one constituent of the compositions can be stable preferentially when at least one nanoparticle or at least one constituent of the composition is not degraded or does not lose partly or fully its coating or can be administered to a body part or keeps or maintains its chain arrangement or geometric figure or the presence of at least one center of activity or free radical/production.

In some other cases, the nanoparticle or at least one constituent of the compositions, the suspension of nanoparticle or at least one constituent of the compositions, the composition or at least one constituent of the composition, or assembly of nanoparticle or at least one constituent of the compositions can be stable when the optical density of the nanoparticle or at least one constituent of the compositions, the suspension of nanoparticle or at least one constituent of the compositions, composition or at least one constituent of the composition, or assembly of nanoparticle or at least one constituent of the composition, preferentially mixed in water, preferentially measured at 480 nm or at another fixed wavelength, does not decrease preferentially by more than 1, 5, 10, 50, 75 or 90 % or by more than 10 ¹⁰, 10⁻³, 10 ', 0.5 or 0.7, preferentially within 1, 5, 10, 10³, 10⁷ or 10²⁰ seconds following homogenization or mixing or optical density measurement or absorption measurement of this suspension or composition or at least one constituent of the composition. This percentage can be equal to (OD_{B}-OD_{A})/OD_{B} or OD_{A}/OD_{B}, where OD_{B} is the optical density of the nanoparticle or at least one constituent of the compositions, the suspension of nanoparticle or at least one constituent of the compositions, the composition or at least one constituent of the composition, or assembly of nanoparticle or at least one constituent of the compositions measured before the homogenization or mixing or optical density measurement or absorption measurement of the nanoparticle or at least one constituent of the composition, the suspension, composition or at least one constituent of the composition, or assembly of nanoparticle or at least one constituent of the composition and OD_{A} is the optical density of the nanoparticle or at least one constituent of the compositions, the suspension of nanoparticle or at least one constituent of the compositions, composition or at least one constituent of the composition, or assembly of nanoparticle or at least one constituent of the compositions measured after the homogenization or mixing or optical density measurement or absorption measurement of the nanoparticle or at least one constituent of the compositions, the suspension of nanoparticle or at least one constituent of the compositions, the composition or at least one constituent of the composition, or assembly of nanoparticle or at least one constituent of the compositions.

In some cases, the composition or at least one constituent of the composition can be stable or considered stable preferentially when it is measured stable at a certain first time to and at a certain second time t₁, where t₁ follows t₀ or is separated from t₀ by a lapse of time ΔT of at least 1 second, 1 minute, 1 hour, 1 day, 1 month, 3 months, 6 months, 1, 2, 5, or 10 years. During ΔT, the composition or at least one constituent of the composition is preferentially being stocked.

In some cases, the nanoparticle or at least one constituent of the composition can be suspended in a liquid or dispersed in a matrix or body part preferentially to yield a homogenous nanoparticle or at least one constituent of the composition dispersion or a highly stable nanoparticle or at least one constituent of the composition or suspension.

In one embodiment of the invention, the cryoprotectant or protectant compound serves to maintain the chain or geometric figure or at least one center of activity or free radical/production stable over a period of time, preferentially ΔT, preferentially at least one or six months. In this case, the chain or geometric figure or at least one center of activity or free radical/production can preferentially be observed or measured, preferentially under electron microscopy measurements, at a first time to and at a second time t₁, where t₁ is preferentially separated from t₀ by ΔT.

In one embodiment of the invention, the cryoprotectant or protectant compound protects or maintains the arrangement of at least two nanoparticle or at least one constituent of the compositions in at least one chain or in at least one geometric figure or the presence of at least one center of activity or free radical/production in the composition or at least one constituent of the composition.

In some cases, the protection or maintenance of the arrangement of at least two nanoparticle or at least one constituent of the compositions in at least one chain or in at least one geometric figure or the presence of at least one center of activity or free radical/production occurs at a temperature preferentially of the composition or at least one constituent of the composition that is preferentially lower than or equal to 10³, 500, 100, 50, 10, 5, 2, 1, 0, -5, -10, -20, -50 -100, -200, or -273 °C.

In some other cases, the protection or maintenance of the arrangement of at least two nanoparticle or at least one constituent of the compositions in at least one chain or in at least one geometric figure or the presence of at least one center of activity or free radical/production occurs at a temperature preferentially of the composition or at least one constituent of the composition that is preferentially larger than or equal to -273, -200, -100, -50 -20, -10, -5, 0, 1, 2, 5, 10, 50, 100, 500, or 10³ °C.

In some cases, the protection or maintenance of the arrangement of at least two nanoparticle or at least one constituent of the compositions in at least one chain or in at least one geometric figure or of the presence of at least one center of activity or free radical/production occurs for a duration that is preferentially larger than 10⁻¹⁰, 10⁻⁵, 10⁻³, 10 ', 0, 1, 2, 5, 10, 10², 10³ or 10⁵ minutes or seconds.

In some other cases, the protection or maintenance of the arrangement of at least two nanoparticle or at least one constituent of the compositions in at least one chain or in at least one geometric figure or of the presence of at least one center of activity or free radical/production occurs for a duration that is preferentially smaller than 10¹⁰, 10⁵, 10³, 10, 5, 2, 1, 0, 10⁻¹, 10⁻³ or 10⁻⁵ minutes or seconds.

The invention also relates to the composition or at least one constituent of the composition according to the invention, wherein C_{1FRPC} and/or C_{2FRPC} is/are preferentially selected in the group consisting of:
i) another metal than iron such as Zinc or Aluminum,
   and
ii) another metal oxide than iron oxide such as Zinc Oxide or Aluminum Oxide.

In some cases, C_{1FRPC} and/or C_{2FRPC} can comprises at least one atom that is preferentially linked to at least one oxygen or iron atom of the core preferentially through at least one metallic bound.

The invention also relates to the composition or at least one constituent of the composition according to the invention, wherein coating comprises a second center of activity or free radical production or capture C_{2FRPC}.

The invention also relates to the composition or at least one constituent of the composition according to the invention, wherein C_{2FRPC} is linked to at least one atom of the coating, preferentially through at least one covalent bond.

In one embodiment of the invention, the center a free radical production or capture is at least one atom, molecule, chemical function, ionized or not, charged or not, which produces or captures at least one free radical.

In one embodiment of the invention, a free radical is at least one atom, molecule, ion or chemical function that has: i) at least one unpaired valence electron, ii) a capacity to dimerize, iii) a short lifetime, and/or iv) two unpaired electrons.

In one embodiment of the invention, a free radical is at least one atom, molecule, ion or chemical function that is: i) a chemically reactive species, ii) a hydroxyl radical HO·, iii) a triplet oxygen, iv) a triplet carbene, and/or v) :CH2.

In one embodiment of the invention, radicals are generated or produced when the composition or at least one constituent of the composition is excited in at least one of the following ways: i) by being exposed to redox reactions, ii) by being subjected to radiation, preferentially ionizing or electromagnetic radiation, iii) by being heated, iv) by being exposed to electrical discharges, v) by undergoing electrolysis, vi) by being exposed to pH variation.

In one embodiment of the invention, the center a free radical production is at least one atom, molecule, chemical function, ionized or not, charged or not, which produces at least one free radical, preferentially in such a way that the quantity or concentration of free radicals present when the composition or at least one constituent of the composition comprises the center of activity or free radical production is more important than the quantity or concentration of free radicals present when the composition or at least one constituent of the composition does not comprise the center of activity or free radical production, where the comparison is preferentially being made using the same or similar conditions of excitation for the composition or at least one constituent of the composition comprising the center of activity or free radical production than for the composition or at least one constituent of the composition not comprising the center of activity or free radical production.

In one embodiment of the invention, the center a free radical capture is at least one atom, molecule, chemical function, ionized or not, charged or not, which captures at least one free radical, preferentially in such a way that the quantity or concentration of free radicals present when the composition or at least one constituent of the composition comprises the center of activity or free radical capture is less important than the quantity or concentration of free radicals present when the composition or at least one constituent of the composition does not comprise the center of activity or free radical capture, where the comparison is preferentially being made using the same or similar conditions of excitation for the composition or at least one constituent of the composition comprising the center of activity or free radical capture than for the composition or at least one constituent of the composition not comprising the center of activity or free radical capture.

The invention also relates to the composition or at least one constituent of the composition according to the invention, wherein C_{1FRPC} and/or C_{2FRPC} is/are at least one anti-oxidizing or reducing compound. The invention also relates to the composition or at least one constituent of the composition according to the invention, wherein C_{1FRPC} and/or C_{2FRPC} is/are at least one oxidizing or anti-reducing compound.

In some cases, the oxidizing compound can be a simple body, a compound, or an ion, that receives or is able to receive at least one electron from another chemical species than the oxidizing compound preferentially during a redox reaction.

In some other cases, the reducing compound can be a simple body, a compound, or an ion, that gives or provides or is able to give or provide at least one electron to another chemical species than the oxidizing compound preferentially during a redox reaction.

In some cases, an anti-oxidizing compound is a compound that prevents oxidation preferentially of the nanoparticle or at least one constituent of the composition or of at least one substance of the composition or at least one constituent of the composition.

In some cases, an oxidizing compound is a compound that favors or triggers oxidation preferentially of the nanoparticle or at least one constituent of the composition or of at least one substance of the composition or at least one constituent of the composition.

In some cases, an anti-reducing compound is a compound that prevents reduction preferentially of the nanoparticle or at least one constituent of the composition or of at least one substance of the composition or at least one constituent of the composition.

In some cases, a reducing compound is a compound that favors or triggers reduction preferentially of the nanoparticle or at least one constituent of the composition or of at least one substance of the composition or at least one constituent of the composition.

The invention also relates to the composition or at least one constituent of the composition according to the invention, wherein at least two nanoparticle or at least one constituent of the compositions in the composition or at least one constituent of the composition arrange in a chain or remained arranged in a chain or comprise at least one center of activity or free radical/production, preferentially at or below 0°C.

In some cases, the at least two nanoparticle or at least one constituent of the compositions can be arranged in chains or comprise at least one center of activity or free radical/production at a temperature that is at or lower than 10³, 500, 100, 50, 20, 5, 2, 1, 0, -2, -5, -20, -50, -77, -100, -200 or -273 °C.

In some cases, the at least two nanoparticle or at least one constituent of the compositions can be arranged in chains or comprise at least one center of activity or free radical/production at a temperature that is at or larger than -273 °C, -200, -100, -77, -50, -20, -5, -2, 0, 2, 5, 10 50 or 100 °C.

The invention relates to the composition or at least one constituent of the composition according to the invention, where the at least one nanoparticle or at least one constituent of the composition is in a liquid suspension and the composition or at least one constituent of the composition preferentially has at least one of the following properties:
i) it is isotonic to animal or human plasma or blood,
ii) the volume occupied by water in the composition or at least one constituent of the composition is larger than the volume occupied by the at least one nanoparticle or at least one constituent of the composition in the composition or at least one constituent of the composition, and iii) the percentage in mass of water in the composition or at least one constituent of the composition is larger than the percentage in mass of the at least one nanoparticle or at least one constituent of the composition in the composition or at least one constituent of the composition.

In one embodiment of the invention, the at least one nanoparticle or at least one constituent of the composition is in liquid suspension when it is mixed with a liquid. In some cases, the liquid can be water, an isotonic liquid, a liquid comprising an excipient, a surfactant, or an oil.

In some cases, the composition or at least one constituent of the composition is isotonic, *i.e.* it preferentially has:
iv) the same osmotic pressure as that of a body part such as a cell, a body fluid, plasma, blood, preferentially of a human or animal,
v) the value of abs(OP_{BP} - OP_{Comp})/OP_{BP} is lower than 100, 50, 10, 5, 2, 1 or 10⁻³%, where OP_{BP} and OP_{Comp} are the osmotic pressures of the body part and composition or at least one constituent of the composition, respectively.

In another embodiment of the invention, OP_{BP} and/or OP_{Comp} is/are larger than 10⁻¹⁰, 10⁻⁵, 10⁻³, 10-1, 0, 1, 5, 10, 50 or 100 bar or atm.

In still another embodiment of the invention, OP_{BP} and/or OP_{Comp} is/are smaller than 10¹⁰, 10⁵, 10³, 10, 0, 10⁻¹, 10⁻³, 10⁻⁵, or 10⁻¹⁰ bar or atm.

In one embodiment, the value of abs(OP_{BP} - OP_{Comp})/OP_{BP} is kept low or the composition or at least one constituent of the composition is maintained isotonic to avoid blood pressure increase, preferentially during or after administration of the composition or at least one constituent of the composition to the body part.

In some cases, the composition or at least one constituent of the composition can be hypertonic, *i.e.* that preferentially in this case, the composition or at least one constituent of the composition causes at least one cell or body part to shrink.

In some other cases, the composition or at least one constituent of the composition can be hypotonic, *i.e.* that preferentially in this case, the composition or at least one constituent of the composition causes at least one cell or body part to swell.

In still some other cases, the composition or at least one constituent of the composition can be isotonic, *i.e.* that preferentially in this case, the composition or at least one constituent of the composition produces no change in cell or body part volume or less change in cell or body part volume than for hypertonic or hypotonic composition or at least one constituent of the composition.

In one embodiment of the invention, the solutes or solute compounds in the composition or at least one constituent of the composition, preferentially the nanoparticle or at least one constituent of the composition, chain of nanoparticle or at least one constituent of the composition and/or cryoprotectant or protectant compound have a concentration, preferentially designated as Cₛₒₗᵤₜₑ, preferentially designated as C_{solute,outside} outside a cell or outside a body part, preferentially designated as C_{solute,inside} inside a cell or inside a body part, which is such that abs(C_{solute,outside} - C_{solute,inside}) / C_{solute,outside} is lower than or equal to 100, 50, 25, 10, 5, 2, 1, 0, 10⁻¹, 10⁻³ or 10⁻⁵%.

In some other cases, (C_{solute,outside} - C_{solute,inside}) / C_{solute,outside} is larger than or equal to 10⁻⁵, 10⁻³, 10⁻¹, 0, 1, 2, 5, 10, 25, 50, 75 or 99%.

In one embodiment of the invention, the composition or at least one constituent of the composition is isosmotic or the osmolarity or osmotic pressure of the composition or at least one constituent of the composition, preferentially outside at least one cell or body part is the same as the osmolarity or osmotic pressure of an intracellular medium or body part or composition or at least one constituent of the composition comprised inside at least one cell or body part.

In some cases, the body part can designate a liquid or liquid medium comprised in a body part such as the intracellular medium or plasma or blood.

In some cases, the composition or at least one constituent of the composition is comprised in the body part, preferentially after or with administration to the body part.

In some other cases, the composition or at least one constituent of the composition is comprised outside the body part, preferentially before or without administration to the body part.

In some cases, the composition or at least one constituent of the composition can be hypoosmotic.

In some other cases, the composition or at least one constituent of the composition can be hyperosmotic. In one embodiment of the invention, the osmolality or osmolarity of the composition or at least one constituent of the composition is larger than or equal to 10⁻¹⁰, 10⁻⁵, 10⁻³, 10⁻¹, 0, 1, 5, 10, 50, 100, 200, 250, 290, 300, 310, 350, 400, 500, 10³, 10⁵ or 10¹⁰ mOsm/kg or mOsm/L.

In another embodiment of the invention, the osmolality or osmolarity of the composition or at least one constituent of the composition is lower than or equal to 10¹⁰, 10⁵, 10³, 500, 350, 310, 300, 250, 200, 100, 10, 5, 1, 0, 10³, 10⁻⁵ or 10⁻¹⁰ mOsm/kg or mOsm/L.

In another embodiment of the invention, the osmolality of the composition or at least one constituent of the composition is equal to, close to, or does not differ by more than 1, 5, 10, 50, or 100% from the human plasma osmolality, preferentially comprised between 275 and 299 milli-osmoles per kilogram. In another embodiment of the invention, the hydrostatic pressure or osmotic pressure or arterial pressure or systolic pressure or diastolic pressure or pressure of the composition or at least one constituent of the composition or in the presence of the composition or at least one constituent of the composition or following the administration of the composition or at least one constituent of the composition or applied by the composition or at least one constituent of the composition on the body part or the force pushing the composition or at least one constituent of the composition or at least one compound or solute of the composition or at least one constituent of the composition preferentially per unit surface of the body part, preferentially in some cases from outside the body part or at least one cell of the body part to inside the body part or inside at least one cell of the body part, preferentially in some other cases from inside the body part or inside at least one cell of the body part to outside the body part or outside at least one cell of the body part, is designated as ξ.

In some cases, ξ can be lower than or equal to 10⁵, 10³, 500, 200, 180, 150, 140, 100, 50, 20, 10, 1, 0, 10 ', 10⁻³ or 10⁻⁵ mmHg or PSI or bar or mbar.

In some other cases, ξ be larger than or equal to 10⁻⁵, 10⁻³, 10⁻¹, 0, 1, 5, 10, 100, 120, 150, 500, 10³, 10⁵, or 10¹⁰ mmHg or PSI or bar or mbar.

In one embodiment of the invention, the volume occupied by water or a liquid and preferentially the cryoprotectant or protectant compound in the composition or at least one constituent of the composition is larger than the volume occupied by the at least one nanoparticle or at least one constituent of the composition or chain in the composition or at least one constituent of the composition. In this case, the at least one nanoparticle or at least one constituent of the composition or chain is preferentially suspended in a liquid, preferentially water, which comprises the cryoprotectant or protectant compound. In this case, the volume of liquid is preferentially maintained sufficiently large or larger than the volume occupied by the at least one nanoparticle or at least one constituent of the composition or chain in the composition or at least one constituent of the composition to enable the suspension of the at least one chain in the composition or at least one constituent of the composition or motion or Brownian motion of the at least one nanoparticle or at least one constituent of the composition or chain in the composition or at least one constituent of the composition.

In some cases, the volume occupied by water or liquid and preferentially the cryoprotectant or protectant compound in the composition or at least one constituent of the composition is larger than the volume occupied by the at least one nanoparticle or at least one constituent of the composition or chain in the composition or at least one constituent of the composition, by a factor α of at least 1.1, 2, 5, 10 or 10³. In this case, the composition or at least one constituent of the composition is preferentially in liquid form.

In some other cases, the volume occupied by water or liquid and preferentially the cryoprotectant or protectant compound in the composition or at least one constituent of the composition is lower than the volume occupied by the at least one nanoparticle or at least one constituent of the composition or chain in the composition or at least one constituent of the composition, by a factor α of at least 1.1, 2, 5, 10 or 10³. In this case, the composition or at least one constituent of the composition is preferentially in powder form.

In some cases, α can be equal to V_{liq}/V_{chain}, where V_{liq} and V_{chain} are the volumes occupied by the liquid preferentially comprising the cryoprotectant or protectant compound and at least one nanoparticle or at least one constituent of the composition or chain in the composition or at least one constituent of the composition, respectively, where the two volumes can preferentially be measured by separating the at least one nanoparticle or at least one constituent of the composition or chain from the liquid, using for example by magnetic separation or using a magnet to attract the nanoparticle or at least one constituent of the composition or chains and separate them from the liquid.

In another embodiment of the invention, the mass of water or liquid and preferentially cryoprotectant or protectant compound in the composition or at least one constituent of the composition is larger than the mass of at least one nanoparticle or at least one constituent of the composition or chain in the composition or at least one constituent of the composition. In this case, the at least one nanoparticle or at least one constituent of the composition or chain is preferentially suspended in a liquid, preferentially water, which comprises the cryoprotectant or protectant compound. In this case, the mass of liquid is preferentially maintained sufficiently large or larger than the mass of the at least one nanoparticle or at least one constituent of the composition or chain in the composition or at least one constituent of the composition to enable the suspension of the at least one nanoparticle or at least one constituent of the composition or chain in the composition or at least one constituent of the composition or motion or Brownian motion of the at least one nanoparticle or at least one constituent of the composition or chain in the composition or at least one constituent of the composition.

In some cases, the mass of water or liquid and preferentially cryoprotectant or protectant compound in the composition or at least one constituent of the composition is larger than the mass of the at least one nanoparticle or at least one constituent of the composition or chain in the composition or at least one constituent of the composition, by a factor α of at least 1.1, 2, 5, 10 or 10³. In this case, the composition or at least one constituent of the composition is preferentially in liquid form.

In some other cases, the mass of water or liquid and preferentially cryoprotectant or protectant compound in the composition or at least one constituent of the composition is lower than the mass of the at least one nanoparticle or at least one constituent of the composition or chain in the composition or at least one constituent of the composition, by a factor α of at least 1.1, 2, 5, 10 or 10³. In this case, the composition or at least one constituent of the composition is preferentially in powder form.

In some cases, α can be equal to m_{liq}/m_{chain}, where m_{liq} and m_{chain} are the masses of the liquid preferentially comprising the cryoprotectant or protectant compound and at least one nanoparticle or at least one constituent of the composition or chain in the composition or at least one constituent of the composition, respectively, where the two masses can preferentially be measured by separating the at least one nanoparticle or at least one constituent of the composition or chain from the liquid, using for example magnetic separation or using a magnet to attract the nanoparticle or at least one constituent of the composition or chains and separate them from the liquid, where m_{chain} and/or m_{liq} can preferentially be measured by or following evaporation of the liquid from the composition or at least one constituent of the composition or lyophilization or desiccation of the composition or at least one constituent of the composition.

In one embodiment of the invention, the percentage in mass of water or liquid, preferentially comprising the cryoprotectant or protectant compound, preferentially not comprising the at least one nanoparticle or at least one constituent of the composition or chain, in the composition or at least one constituent of the composition, is larger than the percentage in mass of the at least one nanoparticle or at least one constituent of the composition or chain in the composition or at least one constituent of the composition. In some cases, the percentage in mass of water or liquid preferentially comprising the cryoprotectant or protectant compound, preferentially not comprising the at least one nanoparticle or at least one constituent of the composition or chain, in the composition or at least one constituent of the composition is larger than the mass of the at least one nanoparticle or at least one constituent of the composition or chain in the composition or at least one constituent of the composition, by a factor α of at least 1.1, 2, 5, 10 or 10³. In this case, the composition or at least one constituent of the composition is preferentially in liquid form.

In one embodiment of the invention, the percentage in mass of water or liquid, preferentially comprising the cryoprotectant or protectant compound, preferentially not comprising the at least one nanoparticle or at least one constituent of the composition or chain, in the composition or at least one constituent of the composition, is smaller than the percentage in mass of the at least one nanoparticle or at least one constituent of the composition or chain in the composition or at least one constituent of the composition by a factor α of at least 1.1, 2, 5, 10 or 10³. In this case, the composition or at least one constituent of the composition is preferentially in powder form.

In some cases, α can be equal to P_{liq}/P_{chain}, where P_{liq} and P_{chain} are the percentages in masses of the liquid preferentially comprising the cryoprotectant or protectant compound and at least one nanoparticle or at least one constituent of the composition or chain in the composition or at least one constituent of the composition, respectively, where the two percentages in masses can preferentially be measured by separating the at least one nanoparticle or at least one constituent of the composition or chain from the liquid, using for example magnetic separation or using a magnet to attract the nanoparticle or at least one constituent of the composition or chains and separate them from the liquid, where P_{chain} and/or P_{liq} can preferentially be measured by or following evaporation of the liquid from the composition or at least one constituent of the composition or lyophilization or desiccation of the composition or at least one constituent of the composition.

The invention also relates to the composition or at least one constituent of the composition according to the invention, where the at least one nanoparticle or at least one constituent of the composition or chain is in powder form, and the composition or at least one constituent of the composition has preferentially at least one of the following properties:
i) preferentially the volume occupied by water or liquid and preferentially the cryoprotectant or protectant compound in the composition or at least one constituent of the composition is smaller than the volume occupied by the at least one nanoparticle or at least one constituent of the composition or chain in the composition or at least one constituent of the composition, and
ii) preferentially the percentage in mass of water or liquid and preferentially the cryoprotectant or protectant compound in the composition or at least one constituent of the composition is smaller than the percentage in mass of the at least one nanoparticle or at least one constituent of the composition or chain in the composition or at least one constituent of the composition,
wherein the composition or at least one constituent of the composition is preferentially lyophilized, desiccated, dried or dehydrated.

In some cases, the composition or at least one constituent of the composition is lyophilized, desiccated, dried or dehydrated when water or liquid is removed, preferentially partly or fully, from the composition or at least one constituent of the composition.

The invention also relates to the composition or at least one constituent of the composition according to the composition or at least one constituent of the composition, in which the composition or at least one constituent of the composition is lyophilized or desiccated or dried or dehydration or treated by lyophilization or desiccation or water removal or liquid removal or dehydration.

In one embodiment of the invention, the composition or at least one constituent of the composition is dehydrated or water or liquid is removed or absent from the composition or at least one constituent of the composition. In this case, the percentage in mass of water or liquid in the composition or at least one constituent of the composition is preferentially larger before the composition or at least one constituent of the composition is dehydrated or before water or liquid is removed from the composition or at least one constituent of the composition than after the composition or at least one constituent of the composition is dehydrated or after water or liquid is removed from the composition or at least one constituent of the composition. In this case, the volume of water or liquid occupied in the composition or at least one constituent of the composition is preferentially larger before the composition or at least one constituent of the composition is dehydrated or before water or liquid is removed from the composition or at least one constituent of the composition than after the composition or at least one constituent of the composition is dehydrated or after water or liquid is removed from the composition or at least one constituent of the composition. In this case, water or liquid is removed from the composition or at least one constituent of the composition without removing the at least one nanoparticle or at least one constituent of the composition or chain and preferentially also the cryoprotectant or protectant compound from the composition or at least one constituent of the composition.

In one embodiment of the invention, the percentage in mass of water in the composition or at least one constituent of the composition is larger than 10⁻³, 10 ', 0, 1, 5, 10, 25, 50, 80 or 99%, preferentially before water is or has been removed from the composition or at least one constituent of the composition. In another embodiment of the invention, the percentage in mass of water in the composition or at least one constituent of the composition is lower than or equal to 100, 99, 80, 50, 25, 10, 5, 2, 1 or 0%, preferentially after water is or has been removed from the composition or at least one constituent of the composition.

In another embodiment of the invention, the composition or at least one constituent of the composition is lyophilized or the composition or at least one constituent of the composition is first cooled down, preferentially below or at 103, 100, 50, 10, 0, -10, -50, -77, -200, -273 °C, and second water or liquid is removed from the composition or at least one constituent of the composition, preferentially without removing the at nanoparticle or at least one constituent of the composition or least one nanoparticle or at least one constituent of the composition or chain and/or cryoprotectant or protectant compound from the composition or at least one constituent of the composition.

In another embodiment of the invention, the composition or at least one constituent of the composition is lyophilized, dehydrated, dried and/or desiccated, and water or liquid is preferentially removed from the composition or at least one constituent of the composition preferentially without removing the at least one nanoparticle or at least one constituent of the composition or chain and/or cryoprotectant or protectant compound from the composition or at least one constituent of the composition.

In one embodiment, the composition or at least one constituent of the composition is comprised in a tablet or is a tablet form or powder form, preferentially after water has been removed from the composition or at least one constituent of the composition.

In one embodiment, the composition or at least one constituent of the composition is stoked or stored or unused, preferentially for more than 0, 1, 10, 10², 10³, 10⁵, or 10¹⁰ hour(s), day(s), month(s), year(s), preferentially without being used or administered to a body part, preferentially in powder form, preferentially without losing at least one its property or without losing its nanoparticle or at least one constituent of the composition coating or nanoparticle or at least one constituent of the composition core, partly or fully.

In one embodiment of the invention, the moisture or water content of the composition or at least one constituent of the composition is lower than or equal to 100, 75, 50, 25, 10, 5, 4, 2, 1 or 0% w/w, preferentially weight of water for weight of composition or at least one constituent of the composition or weight of water by weight of composition or at least one constituent of the composition or mass of water for mass of composition or at least one constituent of the composition or mass of water by mass of composition or at least one constituent of the composition, preferentially after water has been removed from the composition or at least one constituent of the composition.

In another embodiment of the invention, the moisture or water content of the composition or at least one constituent of the composition is larger than or equal to 0, 1, 2, 4, 5, 10, 25, 50, 75 or 100% w/w, preferentially weight of water for weight of composition or at least one constituent of the composition or weight of water by weight of composition or at least one constituent of the composition or mass of water for mass of composition or at least one constituent of the composition or mass of water by mass of composition or at least one constituent of the composition, preferentially before water has been removed from the composition or at least one constituent of the composition.

The invention also relates to the composition or at least one constituent of the composition according to the invention, where the at least one nanoparticle or at least one constituent of the composition or chain and preferentially the cryoprotectant or protectant compound, which is/are preferentially in powder form, is/are suspended or resuspended in a liquid or water or treated by suspension or resuspension in a liquid or water, preferentially more than 1, 2, 5 or 10 times. In this case, water is preferentially removed from the composition or at least one constituent of the composition before the composition or at least one constituent of the composition is suspended or resuspended in a liquid or water.

The invention also relates to the composition or at least one constituent of the composition according to the invention, wherein the coating or at least one constituent of the composition or at least one of its chemical functions or atoms preferentially forms a first type of interaction or chemical bond with an atom or chemical function of the core, wherein the cryoprotectant or protectant compound or at least one of its chemical functions or atoms preferentially forms a second type of interaction or chemical bond with the coating or at least one of its chemical functions or atoms,
wherein the first type of interaction or chemical bond preferentially maintains at least two nanoparticle or at least one constituent of the compositions of the composition or at least one constituent of the composition arranged in chains or at least one center of activity or free radical capture/production comprised in the nanoparticle or at least one constituent of the composition above -100, -10, 0, 5, 10 or 50 °C,
wherein the first and second types of interaction or chemical bond preferentially maintains at least two nanoparticle or at least one constituent of the compositions of nanoparticle or at least one constituent of the compositions arranged in chains at or at least one center of activity or free radical capture/production comprised in the nanoparticle or at least one constituent of the composition below 100, 50, 10, 2, 0, -10, -50, -77, -100, -200 or -273 °C,
wherein the first type of interaction or chemical bond is preferentially different from the second type of interaction or chemical bond,
wherein the first type of interaction or chemical bond is preferentially selected in the group consisting of: i) an electrostatic interaction or bond, *i.e.* preferentially due to the difference in charge between the coating and the surface or core of the central part, ii) a hydrophobic interaction or bond, iii) a Chelating interaction or bond, iv) a metallic interaction or bond, and v) a Covalent interaction or bond,
wherein the second type of interaction or chemical bond is preferentially a hydrogen, London or Van Der Walls interaction or bond,
wherein optionally, the coating or coating material or at least one constituent of the composition comprises a chemical function, preferentially selected in the group consisting of i) OH , ii) NH₂, iii) COOH, iv) Thiol, v) Phosphate, and a basic or acid derivative of at least one of these functions.
wherein optionally, the core or at least one constituent of the composition comprises a chemical function, preferentially located at the surface of the core of the nanoparticle or at least one constituent of the composition, preferentially a hydroxyl group (Fe-OH).

The invention also relates to the composition or at least one constituent of the composition according to the invention, wherein the coating or at least one constituent of the composition is selected in the group of compounds consisting of: 1) citric acid, 2) oleic acid, 3) polymethacrylic acid, 4) poly(ethyleneoxide)-b-poly(methacrylic) acid), 5) polyacrylic acid (PAA), 6) polylactic acid, 7) poly(ethylene oxide)-blockpoly(glutamic) acid, 8) Phosphonic acid 9) Albumin, 10) Alendronate, 11) Alginate, 12) Au, Al₂O₃, 13) Aluminium, 14) Aluminium hydroxide, 15) Arabinogalactan, 16) Bentonite, 17) Carboxymethylcellulose, 18) Cellulose, 19) Chitosan, 20) Cholseterol, 21) Citrate, 22) Dextran, 23) Dimercaptosuccinic acid, 24) Dopamine, 25) DOPC or Dioleoylphosphatidylcholine or phospholipd, 26) DTAP or Di(tert.-amyl)peroxide, 27) DVB or Divinylbenzène, 28) Ethylcellulose, 29) Erythrocyte, 30) at least one fatty acids, 31) Ferrite, 32) Folic acid, 33) Gelatin, 34) serum albumin, preferentially human, 35) Liposome, 36) MIPS or Inositol-3-phosphate synthase, 37) MnO or manganese oxide, 38) Mn₃O₄, 39) Oleic acid, 40) at least one polymer or enantiomer, 41) PEI or Polyetherimide, 42) PEG or Polyethylene glycol, 43) Poly(ethylene oxide) or PEO, 44) PGA or Polyglycolic acid, 45) PLA or poly(lactide acid), 46) PLGA or PLG or poly(lactic-co-glycolic acid), 47) Phosphatidylcholine, 48) Phosphorylcholine, 49) Pluronic, 50) Polyacrylamide, 51) Polyacrylic acid or PAA, 52) Polyaniline, 53) Polyethylene glycol with/without terminal carboxyl groups, 54) Peptide or Polypeptide, 55) Poly(vinyl alcohol) or PVA, 56) Ploy(N-isopropylacrylamide) or PIA, 57) Poly(vinylpyrrolidone) or PVP, 58) Poly(oligoethylene oxide) or POO, 59) Poly(N,N-dimethyl ethylamino acrylate, 60) Poly(imine), 61) Poly(acrylic acid), 62) Poly-D-L lactide, 63) Polyalkylcyanoacrylate, 64) Polymer such as PAMAM or Poly(amidoamine) or PDMAEMA or poly(2-(dimethylamino)ethyl methacrylate) or PPEGMA or Poly (ethylene glycol) methyl ether methacrylate, 65) Poly NIPAAM or Poly (N-isopropylacrylamide) or temperature-responsive polymer or temperature-sensitive polymer 66) Polyacrylic acid, 67) Polydipyrrole or dicarbazole, 68) Poly-L-lysine, 69) Polymethylmethaacrylate, 70) Polymersome, 71) Polystyrene, 72) PVA or Polyvinyl Alcohol, 73) PVP or Polyvinylpyrrolidone, 74) Silica, preferentially amorphous or mesoporous, 75) Silane, 76) SiO₂, 77) Sodium Oleate, 78) Starch, 79) styrene, preferentially styrene-divinylbenzene, 80) TaOx, 81) ZrO₂, 82) at least one metal or semi-metal, 83) at least one metal oxide or semi-metal oxide, 84) at least one alkali metal, 85) at least one alkaline earth metal, 86) at least one transition metal, 87) at least one post-transition metal, 88) at least one metalloid, 89) at least one lanthanide, 90) at least one actinide, 91) at least one non-metal, 92) at least one halogen, 93) at least one noble gas, and 94) any derivative or combination of any of these compounds.

The invention also relates to the composition or at least one constituent of the composition according to the invention, wherein the coating or at least one constituent of the composition is selected in the group of family of compounds consisting of: i) Polysacharides such as Agarose, alginate, carregeenan, chitosan, dextran, haparin, Gum Arabic, Pullulan and Starch, ii) Acids such as citric acids, oleic acids, polymethacrylic acid, poly(ethyleneoxide)-b-poly(methacrylic acid, polyacrylic or PAA acid, polylactic acid, poly(ethylene oxide)-blockpoly(glutamic acid, Phosphonic acid, Dimercaptosuccinic acid, fatty acid, folic acid, oleic acid, poly(lactide acid or PLA, PAA or Polyacrylic acid, compounds comprising at least one carboxylic function, iii) Polymers such as Dextran, Poly(ethylene oxide), Poly(vinyl alcohol), Ploy(N-isopropylacrylamide), Poly(vinylpyrrolidone), Poly(oligoethylene oxide), Poly(N,N-dimethyl ethylamino acrylate), Poly(imine), Poly(acrylic acid), iv) Carboxylates, v) inorganic compounds such as SiO₂, Al₂O₃, ZrO₂, ferrites, MnO, Mn₃O₄, Au, Bentonite, Carbon such as inactivated, activated, graphitized carbon, vi) at least one metals, vii) organic compounds such as MIP, Cellulose, DV8, Ppy, Chitosan, Polyacrylamide, alginate, PEI, surfactants, viii) compounds comprising Phosphate, ix) compounds comprising Silica, x) compounds comprising Gold, xi) compounds comprising Dextran based, xii) compounds comprising PEG, xiii) compounds comprising PVA, xiv) compounds comprising Alginate, xv) compounds comprising Chitosan, xvi) compounds comprising the chemical function Alcohol, xvii) compounds comprising the chemical function Amide, and xviii) compounds comprising the chemical function Aldehyde.

The invention also relates to the composition or at least one constituent of the composition according to the invention, wherein the coating or at least one constituent of the composition has at least one chemical group selected in the group of i) OH⁻, ii) NH₂, iii) COOH, iv) Thiol, v) Phosphate, and a basic or acid derivative of at least one of these compounds.

The invention also relates to the composition or at least one constituent of the composition according to the invention, wherein the coating or coating material or at least one constituent of the composition comprises a chemical function, preferentially selected in the group consisting of i) OH-, ii) NH2, iii) COOH, iv) Thiol, v) Phosphate, and a basic or acid derivative of at least one of these functions, which has an interaction or forms a chemical bond with an atom or chemical group located at the surface of the core, preferentially a hydroxyl group (Fe-OH), where the interaction or bond is preferentially selected in the group consisting of: i) an electrostatic interaction or bond, i.e. preferentially due to the difference in charge between the coating and the surface or core of the core, ii) a hydrophobic interaction or bond, iii) a Chelating interaction or bond, iv) a metallic interaction or bond, v) a Covalent interaction or bond.

In one embodiment of the invention, the cryoprotectant or protectant compound interacts or forms with the core or coating of the nanoparticle or at least one constituent of the composition hydrogen or van der walls or London bonds or interaction, preferentially when water molecules are displaced or cooled down.

In one embodiment of the invention, the cryoprotectant or protectant compound is not used to protect the at least one nanoparticle or at least one constituent of the composition or at least one chain from the denaturation and/or destruction and/or loss of primary, secondary, tertiary or quaternary structure of DNA, RNA, protein(s), lipid(s), enzyme(s), or at least one biological molecule.

In one embodiment of the invention, the cryoprotectant or protectant compound prevents a change, decrease, increase of the size or of at least one nanoparticle or at least one constituent of the composition or chain by more than 100, 50, 10 or 1%, where this percentage is preferentially equal to S2-S1/S1 or P2-P1/P1, where S1, P1, S2 and P2 are preferentially the sizes and properties of at least one nanoparticle or at least one constituent of the composition or chain before (S1, P1) and after (S2, P2) cooling down the at least one nanoparticle or at least one constituent of the composition or chain preferentially by more than 1, 50 or 100°C, preferentially from an initial temperature, preferentially above -273, -100, - 50, 0, 5, or 10°C, preferentially down to a final temperature, preferentially lower than 100, 50, 0 or -50 °C.

In some cases, the property of at least one nanoparticle or at least one constituent of the composition or chain can be: i) the percentage in mass of at least one metal in the at least one nanoparticle or at least one constituent of the composition or chain, ii) the number of nanoparticle or at least one constituent of the composition in the chain, iii) the surface charge of the at least one nanoparticle or at least one constituent of the composition or chain, iii) the percentage of water in the at least one nanoparticle or at least one constituent of the composition or chain, iv) the coercivity or remanent magnetization of the at least one nanoparticle or at least one constituent of the composition or chain, v) the core diameter of the at least one nanoparticle or at least one constituent of the composition, vi) the coating thickness of the at least one nanoparticle or at least one constituent of the composition, vii) the isotonicity or osmolality of the at least one nanoparticle or at least one constituent of the composition or chain.

In one embodiment of the invention, as the cryoprotectant or protectant compound replaces the water molecules, preferentially when the composition or at least one constituent of the composition is cooled down, the coating and/or core of the nanoparticle or at least one constituent of the composition preferentially retains its structure and function and/or at least two nanoparticle or at least one constituent of the compositions remain arranged in chain and/or the at least one center of activity or free radical capture/production remains comprised in the nanoparticle or at least one constituent of the composition. In one embodiment, the composition or at least one constituent of the composition comprising the cryoprotectant or protectant compound does not comprise ice, while the composition or at least one constituent of the composition not comprising the cryoprotectant or protectant compound comprises ice or the composition or at least one constituent of the composition comprising the cryoprotectant or protectant compound comprises more ice than the composition or at least one constituent of the composition not comprising the cryoprotectant or protectant compound, preferentially when the composition or at least one constituent of the composition is cooled down and/or be lyophilized,

In one embodiment of the invention, the composition or at least one constituent of the composition comprising the cryoprotectant or protectant compound has a lower melting point than the composition or at least one constituent of the composition not comprising the cryoprotectant or protectant compound. In one embodiment of the invention, the cryoprotectant or protectant compound is a nonpermeable cryoprotectant or protectant compound.

In some cases, the cryoprotectant or protectant compound can be selected among: a sugar, trehalose, sucrose, starch, hydroxyethyl starch, polyvinyl pyrrolidone, and/or polyethylene oxide.

Preferentially, a nonpermeable cryoprotectant or protectant compound does not enter cells or coatings and thus preferentially stays extracellular or outside the coating when the composition or at least one constituent of the composition is cooled down.

In some cases, the composition or at least one constituent of the composition is cooled during or for cryopreservation of the composition or at least one constituent of the composition.

Preferentially, a nonpermeable cryoprotectant or protectant compound is used to protect cells during the cooling of the composition or at least one constituent of the composition.

Preferentially, when a cooling or slow cooling or slow freezing rate is applied to the composition or at least one constituent of the composition or the composition or at least one constituent of the composition is cooled down, preferentially slowly, there is sufficient time for water or liquid comprised in the nanoparticle or at least one constituent of the composition preferentially in the coating of the nanoparticle or at least one constituent of the composition to move out of the coating, preferentially under the osmotic pressure, preferentially resulting in a reduction in the volume of the coating. In the absence of a cryoprotectant or protectant compound, such behavior can destroy or damage the coating and/or result in the destruction of the nanoparticle or at least one constituent of the composition chain arrangement or of the center of activity or free radical/production.

In one embodiment, a cryoprotectant or protectant compound, preferentially a permeable cryoprotectant or protectant compound can enter a cell or the coating and preferentially preserve the cell or coating, preferentially from the osmotic pressure or damage or destruction.

In some cases, the cryoprotectant or protectant compound can be dimethyl sulfoxide (DMSO), glycerol, ethylene glycol, or propylene glycol.

In some cases, the cryoprotectant or protectant compound can be used to induce the vitrification of intracellular environment or of the coating or of the inner part or surface part of the coating or nanoparticle or at least one constituent of the composition or core, preferentially before ice crystal formation, preferentially in the composition or at least one constituent of the composition, preferentially preventing excessive loss volume of cell or coating or nanoparticle or at least one constituent of the composition or core.

In one embodiment of the invention, the cryoprotectant or protectant compound is selected in the group consisting of: i) natural product, ii) soybean flour product, iii) carbohydrate, iv) lipid, v) saccharide, vi) zwitterionic molecule, vii) 1-carnitine, and viii) antifreeze compounds such as antifreeze proteins.

The invention also relates to the composition or at least one constituent of the composition according to the invention, wherein the coating or at least one constituent of the composition or at least one of its chemical functions or atoms forms a first type of interaction or chemical bond with an atom or chemical function of the core, wherein the cryoprotectant or protectant compound or at least one of its chemical functions or atoms forms a second type of interaction or chemical bond with the coating or at least one of its chemical functions or atoms,
wherein the first type of interaction or chemical bond is preferentially different from the second type of interaction or chemical bond,
wherein the first type of interaction or chemical bond is preferentially selected in the group consisting of: i) an electrostatic interaction or bond, *i.e.* preferentially due to the difference in charge between the coating and the surface or core, ii) a hydrophobic interaction or bond, iii) a Chelating interaction or bond, iv) a metallic interaction or bond, and v) a Covalent interaction or bond,
wherein the second type of interaction or chemical bond is preferentially a hydrogen, London or Van Der Walls interaction or bond.

In some cases, the coating or coating material or at least one constituent of the composition can comprise a chemical function, preferentially selected in the group consisting of i) OH , ii) NH₂, iii) COOH, iv) Thiol, v) Phosphate, and a basic or acid derivative of at least one of these functions.

In some other cases, the core or at least one constituent of the composition can comprise a chemical function, preferentially located at the surface of the core of the nanoparticle or at least one constituent of the composition, preferentially a hydroxyl group (Fe-OH).

The invention also relates to the composition or at least one constituent of the composition according to the composition or at least one constituent of the composition, wherein the cryoprotectant or protectant compound or at least one constituent of the composition is selected in the group consisting of: 1) Acetamide, 2) Acetate, 3) Albumin, 4) Amino acids, 5) Ammonium acetate, 6) Arginine, 7) Alcohols containing at least one or two hydroxyl groups, 8) Bridger, 9) Choline magnesium chloride sodium bromide, 10) Diethyl glycol, 11) Dimethylacetamide, 12) Dimethyl sulfoxide (DMSO), 13) Disaccharide, 14) Erythritol, 15) Ethanol, 16) Ethylene glycol, 17) Formamide, 18) Fructose, 19) Glucose, 20) Glycerol, 21) Glycerol 3-phosphate, 22) Glycol such as Diethyl glycol or Triethylene glycol, 23) Glycine, 24) Lactose, 25) L-tyrosine, 26) lysine hydrochloride, 27) Mannitol, 28) MDP (2-Methyl-2,4-pentanediol), 29) Phenylalanine, 30) Planic, 31) Polymers, 32) Polyethylene glycol such as PEG4000, polyethylene glycol succinate, folate modified distearoylphosphatidyl ethanolamine-polyethylene glycol, 33) Polyethyleneimine (PEI), 34) Polyvinylpyrrolidone (PVP), 35) Proline, 36) Propylene glycol, 37) Protein, 38) Pyridine (Pyridine-N-Oxide), 39) Ribose, 40) Sarcosine, 41) Serine, 42) Serum albumin, 43) Sodium bromide, 44) Sodium chloride, 45) Sodium dodecyl sulfonate, 46) Sodium glutamate, 47) Sodium iodide, 48) Sodium sulfate, 49) Sorbitol, 50) Starch (hydroxyethyl starch), 51) Sugar, 52) Sucrose, 53) The cell bank series, 54) Trehalose, 55) Triethylene glycol, 56) Trimethylamine, 57) Tween 80, 58) Tryptophan, 59) Valine, 60) Xylose, and 61) a combination or derivative of any of these compounds.

In one embodiment of the invention, the composition or at least one constituent of the composition is desiccated or lyophilized or dehydrated or dried. In this case, the percentage in mass of water or liquid in the composition or at least one constituent of the composition is preferentially smaller than 100, 75, 50, 75, 20, 10, 5, 2, 1, 0, 10 ', 10⁻³ or 10⁻⁵%. In this case, the quantity of water or liquid in the composition or at least one constituent of the composition is preferentially smaller than 100, 50, 10, 5, 2, 1, 10 ', 10 ³, or 10⁻⁵ grams of water or liquid in the composition or at least one constituent of the composition per gram of composition or at least one constituent of the composition.

In some other cases, the composition or at least one constituent of the composition can be non-desiccated, non-dehydrated, or non-dehydrated or dried. In this case, the percentage in mass of water or liquid in the composition or at least one constituent of the composition is preferentially larger than 10⁻⁵, 10⁻³, 10 ', 0, 1, 5, 10, 25, 50, 75 or 100%. In this case, the quantity of water or liquid in the composition or at least one constituent of the composition is preferentially larger than 10⁻¹⁰, 10⁻⁵, 10⁻¹, 0, 1, 5, 10 or 50, 10 grams of water or liquid in the composition or at least one constituent of the composition per gram of composition or at least one constituent of the composition.

The invention also relates to the composition or at least one constituent of the composition according to the invention, which is lyophilized or desiccated or dehydrated, wherein the percentage in mass of cryoprotectant or protectant compound is preferentially larger than the percentage in mass of coating in the lyophilized or desiccated or dehydrated composition or at least one constituent of the composition, wherein the lyophilized or desiccated or dehydrated composition or at least one constituent of the composition is preferentially more stable than non-lyophilized or non-desiccated or non-dehydrated composition or at least one constituent of the composition.

The invention also relates to the composition or at least one constituent of the composition, where the percentage in mass of the cryoprotectant or protectant compound is comprised between 0.5 and 50%.

In some cases, the percentage in mass of a substance comprised in the composition or at least one constituent of the composition, *e.g.* the cryoprotectant or protectant compound, nanoparticle or at least one constituent of the composition core, nanoparticle or at least one constituent of the composition coating, chain, liquid or water, is equal to or proportional to the mass of this substance divided by the mass of all the substances comprised in the composition or at least one constituent of the composition. In some cases, the percentage in mass of the cryoprotectant or protectant compound, nanoparticle or at least one constituent of the composition coating, nanoparticle or at least one constituent of the composition core, at least one chain, liquid or water, in the composition or at least one constituent of the composition is larger than 10⁻⁵, 10⁻³, 10⁻¹, 0, 1, 5, 10, 25, 50, 75, 90 or 99%.

In some other cases, the percentage in mass of the cryoprotectant or protectant compound, nanoparticle or at least one constituent of the composition coating, nanoparticle or at least one constituent of the composition core, at least one chain, liquid or water, in the composition or at least one constituent of the composition is lower than 100, 75, 50, 25, 10, 5, 2, 1, 0, 10⁻³ or 10⁻⁵%.

In one embodiment of the invention, the percentage in mass of cryoprotectant or protectant compound in the composition or at least one constituent of the composition according to the invention is larger than the percentage in mass of nanoparticle or at least one other constituent of the composition, coating, nanoparticle core, at least one chain, liquid and/or water.

In another embodiment of the invention, the percentage in mass of cryoprotectant or protectant compound in the composition or at least one constituent of the composition according to the invention is smaller than the percentage in mass of nanoparticle or at least one constituent of the composition coating, nanoparticle or at least one constituent of the composition core, at least one chain, liquid and/or water, in the composition or at least one constituent of the composition.

In one embodiment of the invention, the core of the nanoparticle or at least one constituent of the composition in the composition or at least one constituent of the composition according to the invention has at least one property selected in the group consisting of: a) it is ferrimagnetic, b) it is composed of maghemite or magnetite or of an intermediate composition or at least one constituent of the composition between maghemite and magnetite, c) it has a size comprised between 0.1 and 100 nm, and d) it comprises at least one crystallographic plane.

In another embodiment of the invention, the coating of the nanoparticle or at least one constituent of the composition in the composition or at least one constituent of the composition according to the invention has at least one property selected in the group consisting of: a) it has a thickness lower than 10 µm, b) it comprises a number of crystallographic planes per unit surface that is lower than the number of crystallographic planes per unit surface of the core, c) it has a non-neutral charge, d) it is amorphous and e) it is organic, and f) it has a thickness that is lower than 10 nm or than the diameter of the core. In another embodiment of the invention, the cryoprotectant or protectant compound in the composition or at least one constituent of the composition according to the invention is comprised in a matrix or volume embedding the core and/or coating of the nanoparticle or at least one constituent of the composition.

The invention also relates to the composition or at least one constituent of the composition according to the invention, wherein the core or nanoparticle or at least one constituent of the composition core is synthesized by a living organism or nanoparticle or at least one constituent of the composition producing cells, preferentially a magnetotactic bacterium.

In one embodiment of the invention, the nanoparticle or at least one constituent of the compositions are synthesized biologically or by a living organism, designated as synthetizing living organism, which preferentially consists or comprises at least 1, 2, 5, 10, 10³, 10⁶ or 10⁹ eukaryotic cell(s), prokaryotic cell(s), or part of these cells. In some cases, part of eukaryotic or prokaryotic cell(s) can be biological material originating or produced by these cells such as RNA, DNA, organelle, nucleolus, nucleus, ribosome, vesicle, rough endoplasmic reticulum, Golgi apparatus, cytoskeleton, smooth endoplasmic reticulum, mitochondrion, vacuole, cytosol, lysosome, centrosome, cell membrane. In some cases, a biological synthesis can be defined as a synthesis involving a majority of steps, or more than 1, 2, 5 or 10 steps, or more than 1, 2, 5, 25, 50, 75 or 90% of steps, which involve chemical reactions occurring with the involvement of at least 1, 2, 10, 10³, 10⁶ or 10⁹ living organisms, or parts of living organisms such as DNA, RNA, proteins, enzymes, lipids.

In some cases, the synthetizing living organism can be magnetotactic bacteria, other types bacteria than magnetotactic bacteria or enzymes of certain bacteria, preferentially synthetizing nanoparticle or at least one constituent of the compositions extra-cellularly, such as Mycobacterium paratuberculosis, Shewanella oneidensi, Geothrix fermentans, ants, fungi, or various plants.

In still another embodiment of the invention, the nanoparticle or at least one constituent of the compositions are synthesized or produced or crystallized or assembled or transformed into a nanoparticle or at least one constituent of the composition by a compartment, organelle, or other biological material, such as protein, lipid, enzyme, DNA, or RNA, which is preferentially produced by or originates from an eukaryotic or prokaryotic cell.

In another embodiment of the invention, the nanoparticle or at least one constituent of the compositions are synthesized by or in at least one eukaryotic cell, prokaryotic cell, or part of this cell.

In another embodiment of the invention, the nanoparticle or at least one constituent of the compositions are synthesized by or in: i) the matrix or medium or environment located outside of at least one eukaryotic cell, prokaryotic cell, or part of this cell, or ii) the extracellular matrix.

In one embodiment of the invention, the nanoparticle or at least one constituent of the compositions are synthesized by a living organism when at least 1, 2, 5, 10 or 100 step(s) of their production, such as crystallization of iron oxide, stabilization of the iron oxide mineral, organization of the nanoparticle or at least one constituent of the compositions, for example in chains or aggregates, involves or is due to a living organism.

The invention also relates to the nanoparticle or at least one constituent of the compositions for use, wherein the nanoparticle or at least one constituent of the compositions are magnetosomes synthesized by, originating from, extracted from, or isolated from magnetotactic bacteria.

In one embodiment of the invention, the magnetosome is synthesized by, produced by, originates from, extracted from, isolated from magnetotactic bacteria.

In one embodiment of the invention, magnetotactic bacteria are selected from the group consisting of: *Magnetospirillum magneticum* strain AMB-1, magnetotactic coccus strain MC-1, three facultative anaerobic vibrios strains MV-1, MV-2 and MV-4, the *Magnetospirillum magnetotacticum* strain MS-1, the *Magnetospirillum gryphiswaldense* strain MSR-1, a facultative anaerobic magnetotactic spirillum, *Magnetospirillum magneticum* strain MGT-1, and an obligate anaerobe, and *Desulfovibrio magneticus* RS-1.

In one embodiment of the invention, a magnetotactic bacterium is defined as a bacterium able to synthesize magnetosomes, wherein these magnetosomes are preferentially characterized by at least one of the following properties: i) they are produced intracellularly, ii) they are magnetic, iii) they comprise a mineral, iv) their core is preferentially composed of a metallic oxide such as iron oxide, v) their core is surrounded by biological material such as lipids, proteins, endotoxins, which can preferentially be removed, vi) they are arranged in chains, vii) they produce heat under the application of an alternating magnetic field.

In one embodiment of the invention, the magnetosomes possess one or several property(ies) in common with the nanoparticle or at least one constituent of the compositions such as at least one magnetic, size, composition or at least one constituent of the composition, chain arrangement, charge, core, mineral, coating, or crystallinity property.

In one embodiment of the invention, magnetosomes comprise the mineral part synthesized by magnetotactic bacteria, i.e. preferentially the crystallized iron oxide produced by these bacteria. In this case, magnetosomes or magnetosome mineral parts preferentially do not comprise proteins, lipids, endotoxins, or biological materials comprising carbon or do not comprise more or comprise less than 0.1, 1, 10, 30, 50 or 75% or percent in mass of carbon, which is/are produced by these bacteria.

In some cases, the photo-sensitizing or sono-sensitizing or radio-sensitizing strength is the concentration or quantity of radical species, preferentially free radical species, produced or captured by the at least one photo-sensitizer, radio-sensitizer, sono-sensitizer, center of activity or free radical capture or production, or composition or at least one constituent of the composition, or is or is proportional to the strength or intensity of the acoustic wave, ultrasound, radiation, light, electromagnetic radiation, preferentially applied on the photo-sensitizer, radio-sensitizer, sono-sensitizer, center of activity or free radical capture or production or composition or at least one constituent of the composition.

In one embodiment of the invention, C_{1FRPC} and C_{2FRPC} act anti-synergistically, *i.e.* that the photo-sensitizing or sono-sensitizing or radio-sensitizing strength of the nanoparticle or at least one constituent of the composition comprising C_{1FRPC} and C_{2FRPC} is lower than the photo-sensitizing or sono-sensitizing or radio-sensitizing strength of the nanoparticle or at least one constituent of the composition comprising only C_{1FRPC} or C_{2FRPC}.

In one embodiment of the invention, the composition or at least one constituent of the composition is introduced or administered to or in the body part, preferentially of an animal or human. Preferentially, following its introduction or administration, the nanoparticle or at least one constituent of the composition firstly comprises C_{1FRPC} and C_{2FRPC} during a time t₁ and secondly comprises C_{1FRPC} during a time t₂, where t₂ follows t₁.

In some cases, t₁ and/or t₂ can be longer than 10⁻¹⁰, 10⁻⁵, 10⁻³, 10⁻¹, 0, 1, 5, 10 or 10³ second(s) or minute(s).

In some other cases t₁ and/or t₂ can be shorter than 10¹⁰, 10⁵, 10³, 10, 0, 1,5, 10⁻¹ or 10⁻³ second(s) or minute(s).

In some cases, t₂ can be separated from t₁ by more than 10⁻¹⁰, 10⁻⁵, 10⁻³, 10⁻¹, 0, 1, 5, 10 or 10³ second(s) or minute(s).

In some other cases, t₂ can be separated from t₁ by less than 10¹⁰, 10⁵, 10³, 10, 0, 1, 5, 10⁻¹ or 10⁻³ second(s) or minute(s).

The invention also relates to the composition or at least one constituent of the composition according to the invention, where the composition or at least one constituent of the composition is or comprises: i) a medical device, ii) a drug, iii) a pharmaceutical product or preparation, iv) a medical product or preparation, iv) a biological product or preparation, vi) an adjuvant, vii) an excipient, viii) an active principle, ix) a vaccine or vaccine component, vi) a product, vii) a suspension, viii) a lyophilized suspension or composition or at least one constituent of the composition or preparation, ix) at least one chain of nanoparticle or at least one constituent of the composition, and/or x) at least one center of activity or free radical capture or production.

In one embodiment, the composition or at least one constituent of the composition according to the invention is used for/in: i) the treatment of an infection, ii) the treatment of a viral disease, iii) the treatment of a disease, preferentially a cancerous disease, iv) radiotherapy, v) photodynamic therapy, and/or vi) sonodynamic therapy.

In some cases, the disease can be due to the malfunction of an organ or body part preferentially of an individual.

In some cases, the treatment can be the therapy and/or the diagnosis of a disease or a cosmetic treatment. In some cases, the treatment can induce the death, destruction, denaturation, or inactivation of at least 1 biological material, such as cell, preferentially pathological cell, RNA, DNA, protein, lipid, or enzyme, where the death of cell can occur through apoptosis or necrosis.

The invention also relates to nanoparticle or at least one constituent of the compositions for use according to the invention, the disease is selected from the group consisting of: a disease associated with a proliferation of cells that is different from the cellular proliferation in a healthy individual, a disease associated with the presence of pathological cells in the body part, a disease associated with the presence of a pathological site in an individual or body part, a disease or disorder or malfunction of the body part, a disease associated with the presence of radio-resistant or acoustic-resistant cells, an infectious disease, an auto-immune disease, a neuropathology, a cancer, a tumor, a disease comprising or due to at least one cancer or tumor cell, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, anemia, preferentially iron anemia, and a personality disorder.

In some cases, the disease or disorder can be the disease or disorder of or belonging to the individual or body part, or the disease or disorder from which the individual is suffering.

In one embodiment of the invention, the cancer or tumor selected from the group consisting of: the cancer of an organ, cancer of blood, cancer of a system of a living organism, adrenal cancer, anal cancer, bile duct cancer, bladder cancer, bone cancer, brain cancer, breast cancer, cervical cancer, colon/rectum cancer, endometrial cancer, esophagus cancer, eye cancer, gallbladder cancer, heart cancer, kidney cancer, laryngeal and hypopharyngeal cancer, leukemia, liver cancer, lung cancer, nasal cavity and paranasal sinus cancer, nasopharyngeal cancer, neuroblastoma, non-Hodgkin lymphoma, oral cavity and oropharyngeal cancer, osteosarcoma cancer, ovarian cancer, pancreatic cancer, pancreatic penile cancer, prostate cancer, retinoblastoma, rhabdomyosarcoma, salivary gland cancer, sarcoma, skin cancer, small intestine cancer, stomach cancer, testicular cancer, thymus cancer, thyroid cancer, uterine cancer, uterine sarcoma cancer, vaginal cancer, vulvar cancer, waldenstrom macroglobulinemia wilms tumor, castleman disease ewing family of tumor, gastrointestinal carcinoid tumor, gastrointestinal stromal tumor, myelodysplastic syndrome pituitary tumor, and a cancerous disease such as gestational trophoblastic disease, Hodgkin disease, Kaposi sarcoma, malignant mesothelioma, and multiple myeloma.

The invention also relates to a method for the treatment of anemia or anemia disease or to nanoparticle or at least one constituent of the compositions, in particular magnetosomes, for use in the treatment of anemia disease, preferentially iron anemia disease, wherein magnetosomes are administered to the body part of an individual, preferentially to reduce or stop anemia.

The invention also relates to a method for the treatment of an anemia disease, wherein this disease is selected from the group consisting of: Iron or metal deficiency anemia, Vitamin deficiency anemia, Anemia of chronic disease, Aplastic anemia, Anemia associated with bone marrow disease, Hemolytic anemia, Sickle cell anemia, Thalassaemia, Pernicious anaemia, Fanconi anaemia, Sideroblastic Anemia, Congenital Dyserythropoietic Anemia (CDA), Diamond Blackfan Anemia, and Megaloblastic Anemia. In some cases, anemia is a decrease in the total amount of red blood cells (RBCs) or hemoglobin in the blood, or a lowered ability of the blood to carry oxygen.

In one embodiment of this invention, the nanoparticle or at least one constituent of the composition is or belongs to or is comprised in the group of nanoparticle or at least one constituent of the compositions selected from: a nanosphere, a nanocapsule, a dendrimer, a carbon nanotube, a lipid/solid nanoparticle or at least one constituent of the composition, a lipid or protein or DNA or RNA based nanoparticle or at least one constituent of the composition, a nanoparticle or at least one constituent of the composition with an inner aqueous environment surrounded by a layer, preferentially a stabilizing layer, most preferentially a phospholipid layer, a multilayer nanoparticle or at least one constituent of the composition, a polymeric nanoparticle or at least one constituent of the composition, a quantum dot, a metallic nanoparticle or at least one constituent of the composition, a polymeric micelle or nanoparticle or at least one constituent of the composition, a carbon based nano-structure, a nanobubble, a nanosome, a pharmacyte, a niosome, a nanopore, a microbivore, a liposome, a virus, preferentially recombinant, a herbal nanoparticle or at least one constituent of the composition, an antibody, and a vesicle.

In another embodiment of this invention, the nanoparticle or at least one constituent of the composition is not or does not belong to or is not comprised in at least one nanoparticle or at least one constituent of the composition belonging to the group of: a nanosphere, a nanocapsule, a dendrimer, a carbon nanotube, a lipid/solid nanoparticle or at least one constituent of the composition, a lipid or protein or DNA or RNA based nanoparticle or at least one constituent of the composition, a nanoparticle or at least one constituent of the composition with an inner aqueous environment surrounded by a layer, preferentially a stabilizing layer, most preferentially a phospholipid layer, a multilayer nanoparticle or at least one constituent of the composition, a polymeric nanoparticle or at least one constituent of the composition, a quantum dot, a metallic nanoparticle or at least one constituent of the composition, a polymeric micelle or nanoparticle or at least one constituent of the composition, a carbon based nano-structure, a nanobubble, a nanosome, a pharmacyte, a niosome, a nanopore, a microbivore, a liposome, a virus, preferentially recombinant, a herbal nanoparticle or at least one constituent of the composition, an antibody, and a vesicle.

In some cases, the nanoparticle or at least one constituent of the composition can be in a liquid, gaseous, or solid state, preferentially before, during or after its presence or administration in the body part.

In some other cases, the nanoparticle or at least one constituent of the composition can't be in one or two of the liquid, gaseous, or solid states, preferentially before, during or after its presence or administration in the body part.

In still some other cases, the nanoparticle or at least one constituent of the compositions can be assimilated to or be comprised in a ferrofluid, a chemical or biological ferrofluid, wherein chemical and biological ferrofluids are fluids containing iron, preferentially forming nanoparticle or at least one constituent of the compositions, which are fabricated through a chemical or biological synthesis, respectively.

In still some other cases, the ferrofluid or nanoparticle or at least one constituent of the composition assembly can comprise the nanoparticle or at least one constituent of the compositions and an excipient, a solvent, a matrix, a gel, which preferentially enables the administration of the nanoparticle or at least one constituent of the compositions to the individual or body part.

In still some other cases, the nanoparticle or at least one constituent of the composition can comprise synthetic material and/or biological material and/or inorganic material and/or organic material.

In one embodiment of the invention, (the) nanoparticle or at least one constituent of the composition(s) is/are or designate: i) a suspension of nanoparticle or at least one constituent of the compositions, ii) a composition or at least one constituent of the composition comprising nanoparticle or at least one constituent of the compositions, iii) an assembly of nanoparticle or at least one constituent of the compositions, iv) a nanoparticle or at least one constituent of the composition region, v) the mineral part or core the nanoparticle or at least one constituent of the composition, vi) the organic part of the nanoparticle or at least one constituent of the composition, vii) the inorganic part of the nanoparticle or at least one constituent of the composition, viii) or the coating of the nanoparticle or at least one constituent of the composition.

In one embodiment of the invention, nanoparticle or at least one constituent of the composition(s) or the nanoparticle or at least one constituent of the composition(s) represent(s) or is or are an assembly or suspension or composition or at least one constituent of the composition of more or comprising more than 10⁻¹⁰⁰, 10⁻⁵⁰, 10⁻¹⁰, 10⁻⁵, 10⁻¹, 1, 10, 10², 10³, 10⁵, 10¹⁰, 10²⁰ or 10⁵⁰ nanoparticle or at least one constituent of the composition(s) or mg of nanoparticle or at least one constituent of the composition(s) or mg of iron comprised in nanoparticle or at least one constituent of the composition(s) or mg of nanoparticle or at least one constituent of the composition(s) per cm³ or mg of nanoparticle or at least one constituent of the composition(s) per cm³ of body part or mg of iron comprised in nanoparticle or at least one constituent of the composition(s) per cm³ or mg of iron comprised in nanoparticle or at least one constituent of the composition(s) per cm³ of body part. In some cases, an assembly or suspension or composition or at least one constituent of the composition comprising a large number of nanoparticle or at least one constituent of the compositions can be used to induce or produce a temperature increase, radical or reactive species, or the dissociation of a compound from the nanoparticle or at least one constituent of the compositions.

In another embodiment of the invention, nanoparticle or at least one constituent of the composition(s) or the nanoparticle or at least one constituent of the composition(s) represent(s) or is or are an assembly or suspension or composition or at least one constituent of the composition of less or comprising less than 10¹⁰⁰, 10⁵⁰, 10²⁰, 10¹⁰, 10⁵, 10², 10, 1, 5, 2, 1, 10⁻¹, 10⁻⁵, 10⁻¹⁰ or 10⁻⁵⁰ nanoparticle or at least one constituent of the composition(s) or mg of nanoparticle or at least one constituent of the composition(s) or mg of iron comprised in nanoparticle or at least one constituent of the composition(s) or mg of nanoparticle or at least one constituent of the composition(s) per cm³ or mg of nanoparticle or at least one constituent of the composition(s) per cm³ of body part or mg of iron comprised in nanoparticle or at least one constituent of the composition(s) per cm³ or mg of iron comprised in nanoparticle or at least one constituent of the composition(s) per cm³ of body part. In some cases, an assembly or suspension or composition or at least one constituent of the composition of nanoparticle or at least one constituent of the compositions comprising a low number of nanoparticle or at least one constituent of the composition(s) can be used to prevent toxicity.

In one embodiment of the invention, the nanoparticle or at least one constituent of the composition(s) or nanoparticle or at least one constituent of the composition(s) or nanoparticle or at least one constituent of the composition assembly can represent or be the region, also designated as nanoparticle or at least one constituent of the composition region, volume, surface, length, which comprises the nanoparticle or at least one constituent of the compositions or where nanoparticle or at least one constituent of the compositions are located. In some cases, the volume of the region occupied by the nanoparticle or at least one constituent of the compositions in the body part is designated as nanoparticle or at least one constituent of the composition region.

In some cases, the nanoparticle or at least one constituent of the composition region can be the volume occupied by an assembly of nanoparticle or at least one constituent of the compositions in the body part, where the nanoparticle or at least one constituent of the compositions are preferentially separated by less than 10⁹, 10⁶, 10³ or 10 nm.

In some cases, the nanoparticle or at least one constituent of the composition assembly is a more general term than nanoparticle or at least one constituent of the composition region, which could designate any type of nanoparticle or at least one constituent of the composition assembly, before, during, or after nanoparticle or at least one constituent of the composition administration to or in the body part.

In some cases, the separating distance between the nanoparticle or at least one constituent of the compositions within the nanoparticle or at least one constituent of the composition assembly or nanoparticle or at least one constituent of the composition region can correspond to the average or maximum distance separating the nanoparticle or at least one constituent of the compositions within this assembly.

In some cases, the distribution in separating distances between nanoparticle or at least one constituent of the compositions can highlight the presence of a minority of nanoparticle or at least one constituent of the compositions, *i.e.* preferentially less than 50, 10, 1, 10⁻² or 10⁻⁵ % of the total number of nanoparticle or at least one constituent of the compositions in the individual, with either small separating distances, *i.e* separating distances preferentially lower than 10⁹, 10⁶, 10³ or 10 nm, or with large separating distances, i.e. separating distances preferentially larger than 10⁹, 10⁶, 10³ or 10 nm. In this case, the presence of this minority of nanoparticle or at least one constituent of the compositions is preferentially not taken into consideration to estimate the average or maximum separating distance between the nanoparticle or at least one constituent of the compositions.

The invention also relates to nanoparticle or at least one constituent of the compositions for use according to the invention, wherein the nanoparticle or at least one constituent of the compositions are crystallized, metallic, or magnetic.

In an embodiment of the invention, the nanoparticle or at least one constituent of the compositions are crystallized. In this case, they preferentially possess more than or at least 1, 2, 10, 10², 10³, 10⁶ or 10⁹ crystallographic plane(s) or regular atomic arrangement(s), preferentially observable by electron microscopy.

In one embodiment of the invention, the nanoparticle or at least one constituent of the compositions are metallic. In this case, they contain at least 1, 10, 10³, 10⁵ or 10⁹ metallic atom(s) or contain at least 1, 10, 50, 75 or 90% of metallic atoms, where this percentage can be the ratio between the number or mass of metallic atoms in the nanoparticle or at least one constituent of the composition divided by the total number or mass of all atoms in the nanoparticle or at least one constituent of the composition. The nanoparticle or at least one constituent of the compositions, preferentially metal oxide nanoparticle or at least one constituent of the compositions, can also contain at least 1, 10, 10³, 10⁵ or 10⁹ oxygen atom(s), or contain at least 1, 10, 50, 75 or 90% of oxygen atoms, where this percentage can be the ratio between the number or mass of oxygen atoms in the nanoparticle or at least one constituent of the compositions divided by the total number or mass of all atoms in the nanoparticle or at least one constituent of the compositions.

In another embodiment of the invention, the metal or metal atom is selected in the list consisting of: Lithium, Beryllium, Sodium, Magnesium, Aluminum, Potassium, Calcium, Scandium, Titanium, Vanadium, Chromium, Manganese, Iron, Cobalt, Nickel, Copper, Zinc, Gallium, Rubidium, Strontium, Yttrium, Zirconium, Niobium, Molybdenum, Technetium, Ruthenium, Rhodium, Palladium, Silver, Cadmium, Indium, Tin, Cesium, Barium, Lanthanum, Cerium, Praseodymium, Neodymium, Promethium, Samarium, Europium, Gadolinium, Terbium, Dysprosium, Holmium, Erbium, Thulium, Ytterbium, Lutetium, Hafnium, Tantalum, Tungsten, Rhenium, Osmium, Iridium, Platinum, Gold, Mercury, Thallium, Lead, Bismuth, Polonium, Francium, Radium, Actinium, Thorium, Protactinium, Uranium, Neptunium, Plutonium, Americium, Curium, Berkelium, Californium, Einsteinium, Fermium, Mendelevium, Nobelium, Lawrencium, Rutherfordium, Dubnium, Seaborgium, Bohrium, Hassium, Meitnerium, Darmstadtium, Roentgenium, Copernicium, Nihonium, Flerovium, Moscovium, and Livermorium or Livermorium atom.

In another embodiment of the invention, the nanoparticle or at least one constituent of the composition contains or comprises less than 1, 10, 10³, 10⁵ or 10⁹ metallic atom(s) or contains less than 1, 10, 50, 75 or 90% of metallic atoms, where this percentage can be the ratio between the number or mass of metallic atoms in the nanoparticle or at least one constituent of the composition divided by the total number or mass of all atoms in the nanoparticle or at least one constituent of the composition. It can also contain less than 1, 10, 10³, 10⁵ or 10⁹ oxygen atom(s), or contain less than 1, 10, 50, 75 or 90% of oxygen atoms, where this percentage can be the ratio between the number or mass of oxygen atoms in the nanoparticle or at least one constituent of the composition divided by the total number or mass of all atoms in the nanoparticle or at least one constituent of the composition.

In one embodiment of the invention, the nanoparticle or at least one constituent of the composition is magnetic when it has a magnetic behavior or property, where the magnetic behavior or property is preferentially selected from the group consisting of a diamagnetic, superparamagnetic, paramagnetic, ferromagnetic, and ferrimagnetic behavior or property.

In some cases, the magnetic behavior or property can be observed or exists at a temperature, which is lower than: i) 10⁵, 10³, 500, 350, 200, 100, 50, 20, 10, 1, 0.5 or 1 K (Kelvin), ii) the Curie temperature, or iii) the blocking temperature.

In some other cases, the magnetic behavior or property can be observed or exists at a temperature, which is larger than: i) 0.5, 1, 10, 20, 50, 100, 200, 350, 500, 10³ or 10⁵ K, ii) the Curie temperature, or iii) the blocking temperature.

In still some other cases, the magnetic behavior or property can be observed or exists at a temperature, which is between 10⁻²⁰ and 10²⁰ K, or between 0.1 and 1000 K.

In one embodiment of the invention, the nanoparticle or at least one constituent of the compositions have or are characterized by at least one of the following properties: i) the presence of a core, preferentially magnetic, preferentially mineral, preferentially composed of a metallic oxide such as iron oxide, most preferentially maghemite or magnetite, or an intermediate composition or at least one constituent of the composition between maghemite and magnetite, ii) the presence of a coating that surrounds the core and preferentially prevents nanoparticle or at least one constituent of the composition aggregation, preferentially enabling nanoparticle or at least one constituent of the composition administration in an organism or in the body part or stabilizing the nanoparticle or at least one constituent of the composition core, where coating thickness may preferably lie between 0.1 nm and 10 µm, between 0.1 nm and 1 µm, between 0.1 nm and 100 nm, between 0.1 nm and 10 nm, or between 1 nm and 5 nm, iii) magnetic properties leading to diamagnetic, paramagnetic, superparamagnetic, ferromagnetic, or ferrimagnetic behavior, iv) a coercivity larger than 0.01, 0.1, 1, 10, 100, 10³, 10⁴, 10⁵, 10⁹ or 10²⁰ Oe, v) a ratio between remanent and saturating magnetization larger than 0.01, 0.1, 0.2, 0.3, 0.4, 0.5, 0.75, 0.9 or 0.99, vi) a saturating magnetization larger than 0.1, 1, 5, 10 or 50 emu/g, vii) magnetic properties such as coercivity, remanent and saturating magnetization, preferentially measured or observed at a temperature larger than 0.1 K, 1 K, 10 K, 20 K, 50 K, 100 K, 200 K, 300 K, 350 K or 3000 K, viii) a crystallinity, *i.e.* nanoparticle or at least one constituent of the compositions preferentially possessing at least 1, 2, 5, 10 or 100 crystalline plane(s), preferentially observable or measured by electron microscopy, ix) the presence of a single domain, x) a size that is larger than 0.1, 0.5, 1.5, 10, 15, 20, 25, 30, 50, 60, 70, 80, 100, 120, 150 or 200 nm, xi) a size lying between 0.1 nm and 10 µm, between 0.1 nm and 1 µm, between 0.1 nm and 100 nm, between 1 nm and 100 nm, or between 5 nm and 80 nm, xii) a non-pyrogenicity or apyrogenicity, which preferentially means that nanoparticle or at least one constituent of the compositions possess an endotoxin concentration lower than 10²⁰, 10000, 1000, 100, 50, 10, 5, 2 or 1 EU (endotoxin unit) per mg of nanoparticle or at least one constituent of the composition or per mg of iron comprised in nanoparticle or at least one constituent of the compositions, or which means that nanoparticle or at least one constituent of the compositions do not trigger fever or an increase in whole body temperature larger than 100, 50, 6.6, 5, 3, 2 or 1 °C following their administration to a living organism or body part, xiii) a synthesis by a synthetizing living organism, preferentially by bacteria, xiv) a chemical synthesis, xv) the presence of less than 50, 25, 15, 10, 5, 2 or 1% of organic or carbon material originating from the synthetizing living organism, xv), the presence of more than 99, 95, 80, 70, 60, 50 or 25% of mineral material originating from the synthetizing living organism, or xvi) a specific absorption rate (SAR) that is larger than 1, 10, 1000 or 10⁴ Watt per gram of nanoparticle or at least one constituent of the composition, preferentially measured under the application of an alternating magnetic field of strength preferentially larger than 0.1, 1, 10 or 100 mT, and/or frequency larger than 1, 10, 100 or 1000 KHz, alternatively preferentially measured under the application of the acoustic wave, alternatively under the application of a radiation such as an electromagnetic acoustic, or light radiation.

In another embodiment of the invention, the nanoparticle or at least one constituent of the compositions have or are characterized by at least one of the following properties: i) a coercivity lower than 0.01, 0.1, 1, 10, 100, 10³, 10⁴, 10⁵, 10⁹ or 10²⁰ Oe, ii) a ratio between remanent and saturating magnetization lower than 0.01, 0.1, 0.2, 0.3, 0.4, 0.5, 0.75, 0.9 or 0.99, iii) a saturating magnetization lower than 0.1, 1, 5, 10, 50, 200, 1000 or 5000 emu/g, iv) magnetic properties preferentially measured or observed at a temperature lower than 0.1 K, 1 K, 10 K, 20 K, 50 K, 100 K, 200 K, 300 K, 350 K or 3000 K, v) a size that is lower than 0.1, 0.5, 1.5, 10, 15, 20, 25, 30, 50, 60, 70, 80, 100, 120, 150 or 200 nm, vi) the presence of more than 50, 25, 15, 10, 5, 2 or 1% of organic or carbon material originating from the synthetizing living organism, vii) the presence of less than 99, 95, 80, 70, 60, 50 or 25% of mineral material originating from the synthetizing living organism, or xi), a specific absorption rate (SAR) that is lower than 1, 10, 1000 or 10⁴ Watt per gram of nanoparticle or at least one constituent of the composition, preferentially measured under the application of an alternating magnetic field of strength preferentially lower than 0.1, 1, 10, or 100, 200, 500, 10³ or 10⁵ mT, and/or of frequency preferentially lower than 1, 10, 100, 10³, 10⁵ or 10⁹ KHz, alternatively preferentially measured under the application of the acoustic wave, alternatively under the application of a radiation such as an electromagnetic acoustic, or light radiation.

In some cases, the mineral can be the part of the nanoparticle or at least one constituent of the composition or magnetosome that does not comprise organic material or comprises a low percentage in mass of organic material, preferentially less than 100, 99, 50, 20, 10, 5, 1, 10⁻¹ or 10⁻² percent or percent in mass of organic material. The mineral is preferentially the core of the nanoparticle or at least one constituent of the composition.

In some other cases, the mineral can comprise a percentage in mass of organic material larger than 0, 10⁻⁵⁰, 10⁻¹⁰, 10⁻², 10⁻¹ or 1 percent or percent in in mass of organic material. This can be the case when the purification step unsuccessfully removes the organic material or when organic material is added to the mineral after the purification step.

In some cases, the nanoparticle or at least one constituent of the compositions can be surrounded by a coating. The coating can be made of a synthetic, organic, or inorganic material or of a substance comprising a function selected in the group consisting of carboxylic acids, phosphoric acids, sulfonic acids, esters, amides, ketones, alcohols, phenols, thiols, amines, ether, sulfides, acid anhydrides, acyl halides, amidines, amides, nitriles, hydroperoxides, imines, aldehydes, and peroxides. In some cases, the coating can be made of carboxy-methyl-dextran, citric acid, phosphatidylcholine (DOPC), or oleic acid. In some cases, the coating can enable the dispersion of the nanoparticle or at least one constituent of the compositions in a matrix or solvent such as water, preferentially without aggregation or sedimentation of the nanoparticle or at least one constituent of the compositions. In some cases, the coating can enable internalization of the nanoparticle or at least one constituent of the compositions in cells. In some other cases, the coating can enable: i) to bind two or more nanoparticle or at least one constituent of the composition(s) together preferentially in a chain, ii) to prevent nanoparticle or at least one constituent of the composition aggregation and/or, iii) to obtain uniform nanoparticle or at least one constituent of the composition distribution, and/or iv) to encapsulate or embedd at least one center of activity or of free radical/production.

In one embodiment of the invention, the nanoparticle or at least one constituent of the compositions are non-pyrogenic. Non-pyrogenic nanoparticle or at least one constituent of the compositions preferentially: i) comprise less than 10¹⁰⁰, 10⁵⁰, 10²⁰, 10⁸, 10⁵, 10³, or 10 EU (endotoxin unit) or EU per cm³ of body part or EU per mg of nanoparticle or at least one constituent of the composition or EU per cm³ of body part per mg of nanoparticle or at least one constituent of the composition, or ii) induce a temperature increase of the individual or body part of less than 10⁵, 10³, 10², 50, 10, 5, 4, 3, 2 or 1°C, preferentially above physiological temperature, preferentially before, after or without the application of the acoustic wave or radiation on the nanoparticle or at least one constituent of the composition.

In one embodiment of this invention, the nanoparticle or at least one constituent of the composition or compound is composed of or comprises a chemical element of the families selected from the group consisting of: metals (alkali metal, alkaline earth metal, transition metals), semimetal, non-metal (halogens element, noble gas), chalcogen elements, lanthanide, and actinide.

In another embodiment of the invention, the nanoparticle or at least one constituent of the composition or compound is composed of or comprises a chemical element selected from the group consisting of: hydrogen, lithium, sodium, potassium, rubidium, caesium, francium, beryllium, magnesium, calcium, strontium, barium, radium, scandium, yttrium, lanthanide, actinide, titanium, zirconium, hafnium, rutherfordium, vanadium, niobium, tantalum, dubnium, chromium, molybdenum, tungsten, seaborgium, manganese, technetium, rhenium, bohrium, iron, ruthenium, osmium, hessium, cobalt, rhodium, iridium, meitherium, nickel, palladium, platinum, darmstadtium, copper, silver, gold, roentgenium, zinc, cadmium, mercury, copernicum, boron, aluminium, gallium, indium, thallium, ununtrium, carbon, silicon, germanium, tin, lead, fleovium, nitrogen, phosphorus, arsenic, antimony, bismuth, ununpentium, oxygen, sulphur, selenium, tellurium, polonium, livermorium, fluorine, chlorine, bromine, iodine, astatine, ununseptium, helium, neon, argon, krypton, xenon, radon, ununoctium, lanthanum, cerium, praseodymium, neodymium, promethium, samarium, europium, gadolinium, terbium, dysprosium, holmium, erbium, thulium, ytterbium, lutetium, actinium, thorium, proctactinium, uranium, neptunium, plutonium, americium, curium, berkelium, californium, einsteinium, fermium, mendelevium, nobelium, and lawrencium.

In some cases, the nanoparticle or at least one constituent of the composition or compound can also be composed of or comprise an alloy, a mixture, or an oxide of this(these) chemical element(s).

In some cases, the nanoparticle or at least one constituent of the composition or compound can be composed of more than 10⁻⁵⁰, 10⁻²⁰, 10⁻¹⁰, 10⁻⁵, 10⁻², 1, 5, 10, 50, 75, 80, 90, 95 or 99% of one or several of this(these) element(s), where this percentage can represent the mass or number of this(these) chemical elements comprised in the nanoparticle or at least one constituent of the composition or compound divided by the total number or total mass of all chemical elements comprised in the nanoparticle or at least one constituent of the composition or compound or by the total mass of the nanoparticle or at least one constituent of the composition or compound.

In some other cases, the nanoparticle or at least one constituent of the composition or compound can be composed of or comprise less than 10⁻⁵⁰, 10⁻²⁰, 10⁻¹⁰, 10⁻⁵, 10⁻², 1, 5, 10, 50, 75, 80, 90, 95 or 99% of one or several of this(these) chemical element(s).

In still some other cases, this(these) chemical element(s) is(are) comprised inside the nanoparticle or at least one constituent of the composition or compound, or at the surface of the nanoparticle or at least one constituent of the composition or compound, or in the mineral or core of the nanoparticle or at least one constituent of the composition or compound, or in the coating of the nanoparticle or at least one constituent of the composition or compound.

In one embodiment of this invention, the nanoparticle or at least one constituent of the composition or compound is not composed of or does not comprise at least one chemical element belonging to the family selected from the group consisting of: metals (alkali metal, alkaline earth metal, transition metals), semimetal, non- metal (halogens element, noble gas), chalcogen elements, lanthanide, actinide. In another embodiment of the invention, the nanoparticle or at least one constituent of the composition or compound is devoid of or does not comprise at least one chemical element selected from the group consisting of: hydrogen, lithium, sodium, potassium, rubidium, caesium, francium, beryllium, magnesium, calcium, strontium, barium, radium, scandium, yttrium, lanthanide, actinide, titanium, zirconium, hafnium, rutherfordium, vanadium, niobium, tantalum, dubnium, chromium, molybdenum, tungsten, seaborgium, manganese, technetium, rhenium, bohrium, iron, ruthenium, osmium, hessium, cobalt, rhodium, iridium, meitherium, nickel, palladium, platinum, darmstadtium, copper, silver, gold, roentgenium, zinc, cadmium, mercury, copernicum, boron, aluminium, gallium, indium, thallium, ununtrium, carbon, silicon, germanium, tin, lead, fleovium, nitrogen, phosphorus, arsenic, antimony, bismuth, ununpentium, oxygen, sulphur, selenium, tellurium, polonium, livermorium, fluorine, chlorine, bromine, iodine, astatine, ununseptium, helium, neon, argon, krypton, xenon, radon, ununoctium, lanthanum, cerium, praseodymium, neodymium, promethium, samarium, europium, gadolinium, terbium, dysprosium, holmium, erbium, thulium, ytterbium, lutetium, actinium, thorium, proctactinium, uranium, neptunium, plutonium, americium, curium, berkelium, californium, einsteinium, fermium, mendelevium, nobelium, and lawrencium.

In another embodiment of the invention, the nanoparticle or at least one constituent of the composition or compound is not composed of or does not comprise an alloy, a mixture, or an oxide of this(these) chemical element(s).

In one embodiment of the invention, the nanoparticle or at least one constituent of the composition is defined as a particle with a size in one dimension, which is larger than 10⁻¹, 1, 2, 5, 10, 20, 50, 70, 100, 200 or 500 nm. A nanoparticle or at least one constituent of the composition with a large size can have a larger coercivity and/or a larger remanent magnetization and/or can more strongly or more efficiently absorb the energy or power of the acoustic wave than a nanoparticle or at least one constituent of the composition with a small size. In some cases, the amount of energy or power absorbed by a nanoparticle or at least one constituent of the composition is increased by a factor of more than 1.001, 1.01, 1.1, 1.2, 1.5, 2, 5, 10, 10³, 10⁵ or 10⁷ by increasing the size of the nanoparticle or at least one constituent of the composition by a factor of more than 1.001, 1.01, 1.1, 1.2, 1.5, 2, 5, 10, 10³, 10⁵ or 10⁷.

In another embodiment of the invention, the nanoparticle or at least one constituent of the composition is defined as a particle with a size in one dimension, which is lower than 10⁴, 10³, 10², 10, 1 or 10⁻¹ nm. Preferentially, a nanoparticle or at least one constituent of the composition with a small size can more easily be administered, for example intravenously, or can enable the avoidance of some toxicity effects, such as embolism.

In still another embodiment of the invention, the nanoparticle or at least one constituent of the composition size lies between 10⁻² and 10²⁰ nm, 10⁻² and 10⁴ nm, between 10⁻¹ and 10³ nm, or between 1 and 10² nm. This can be the case when the nanoparticle or at least one constituent of the composition or nanoparticle or at least one constituent of the composition assembly possesses a well-defined, preferentially narrow, distribution in sizes.

In still another embodiment of the invention, the nanoparticle or at least one constituent of the composition size distribution is lower than 1000, 100, 75, 50, 25, 10, 5, 2 or 1 nm. Preferentially, a narrow nanoparticle or at least one constituent of the composition size distribution may be desired to prevent aggregation, or to favor an organization in chains of the nanoparticle or at least one constituent of the compositions.

In still another embodiment of the invention, the nanoparticle or at least one constituent of the composition size distribution is larger than 1000, 100, 75, 50, 25, 10, 5, 2 or 1 nm. Preferentially, a large nanoparticle or at least one constituent of the composition size distribution may in some cases enable nanoparticle or at least one constituent of the compositions to be eliminated more rapidly.

In another embodiment of the invention, the nanoparticle or at least one constituent of the composition has a surface charge, which is larger than -200, -100, -50, -10, -5, 0.1, 1, 2, 5, 10, 50 or 100 mV, preferentially at a pH lower than 0.1, 1, 2, 3, 4, 5, 6, 7, 8, 9, 10, 11, 12, 13 or 14. Preferentially, a nanoparticle or at least one constituent of the composition can have a large surface charge at low pH when it is surrounded by a coating that enables to reach such charge without being destroyed.

In another embodiment of the invention, the nanoparticle or at least one constituent of the composition has a surface charge, which is lower than -200, -100, -50, -10, -5, 0.1, 1, 2, 5, 10, 50 or 100 mV, preferentially at a pH larger than 0.1, 1, 2, 3, 4, 5, 6, 7, 8, 9, 10, 11, 12, 13 or 14. Preferentially, a nanoparticle or at least one constituent of the composition can have a low surface charge at high pH when it is surrounded by a coating that enables to reach such charge without being destroyed.

In still another embodiment of the invention, the nanoparticle or at least one constituent of the composition has a surface charge comprised between +200 and -200 mV, +100 and -100 mV, +50 and -50 mV, +40 et-40mV, +20 and -20, +10 and -10 mV, or between +5 and -5 mV, preferentially at a pH lower than 0.1, 1, 2, 3, 4, 5, 6, 7, 8, 9, 10, 11, 12, 13 or 14.

In still another embodiment of the invention, the nanoparticle or at least one constituent of the composition has a surface charge comprised between +200 and -200 mV, +100 and -100 mV, +50 and -50 mV, +40 et-40mV, +20 and -20, +10 and -10 mV, or between +5 and -5 mV, preferentially at a pH larger than 0.1, 1, 2, 3, 4, 5, 6, 7, 8, 9, 10, 11, 12, 13 or 14.

In another embodiment of the invention, the nanoparticle or at least one constituent of the composition has a weight or a mass, preferentially expressed in unit such as gram (g), kilogram (kg), or milligram (mg). A gram of nanoparticle or at least one constituent of the composition can be a gram of metal such as iron comprised in the nanoparticle or at least one constituent of the composition. The mass or weight of the nanoparticle or at least one constituent of the composition can correspond to the mass or weight of one nanoparticle or at least one constituent of the composition or to the mass or weight of an assembly of nanoparticle or at least one constituent of the compositions.

In an embodiment, the mass of the nanoparticle or at least one constituent of the composition is larger than 10⁻²⁰, 10⁻¹⁰, 10⁻⁵, 10⁻², 1, 10, 10³, 10⁹ or 10²⁰ gram. In some cases, a large nanoparticle or at least one constituent of the composition mass may be desired to increase the quantity of acoustic wave energy absorbed by the nanoparticle or at least one constituent of the composition.

In an embodiment, the mass of the nanoparticle or at least one constituent of the composition is lower than 10⁻²⁰, 10⁻¹⁰, 10⁻⁵, 10⁻², 1, 10, 10³, 10⁹ or 10²⁰ gram. In some cases, a low nanoparticle or at least one constituent of the composition mass may be desired to prevent or minimize nanoparticle or at least one constituent of the composition toxicity.

In one embodiment of the invention, the nanoparticle or at least one constituent of the compositions are arranged in chains comprising more than 1, 2, 3, 4, 5, 6, 7, 8, 9, 10, 15, 20, 25, 30, 35 or 40 nanoparticle(s) or constituent(s) of the compositions.

In another embodiment of the invention, the nanoparticle(s) or constituent(s) of the compositions are arranged in chains, which have: i) a length smaller than 2.10¹⁰, 2.10⁵, 2.10³ or 2.10² nm, or ii) a number of nanoparticle or at least one constituent of the compositions in each chain smaller than 2, 5, 10, 10⁻² or 10³. In some cases, short chains of nanoparticle or at least one constituent of the compositions may be desired or obtained, for example to favor nanoparticle or at least one constituent of the composition internalization in cells or after partial or total destruction of long chains.

In another embodiment of the invention, the nanoparticle(s) or constituent(s) of the compositions are arranged in chains, which have: i) a length longer than 10⁻¹, 1, 5, 10, 2.10², 2.10³ or 2.10⁵ nm or ii) a number of nanoparticle or at least one constituent of the compositions in each chain larger than 2, 5, 10, 10² or 10³. In some cases, long chains of nanoparticle or at least one constituent of the compositions may be desired or obtained to increase the quantity of heat or compounds dissociated from the nanoparticle or at least one constituent of the compositions under the application of an acoustic wave or radiation or to prevent nanoparticle or at least one constituent of the composition aggregation or enable uniform nanoparticle or at least one constituent of the composition distribution.

In still another embodiment of the invention, the nanoparticle or at least one constituent of the compositions are arranged in chains, which have: i) a length between 10⁻¹ and 10¹⁰ nm, or between 1 and 10⁵ nm, or ii) a number of nanoparticle or at least one constituent of the compositions in each chain between 2 and 10⁵, 2 and 10³, 2 and 10², or 2 and 50.

In still another embodiment of the invention, the nanoparticles or constituents of the compositions are arranged in chains when they are bound or linked to each other or when the crystallographic directions of two adjacent nanoparticle or at least one constituent of the compositions in the chain are aligned, wherein such alignment is preferentially characterized by an angle between two crystallographic directions belonging to two adjacent nanoparticle or at least one constituent of the compositions in the chains of less than 90, 80, 70, 60, 50, 20, 10, 3, or 2 ° (degree).

Preferentially when the nanoparticles or at least one constituent of the compositions are biologically synthesized, the nanoparticle or at least one constituent of the compositions can be arranged in chains: i) inside the organism that synthesizes them, also designated as synthetizing living organism, or ii) outside this organism. Preferentially, nanoparticle or at least one constituent of the compositions are arranged in chains after or before their extraction or isolation from this organism.

In one embodiment of the invention, the nanoparticles or at least one constituent of the compositions are not arranged in chains.

In another embodiment of the invention, the nanoparticles or at least one constituent of the compositions are synthesized chemically or are not synthesized by a living organism when less than 1, 2, 5, 10 or 100 step(s) of their production, such as crystallization of iron oxide, stabilization of the iron oxide mineral, organization of the nanoparticle or at least one constituent of the compositions, involves or is due to a living organism. In some cases, a chemical synthesis can be defined as a synthesis involving a majority of steps, or more than 1, 2, 5 or 10 steps, or more than 1, 2, 5, 25, 50, 75 or 90% of steps, which involve chemical reactions occurring without the involvement of living organisms, or parts of living organisms such as DNA, RNA, proteins, enzymes, lipids.

In another embodiment of the invention, a chemical synthesis can be used to produce a chemical substance or compound that mimics, copies, or reproduces the compartment, organelle, or other biological material, wherein this chemical synthesis or chemical substance can be used or can result in the production of the nanoparticle or at least one constituent of the compositions. In some cases, the compartment, organelle, or other biological material, can be a lysosome, an endosome, a vesicle, preferentially biological material that has the capacity or the function either to dissolve or transform crystallized iron into free iron or to transform free iron into crystalized iron. In some cases, this transformation is partial and preferentially results in the destruction or formation of partly crystallized assembly of iron atoms or ions, or preferentially results in a mixture of crystallized iron and non-crystallized iron. In some cases, crystallized iron can be defined as an assembly of iron atoms or ions that leads to the presence of crystallographic planes, preferentially observable using a technique such as transmission or scanning electron microscopy as a characterization method, and free iron can preferentially be defined as one of several iron atoms or ions that do not lead to the presence of crystallographic planes, preferentially highlighted by the absence of diffraction patterns, using for example transmission or scanning electron microscopy as a characterization method.

The invention also relates to nanoparticle or at least one constituent of the compositions for use, wherein nanoparticle or at least one constituent of the compositions are or are assimilated to chemical analogues of magnetosomes, such as iron oxide nanoparticle or at least one constituent of the compositions designated as Sigma nanoparticle or at least one constituent of the compositions (ref: 637106-25G), SPION20 (nanomag^{®}-D-spio 20, Ref: 79-02-201), SPION50 (synomag-D50, Ref: 104-000-501), SPION100 (nanomag^{®}-D-spio 100, Ref: 79-00-102) or nanoparticle or at least one constituent of the compositions synthesized using a similar method as for these nanoparticle or at least one constituent of the compositions but yielding improved or additional properties such as an arrangement in chains.

In some cases, chemical analogues of magnetosomes can be synthesizes chemically and/or are not synthesized by magnetotactic bacteria.

In some cases, chemical analogues of magnetosomes possess at least 1, 2, 3, 4, 5, 6, 7, 8, 9, or 10 common property(ies) with the magnetosomes, where these common properties are preferentially a ferrimagnetic behavior, preferentially a coercivity larger that 10⁻⁵⁰, 10⁻¹⁰, 10⁻², 1, 5, 10 or 100 Oe at a temperature preferentially larger than 0, 5, 10, 50, 100, 200, 300, 500 or 1000 K, a large size, preferentially a size larger than 1, 5, 10, 20, 50 or 70 nm, and/or a chain arrangement, preferentially an arrangement of more than 1, 2, 5 or 10 nanoparticle or at least one constituent of the compositions in chain.

In one embodiment of the invention, the nanoparticle or at least one constituent of the compositions or magnetosomes are purified to remove more than 10, 50 or 90 percent or percent in mass of endotoxins and/or other biological material such as proteins or lipids originating from the synthetizing living organism or magnetotactic bacteria. In some other cases, the nanoparticle or at least one constituent of the compositions or magnetosomes are purified to remove less than 100, 99.9, 99, 95 or 90 percent or percent in mass of endotoxins and/or other biological material. This purification step preferentially yields purified nanoparticle or at least one constituent of the compositions or magnetosomes. In some cases, this percentage can be equal to [Q_{BP}-Q_{AP}]/Q_{BP} or Q_{AP}/Q_{BP}, where Q_{BP} and Q_{AP} are the quantities of endotoxins, biological material, proteins, or lipids before and after the purification step, respectively. In some cases, the purification step can consist in using a method or detergent(s) such as NaOH and/or KOH, which is/are preferentially mixed with the synthetizing living organism or magnetotactic bacteria or bacterial debris, preferentially to remove organic material or separate the organic material from the inorganic material comprised in the nanoparticle or at least one constituent of the compositions or magnetosomes and preferentially then be able to harvest the nanoparticle or at least one constituent of the composition or magnetosome mineral, preferentially comprised in the nanoparticle or at least one constituent of the compositions or magnetosomes.

In some cases, the purified nanoparticle or at least one constituent of the compositions or magnetosomes are nanoparticle or at least one constituent of the composition or magnetosome minerals.

In an embodiment of the invention, the nanoparticles or at least one constituent of the composition according to the invention are drugs, medical devices, cosmetic products, biological products, products used for research purposes, or products used to determine the properties of biological samples.

The invention relates to the composition or at least one constituent of the composition according to the invention comprising at least one nanoparticle or at least one constituent of the composition, wherein preferentially each nanoparticle or at least one constituent of the composition comprises preferentially an iron oxide mineral core preferentially surrounded by a coating,
wherein the composition or at least one constituent of the composition further preferentially comprises a protectant compound preferentially selected in the group consisting of:
   i) a cryoprotectant or protectant compound or protectant compound or a compound that preferentially protects or maintains at least one property of the composition or at least one constituent of the composition when it is cooled down, preferentially below 0°C,
   ii) a thermo-protectant or protectant compound or a compound that preferentially protects or maintains at least one property of the composition or at least one constituent of the composition when it is exposed to a temperature gradient, preferentially of more than 0.1, 1 or 10°C,
   iii) an oxydo-reduction-protectant or protectant compound or a compound that preferentially protects or maintains at least one property of the composition or at least one constituent of the composition when it is exposed to a reduction or an oxidation, preferentially resulting in a different oxidation state of the nanoparticle or at least one constituent of the composition,
   iv) a pressure-protectant or protectant compound or a compound that preferentially protects or maintains at least one property of the composition or at least one constituent of the composition when it is exposed to a pressure or pressure variation, preferentially a pressure larger than 10⁻¹⁰, 10⁻³, 1, 0, 1, 10³ or 10⁵ bar or atm or mbar or Pa,
   v) a pH-protectant or protectant compound or a compound that preferentially protects or maintains at least one property of the composition or at least one constituent of the composition when it is exposed to a pH variation, preferentially a pH variation of more than 0, 1, 3, 5, 10 or 14 pH unit(s),
   vi) a radiation-protectant or protectant compound or a compound that preferentially protects or maintains at least one property of the composition or at least one constituent of the composition when it is exposed to a radiation, preferentially a radiation selected in the group consisting of: : i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave.
wherein the at least one property of the composition or at least one constituent of the composition is preferentially selected in the group consisting of: i) a chain arrangement of at least two nanoparticle or at least one constituent of the compositions, ii) an activity, iii) the size, iv) the cohesion, v) the magnetic property, and vi) the composition of at least one constituent of the composition,
wherein preferentially the chain arrangement of the two nanoparticles or constituents of the compositions is maintained when at least two nanoparticle or at least one constituent of the compositions are or remain arranged in changed in the composition or at least one constituent of the composition, wherein preferentially the activity of at least one constituent of the composition is maintained when it does not decrease or disappear,
wherein preferentially the size of at least one constituent of the is maintained when it does not vary by more than 50% or by more than 0.1, 1, 10 or 10⁶ nm,
wherein preferentially the cohesion of at least one constituent of the composition is maintained when at least 1, 2, or 3 or all constituents of the composition or at least one constituent of the composition remain in the composition or at least one constituent of the composition,
wherein preferentially the magnetic property of at least one constituent of the composition is maintained when the coercivity, magnetization, remanent magnetization, saturation magnetization does not vary by more than 50% or by more than 100 mT or 10³ Oe or Oe per mg of constituent(s) or when at least one constituent of the composition does not change from one magnetic state to another magnetic state,
wherein preferentially the composition or at least one constituent of the composition is maintained when less than 1, 5, 10, 10⁵ or 10¹⁰ atom(s) preferentially per constituent or 50% of constituents remain in the composition or at least one constituent of the composition,
wherein preferentially the activity of the composition or at least one constituent of the composition is preferentially selected in the group consisting of: a therapeutic, immunological, pharmacological, chemotherapeutical, metabolic, thermal, vaccine, hyperthermia, cryo-thermal, ablative, prophylactic, and diagnosis activity of at least one constituent of the composition or at least one constituent of the composition,
wherein preferentially the magnetic state is selected in the group consisting of: a diamagnetic, paramagnetic, superparamagnetic, ferromagnetic, and ferrimagnetic state.
wherein the core preferentially comprises a first center activity, C_{A1},
   and/or
wherein preferentially the coating comprises a second center of activity, C_{A2},
wherein preferentially C_{A1} and C_{A2} are different compounds,
   wherein preferentially C_{A1} and C_{A2} are selected in the group consisting of:
   A) a radio-sensitizer or amplifier of radiation, a radio-photosensitizer or amplifier of light radiation, an acoustic sensitizer or amplifier of acoustic radiation or wave, a sonosensitizer or amplificatory of acoustic wave, a particle radiation sensitizer or amplifier of particle radiation, where the particle comprises (or not) a mass, it is a thermal-sensitizer or amplifier of heat or cold or thermal treatment, an amplifier of the medical effect of a compound,
      and
   B) an attenuator of radiation, of light radiation, of acoustic radiation or wave, of particle radiation, where the particle comprises (or not) a mass, of heat or cold, of thermal treatment, and/or of the medical effect of a compound,
wherein preferentially the volume occupied by the protectant compound in the composition or at least one constituent of the composition is larger than the volume occupied by at least one chain in the composition or at least one constituent of the composition, preferentially by a factor of preferentially at least 1, 2, 5, 10 or 10³,
wherein preferentially the percentage in mass of protectant compound in the composition or at least one constituent of the composition is comprised between 0.5 and 50 %,
wherein preferentially the distance separating C_{A1} and C_{A2} in the composition or at least one constituent of the composition is preferentially larger than 1 nm,
wherein preferentially the composition or at least one constituent of the composition is in the form of a powder or a liquid suspension,
wherein preferentially the composition or at least one constituent of the composition is isotonic,

The invention also relates to the composition or at least one constituent of the composition according to invention, wherein preferentially an amplifier of radiation is at least one atom that is the center of production of radical species, which is preferentially activated or produces radical species when it is exposed to radiation.

The invention also relates to the composition or at least one constituent of the composition according to the invention, wherein preferentially an attenuator of radiation is at least one atom that is the center of capture of radical species, which is preferentially activated or captures radical species when it is exposed to radiation.

The invention also relates to the composition or at least one constituent of the composition according to the invention, wherein C_{A1} is preferentially a first center of activity or free radical production or capture, C_{1FRPC}, and/or C_{A2} is preferentially a second center of activity or free radical production or capture, C_{2FRPC}, which is/are preferentially selected in the group consisting of:
vii) another metal than iron such as Zinc or Aluminum,
viii) another metal oxide than iron oxide such as Zinc Oxide or Aluminum oxide,
ix) at least one anti-oxidizing compound,
x) at least one oxidizing compound.

The invention relates to the composition or at least one constituent of the composition according to the invention, wherein C_{A1} and/or C_{A2} is/are preferentially selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or at least one constituent of the composition or nanomaterial, 56) Natural products or compounds, 57) Non-Steroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66) Silicon phthalocyanine (PC4), 67) RLP068, 68) Porfimer sodium, 69) Porfins, 69) Porphyrins, such as 5,10,15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene, 91) ABS-FA, 92) Acrylonitrile Butadiene Styrene, 93) Styrene, 94) Folic acid, 95) AIMP NP, aminoacyl tRNA synthetase complex-interacting multifunctional protein, 96) Au Nanomaterial, 97) gold, 98) Au-MnO nanomaterial, 99) manganese oxide, 100) Antineoplastic drugs, 101) NSAIDs, 102) nonsteroidal anti-inflammatory drug, 103) Artemether, 104) 5-ALA (5-aminolevulinic acid), 105) Acridine, Acridine Orange, 106) Au-doped TiO2, 107) Carbon based nanomaterial, 108) carbon nanotube, 109) Chlorine, 110) Ce6, 111) PTX, Paclitaxel, 112) chemotherapeutic drug or compound, 113) infrared dye or IR783, 114) Curcumin, 115) Cyanine or Cu-Cyanine, 116) DHMS, 117) dimethylsulfure, 118) Docetaxel, 119) chemotherapeutic drug or compound, 119) DOX/Mn-TPPS@RBCS, 120) doxorubicin, 121) manganese, 122) blood cell, 123) red blood cell, cell, 124) polymer, 125) elastomer, 126) Erythosin or Erythosin B, 127) FA or FA-OI or FA-OI NP or folic acid, 128) F3-PLGA@MB/Gd NPs, 129) poly(lactic-co-glycolic acid), 130) gadolinium, 131) Fe-TiO2 or titanium oxide, 132) Fe-VS2, 133) iron, 134) vanadium disulfide, 135) FMSNs-DOX, 136) silica, 137) HCQ, 138) hydrochloroquine, 139) HP, 140) hematoporphyrin, 141) HMME, 142) hematoporphyrin monomethyl ether, 143) HSYA or Hydroxysafflor yellow A, 144) Hypocrellin, Hypocrellin B, 145) IR780, 146) Levofloxacin, 147) LIP3 or Lithium phosphide, 148) Lithium, 149) Liposome or Liposomal nanomaterial, 150) Lomefoxacin, 151) MG@P NPs, 152) MnP or Manganese peroxidase, 153) MnTTP-HSAs, 154) HSA-wrapped metal-porphyrin complex, 155) albumin, 156) MnWOx, 157) MnWOx-PEG, 158), PEG, 159) metallic or bimetallic or multi-metallic compound preferentially oxide, 160) Mn (III)-HFs, 161) managense, 162) hemoporfin, 163) nano-compound or nanoroad or nanoflower or nanowire or quantum dot, 164) Noble or halogen or Hydrogen or alkali metals or Alkaline earth metals or Triels or Tetrels or Pnicto-gen or Chal-co-gens or metal or gas or liquid or solid preferentially nanomaterial, 165) oxygen indyocyanine preferentially nanoparticle or at least one constituent of the composition, 166) Phthalocyanines, 167) PIO or Pioglitazone, 168) Polymeric nanomaterial, 169) Porphyrin, 170) Pt-doped TiO2, 171) R837, 172) Rose Bengal, 173) Sparfloxacin, 174) TAPP or 5,10,15,20-tetrakis (4-aminophenyl) porphyrin, 175) TiO2 or titanium dioxide nanomaterial, 176) TCPP, isomer, or Tris(1-chloro-2-propyl) phosphate 177) TPI or Thermoplastic Polyimide or thermoplastic polymer, 178) TPZ or Tirapazamine, 179) Transition metal oxide, 180) nanoparticle or at least one constituent of the composition or Janus nanoparticle or at least one constituent of the composition, and 181) Xanthones, 182) AQ4N, 183) Apaziquone (E09), 184) Bromodeoxyuridine, 185) Carbogen, 186) Cetuximab, 187) Chemotherapeutic drug or compound, 188) Chlorpromazine, 189) C-reactive peptide, 190) Curcumin, 191) Diamide, 192) Diethylmaeate, 193) Dihydroartemisinin, 194) Docetaxel, 195) ECI301, 196) Etanidazole, 197) Fludarabine, 198) 5-Fluorouracil, 199) Fluorodeoxyuridine, 200) Gadolynium, 201) Gemcitabine, 202) HER-3 ADC, 203) HSP, 204) Hydrogen peroxide, 205) Hydroxyurea, 206) Hyperbaric oxygen, 207) Hyperthermia, 208) Hypoxic cell cytotoxic agent, 209) Irinotecan, 210) lanthanide-doped radiosensitizer-based metal-phenolic network, 211) Lidocaine, 212) Lododeoxyuridine, 213) Metronidazole, 214) misonidazole, 215) etanidazole, 216) nimorazole, 217) N-Ethylmalemide, 218) malmeide, 219) ethylmalmeide, 220) Nanomaterial such as those consisting of or composed of at least partly or fully gold, silver, bismuth, gadolinium, polysiloxane matrix and gadolinium chelates, hafnium, Tantalum, Zinc, Gadolinium, Germanium, Chromium, Praseodymium, Silicon, iron, platinum, cobalt, manganese, magnesium, iron, Titanium, carbon nanotube, quantum dot, nanoroad, Triflate, or metal oxide, 221) Nelfinavir, 222) Nicotinamide, 223) Nimotuzumab, 224) RNA, or miRNA, or miR-201, or miR-205, or miR-144-5p, or miR-146a-5p, or miR-150, or miR-99a, or miR-139-5p, or miR-320a, 225) Membrane active agent, 226) Mitomycin-C or Mitomycin, 227) Motexafin, 228) NBTXR3, 229) Oligonucleotide, 230) Paclitaxel, 231) Papaverine or Papaverine hydrochloride, 232) Paraxonase-2, 233) Pocaine, 234) Porfiromycin (POR), 235) Protein, 236) Peptide, 237) Radiosensitizing nucleosides or compounds, 238) Resveratrol, 239) RRx-001, 240) SiRNa, 241) Suppressors of sulfhydral groups, 242) SYM004, 243) Texaphyrins, 244) TH-302, and 245) Tirapazamine.

The invention also relates to the composition or at least one constituent of the composition according to the invention, wherein at least two nanoparticle or at least one constituent of the compositions in the composition preferentially arranges in a chain or remained arranged in a chain, preferentially at or below 0°C.

The invention also relates to the composition or at least one constituent of the composition according to the invention, wherein the nanoparticle or at least one constituent of the composition core is preferentially predominantly or in majority metallic, and preferentially the coating of the nanoparticle or at least one constituent of the composition is predominantly or in majority organic or non-metallic. The invention also relates to the composition or at least one constituent of the composition according to the invention, wherein preferentially the core is synthesized by a living organism or nanoparticle-producing cells, and/or preferentially the coating is not synthesized by a living organism or nanoparticle or at least one constituent of the composition producing cells,
wherein the nanoparticle-producing cell is preferentially a magnetotactic bacterium.

The invention also relates to the composition or at least one constituent of the composition according to the invention, wherein preferentially C_{1FRPC} and/or C_{2FRPC} is/are is/are :
A) photosensitizer(s), preferentially selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or at least one constituent of the composition or nanomaterial, 56) Natural products or compounds, 57) Non-Steroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66) Silicon phthalocyanine (PC4), 67) RLP068, 68) Porfimer sodium, 69) Porfins, 69) Porphyrins, such as 5,10,15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene,
   and/or
B) sonosensitizer(s), preferentially selected in the group consisting of: 1) ABS-FA, 2) Acrylonitrile Butadiene Styrene, 3) Styrene, 4) Folic acid, 5) AIMP NP, aminoacyl tRNA synthetase complex-interacting multifunctional protein, 6) Au Nanomaterial, 7) gold, 8) Au-MnO nanomaterial, 9) manganese oxide, 10) Antineoplastic drugs, 11) NSAIDs, 12) nonsteroidal anti-inflammatory drug, 13) Artemether, 14) 5-ALA (5-aminolevulinic acid), 15) Acridine, Acridine Orange, 16) Au-doped TiO2, 17) Carbon based nanomaterial, 18) carbon nanotube, 19) Chlorine, 20) Ce6, 21) PTX, Paclitaxel, 22) chemotherapeutic drug or compound, 23) infrared dye or IR783, 24) Curcumin, 25) Cyanine or Cu-Cyanine, 26) DHMS, 27) dimethylsulfure, 28) Docetaxel, 29) chemotherapeutic drug or compound, 30) DOX/Mn-TPPS@RBCS, 31) doxorubicin, 32) manganese, 33) blood cell, 34) red blood cell, cell, 35) polymer, 36) elastomer, 37) Erythosin or Erythosin B, 38) FA or FA-OI or FA-OI NP or folic acid, 39) F3-PLGA@MB/Gd NPs, 40) poly(lactic-co-glycolic acid), 41) gadolinium, 42) Fe-TiO2 or titanium oxide, 43) Fe-VS₂, 44) iron, 45) vanadium disulfide, 46) FMSNs-DOX, 47) silica, 48) HCQ, 49) hydrochloroquine, 50) HP, 51) hematoporphyrin, 52) HMME, 53) hematoporphyrin monomethyl ether, 54) HSYA or Hydroxysafflor yellow A, 55) Hypocrellin, Hypocrellin B, 56) IR780, 57) Levofloxacin, 58) LIP3 or Lithium phosphide, 59) Lithium, 60) Liposome or Liposomal nanomaterial, 61) Lomefoxacin,, 62) MG@P NPs, 63) MnP or Manganese peroxidase, 64) MnTTP-HSAs, 65) HSA-wrapped metal-porphyrin complex, 66) albumin, 67) MnWOx, 68) MnWOx-PEG, 69), PEG, 70) bimetallic oxide, 71) Mn (III)-HFs, 72) managense, hemoporfin, 73) Nanoroads, 74) Noble metal nanomaterial, 75) OI NP or oxygen indyocyanine, 76) Phthalocyanines, 77) PIO or Pioglitazone, 78) Polymeric nanomaterial, 79) Porphyrin, 80) Pt-doped TiO₂, 81) R837, 82) Rose Bengal, 83) Sparfloxacin,, 84) TAPP or 5,10,15,20-tetrakis (4-aminophenyl) porphyrin, 85) TiO₂ or titanium dioxide nanomaterial, 86) TCPP, isomer, or Tris(1-chloro-2-propyl) phosphate 87) TPI or Thermoplastic Polyimide or thermoplastic polymer, 88) TPZ or Tirapazamine, 89) Transition metal oxide, 90) nanoparticle or at least one constituent of the composition or Janus nanoparticle or at least one constituent of the composition, and 91) Xanthones, and/or
C) radio-sensitizer, preferentially selected in the group consisting of: 1) AMG102, 2) AQ4N, 3) Apaziquone (E09), 4) Bromodeoxyuridine, 5) Carbogen, 6) Cetuximab, 7) Chemotherapeutic drug or compound, 8) Chlorpromazine, 9) C-reactive peptide, 10) Curcumin, 11) Diamide, 12) Diethylmaeate,, 13) Dihydroartemisinin, 14) Docetaxel, 15) ECI301, 16) Etanidazole, 17) Fludarabine, 18) 5-Fluorouracil, 19) Fluorodeoxyuridine, 20) Gadolynium, 21) Gemcitabine, 22) HER-3 ADC, 23) HSP, 24) Hydrogen peroxide, 25) Hydroxyurea, 26) Hyperbaric oxygen, 27) Hyperthermia, 28) Hypoxic cell cytotoxic agent, 29) Irinotecan, 30) lanthanide-doped radiosensitizer-based metal-phenolic network, 31) Lidocaine, 32) Lododeoxyuridine, 33) Metronidazole, 34) misonidazole, 35) etanidazole, 36) nimorazole, 37) N-Ethylmalemide, 38) malmeide, 39) ethylmalmeide, 40) Nanomaterial such as those consisting of or composed of at least partly or fully gold , silver, bismuth, gadolinium, polysiloxane matrix and gadolinium chelates, hafnium, Tantalum, Zinc, Gadolinium, Germanium, Chromium, Praseodymium, Silicon, iron, platinum, cobalt, manganese, magnesium, iron, Titanium, carbon nanotube, quantum dot, nanoroad, Triflate, or metal oxide, 41) Nelfinavir, 42) Nicotinamide, 43) Nimotuzumab, 44) RNA, or miRNA, or miR-201, or miR-205, or miR-144-5p, or miR-146a-5p, or miR-150, or miR-99a, or miR-139-5p, or miR-320a, 45) Membrane active agent, 46) Mitomycin-C or Mitomycin, 47) Motexafin, 48) NBTXR3, 49) Oligonucleotide, 50) Paclitaxel, 51) Papaverine or Papaverine hydrochloride, 52) Paraxonase-2, 53) Pocaine, 54) Porfiromycin (POR), 55) Protein, 56) Peptide, 57) Radiosensitizing nucleosides or compounds, 58) Resveratrol, 59) RRx-001, 60) SiRNa, 61) Suppressors of sulfhydral groups, 62) SYM004, 63) Texaphyrins, 64) TH-302, and 65) Tirapazamine.

The invention also relates to a method of fabrication of the composition or at least one constituent of the composition according to the invention, which comprises at least one of the following steps:
**i. Step 1** of preferentially amplifying magnetotactic bacteria, preferentially in at least one medium, comprising:
   1. preferentially the compounds necessary for the growth of magneto tactic bacteria and the production of magnetosomes, which are preferentially selected in the group consisting of:
      - a source of carbon preferentially selected from the group consisting of: at least one compound comprising at least one atom of carbon, lactic acid, Na lactate, lactic acid, acetate, glycolate, glucose, pyruvate, succinate, carbon dioxide, glycerol and combinations thereof, at a concentration preferentially comprised between 1 nM and 2 Mol/L;
      - a source of iron preferentially selected from the group consisting of: at least one compound comprising at least one atom of iron, iron citrate, iron quinate, iron chloride, iron sulfate, FeCl₃, and combinations thereof, at a concentration preferentially comprised between 1 nM and 2.10⁻³ Mol/L;
      - a source of nitrogen preferentially selected from the group consisting of: at least one compound comprising at least one atom of nitrogen, nitrate salt, nitrogen gas, ammonium, ammonia, ammonium salt, urea, an amino acid, ammonia gas, and combinations thereof, at a concentration preferentially comprised between 1 nM and 4 Mol/L;
      - a source of oxygen preferentially selected from the group consisting of: at least one compound comprising at least one atom of oxygen, oxygen or air or compressed air, preferentially in the form of a gas, the source of oxygen being in some cases bubbled or introduced to the growth medium, at a gas rate that is preferentially comprised between 5 mL of gas per minute and 50000 mL of gas per minute;
      - a source of phosphate preferentially consisting of at least one compound comprising at least one atom of phosphate, at a concentration preferentially comprised between 1 nM and 2.10 ¹ Mol/L;
      - a source of potassium preferentially consisting of at least one compound comprising at least one atom of potassium, at a concentration preferentially comprised between 1 nM and 2.10 ¹ Mol/L;
      - a source of sulfur or sulfate preferentially consisting of at least one compound comprising at least one atom of sulfur or sulfate, at a concentration preferentially comprised between 1 nM and 4.10⁻¹ Mol/L;
      - a source of manganese preferentially consisting of at least one compound comprising at least one atom of manganese, at a concentration preferentially comprised between 1 nM and 4.10⁻¹ Mol/L;
      - a source of vitamin preferentially selected from the group consisting of: at least one compound comprising at least one vitamin, Biotin, Calcium, pantothenate, Folic acid, Inositol, Nicotinic acid, p-Aminobenzoic acid, Pyridoxine HCl, Riboflavin, Thiamine, Thiamine HCL and derivatives thereof and combinations thereof, at a concentration preferentially comprised between 1 nM and 10⁻⁴ Mol/L, and
      - a source of calcium preferentially consisting of at least one compound comprising at least one atom of calcium, at a concentration preferentially comprised between 1 nM and 10⁻¹ Mol/L.
   2. preferentially at least one compound necessary for doping the magnetosomes with C_{1FRPC} or C_{A1} or another metal than iron, preferentially zinc or aluminum, for example a source of zinc, preferentially zinc sulfate or zinc citrate or zinc chlorate or zinc quinate.
**ii. Step 2** of preferentially extracting magnetosomes from magnetotactic bacteria and/or of preferentially purifying the extracted magnetosomes preferentially by heating them preferentially to yield magnetosome minerals preferentially comprising a percentage in mass of organic material coming from magnetotactic bacteria that is preferentially lower than 50 or 10 or 1%,
**iii. Step 3** of preferentially coating the magnetosome minerals preferentially with a coating material comprising the compound C_{A2} or C_{2FRPC} by mixing the magnetosome minerals with the coating material, where the mixing is preferentially realized in at least one of the following conditions:
   i) under sonication,
   ii) under the application of radiation,
   iii) under temperature variation,
   iv) under pH changes or adjustment,
   v) under oxidoreduction potential adjustment, and
   vi) using a ratio between the quantity or mass of magnetosome minerals and the quantity or mass of coating material, preferentially of compound D, that is adjusted or varied or larger than 1,
iv. **Step 4** of preferentially adding at least one cryoprotectant or protectant compound or protectant compound to the coated magnetosome minerals obtained at the end of step 3,
v. **Step 5** of preferentially lyophilizing or dehydrating or drying or desiccating the composition or at least one constituent of the composition obtained at the end of step 4,
vi. **Step 6** of preferentially re-suspending the lyophilized composition or at least one constituent of the composition obtained of step **5,** preferentially in water.

The invention also relates to a method for storing the composition or at least one constituent of the composition according to invention, which preferentially comprises at least one of the following step(s):
- **Step 1:** preferentially choosing or preparing the composition or at least one constituent of the composition in the form of a liquid suspension,
- **Step 2:** preferentially lyophilizing, desiccating, dehydrating the composition or at least one constituent of the composition, or removing water from the composition or at least one constituent of the composition,
- **Step3:** preferentially Storing the composition or at least one constituent of the composition preferentially in a powder form, preferentially during more than 3 months,
   and
- **Step 4:** preferentially suspending or resuspending the composition or at least one constituent of the composition, preferentially in water.

In some cases, the at least one constituent of the composition is selected in the group consisting of: i) the nanoparticle or at least one constituent of the composition, ii) the nanoparticle or at least one constituent of the composition coating, iii) the nanoparticle or at least one constituent of the composition core, iv) the cryoprotectant or protectant compound or protectant compound, v) the other compound, vi) at least one link or bond or force or interaction of or between at least one or two constituent(s) of the composition, vii) a solute, viii) a solvent, ix) an excipient, x) an active part or principle, xi) an inert part, xii) an organic or carbon or carbonaceous part, xiii) an inorganic or metallic part, xiv) a medical device or drug, xv) a pharmaceutical compound, xvi) an immunological compound, xvii) a metabolic compound, xviii) a chemotherapeutic compound, xix) a surgical compound, xx) a radio-sensitizer, xxi) a contrast agent, and xxii) a sonosensitizer of the composition or at least one constituent of the composition.

In some cases, the constituent is comprised in the organic or inorganic part of the composition or at least one constituent of the composition.

In some cases, the constituent is comprised in the inert or active part of the composition or at least one constituent of the composition.

The invention also relates to a composition or at least one constituent of the composition comprising at least one nanoparticle or at least one constituent of the composition or at least one chain of at least two nanoparticle or at least one constituent of the compositions,
wherein preferentially the at least one nanoparticle or at least one constituent of the composition or each nanoparticle or at least one constituent of the composition in the chain comprises a core, preferentially a mineral core, preferentially a metallic core, preferentially a crystallized core, most preferentially an iron oxide and/or mineral core, preferentially surrounded preferentially partly or fully by a coating,
wherein the composition or at least one constituent of the composition preferentially further comprises a cryoprotectant or protectant compound or protectant compound,
wherein preferentially the volume occupied by the cryoprotectant or protectant compound in the composition or at least one constituent of the composition is larger than the volume occupied by the at least one nanoparticle or at least one constituent of the composition or the at least one chain in the composition or at least one constituent of the composition, preferentially by a factor preferentially α of at least 1, 2, 5, 10 or 10³,
wherein preferentially the percentage in mass of cryoprotectant or protectant compound in the composition or at least one constituent of the composition is comprised between 0.5 and 50 %.

The invention also relates to the composition or at least one constituent of the composition according to the invention comprising at least one nanoparticle or at least one constituent of the composition or at least one constituent of the composition or at least one constituent of the composition or at least one chain of at least two nanoparticle or at least one constituent of the compositions, wherein preferentially each nanoparticle or at least one constituent of the composition in the chain comprises an iron oxide mineral core surrounded by a coating,
wherein preferentially the composition or at least one constituent of the composition further comprises a cryoprotectant or protectant compound or protectant compound,
wherein preferentially the volume occupied by the cryoprotectant or protectant compound or protectant compound in the composition or at least one constituent of the composition is larger than the volume occupied by at least one chain in the composition or at least one constituent of the composition, by a factor of at least 1, 2, 5, 10 or 10³,
wherein preferentially the percentage in mass of cryoprotectant or protectant compound or protectant compound in the composition or at least one constituent of the composition is comprised between 0.5 and 50 %,
wherein preferentially the composition or at least one constituent of the composition is isotonic.

In one embodiment, the protectant compound is selected in the group consisting of: i) a cryoprotectant or protectant compound, ii) a thermo-protectant, iii) an oxydo-protectant, iv) a chain-protectant, v) an activity-protectant and vi) a protectant of the composition or at least one constituent of the composition or size or cohesion or at least one property or magnetic property of at least one nanoparticle or at least one constituent of the composition or composition or at least one constituent of the composition or constituent of the composition or at least one constituent of the composition.

In some cases, an activity-protectant compound is a compound that protects the activity, preferentially a therapeutic, immunological, pharmacological, chemotherapeutical, metabolic, thermal, and/or vaccine-type activity of the nanoparticle or at least one constituent of the composition or of at least one constituent of the composition, i.e. preferentially without the activity-protectant compound the activity of the nanoparticle or at least one constituent of the composition is diminished or decreased or lost partly or fully preferentially over time or under thermal variation or under the application of a radiation on the composition or at least one constituent of the composition.

In some cases, the composition or at least one constituent of the composition can comprise an active part and an inactive part or different parts with different types of activity, for example one part with a medical or thermal activity such as the part comprising the nanoparticle or at least one constituent of the composition or nanoparticle core or nanoparticle coating and another part with an activity consisting in preserving the at least one property of the nanoparticle or at least one constituent of the composition such as the part comprising the cryoprotectant or protectant compound.

In some cases, the composition or at least one constituent of the composition is of high purity, preferentially in terms of metallic composition or of composition in iron.

In some cases, a high purity composition designates a composition that comprises more than 50, 90, 96, 99 or 99% of iron preferentially in terms of metallic composition.

In some cases, a cryoprotectant or protectant compound can be a compound that protects or maintains or avoids a change of at least one property of the composition or at least one constituent of the composition or nanoparticle, preferentially when the composition or nanoparticle or at least one constituent of the composition is cooled down, preferentially by at least or below 100, 10, 0, -1, -20, - 50, -100, or -273 °C.

In some cases, a thermo-protectant can be a compound that protects or maintains or avoids a change of at least one property of the composition or at least one constituent of the composition or nanoparticle or at least one constituent of the composition, preferentially when the composition or at least one constituent of the composition or nanoparticle is subject to a temperature gradient, preferentially by at least 0, 1, 2, 5, 10 or 100 °C.

In some cases, an oxydo-protectant can be a compound that protects or maintains or avoids a change of at least one property of the composition or at least one constituent of the composition or nanoparticle or at least one constituent of the composition such as the oxidation state of the nanoparticle or at least one constituent of the composition or composition, preferentially when the composition or at least one constituent of the composition or nanoparticle or at least one constituent of the composition is subject to oxidation or reduction.

In some cases, a chain-protectant can be a compound that protects or maintains or avoids a change or destruction or diminution or increase or variation in size of the chain of nanoparticle or at least one constituent of the composition, preferentially when the composition or at least one constituent of the composition or nanoparticle is subject to oxidation or reduction or subject to a temperature gradient or is cooled down or is exposed to a radiation.

In some cases, a size-protectant can be a compound that protects or maintains or avoids a change or destruction or diminution or increase or variation in size of the nanoparticle or at least one constituent of the composition, preferentially by more than 0, 1, 10, 100, 10³ or 10⁵ nm, preferentially when the composition or at least one constituent of the composition or nanoparticle is subject to oxidation or reduction or subject to a temperature gradient or is cooled down or is exposed to a radiation.

In some cases, a composition or at least one constituent of the composition-protectant can be a compound that protects or maintains or avoids a change or destruction or diminution or increase or variation in composition of the nanoparticle or at least one constituent of the composition, preferentially by more than 1, 10, 99, 100, 10³, 10⁵ or 10¹⁰ atoms or percent of atoms, preferentially when the composition or at least one constituent of the composition or nanoparticle is subject to oxidation or reduction or subject to a temperature gradient or is cooled down or is exposed to a radiation.

In some cases, a cohesion-protectant can be a compound that protects or maintains or avoids a change or destruction or diminution or increase or variation in cohesion of the nanoparticle or at least one constituent of the composition or composition, preferentially when the composition or at least one constituent of the composition or nanoparticle is subject to oxidation or reduction or subject to a temperature gradient or is cooled down or is exposed to a radiation.

In some cases, the cohesion of the nanoparticle or at least one constituent of the composition can be at least one interaction or at least one bond or interaction or force or link between at least two constituents of the composition, which preferentially maintain the cohesion or assembly or at least one property or the content of the composition or at least one constituent of the composition or nanoparticle or at least one constituent of the composition.

In some cases, a magnetic property-protectant can be a compound that protects or maintains or avoids a change or destruction or diminution or increase or variation in magnetic property or coercivity or magnetization or saturating magnetization of the nanoparticle or at least one constituent of the composition or composition or at least one constituent of the composition, preferentially by more than 1, 10, 99, 100, 10³, 10⁵ or 10¹⁰ Oe or mT or percent of this property, preferentially when the composition or at least one constituent of the composition or nanoparticle or at least one constituent of the composition is subject to oxidation or reduction or subject to a temperature gradient or is cooled down or is exposed to a radiation.

In some cases, at least one magnetic property of the nanoparticle or at least one constituent of the composition or composition or at least one constituent of the composition can be a diamagnetic, paramagnetic, superparamagnetic, ferromagnetic or ferrimagnetic property.

In some cases, the at least one property of the nanoparticle or at least one constituent of the composition or composition or at least one constituent of the composition exists or is measured at a temperature lower than 10⁵⁰, 10⁵, 10³, 100, 50, 10, 5, 2, 1, 0.1 or 0 K (Kelvin) or °C (Celsius degree).

In some cases, the at least one property of the nanoparticle or at least one constituent of the composition or composition or at least one constituent of the composition exists or is measured at a temperature larger than 0, 0.1, 1, 5, 10, 50, 100, 10³, 10⁵ or 10¹⁰ K (Kelvin) or °C (Celsius degree).

In still some other cases, the nanoparticle or at least one constituent of the composition can result from the assembly of atoms, entities, compounds that don't have a nanometer size, preferentially before assembly or taken individually, or are preferentially smaller than 100, 10, 1 or 0.1 nm, and preferentially display a nanometer size following their assembly, or are preferentially larger than 0.1, 1, 10 or 100 nm. In still some other cases, the nanoparticle or at least one constituent of the composition can result from the dis-assembly of atoms, entities, compounds that don't have a nanometer size, preferentially before dis-assembly, or are preferentially larger than 0.1, 1, 10 or 100 nm 1 µm, and preferentially display a nanometer size following their dis-assembly, or are preferentially smaller 1µm, 100, 10, 1 or 0.1 nm. In still some other cases, assembly and/or dis-assembly occur(s) inside the body part or outside the body part.

In still some other cases, the composition or at least one constituent of the composition comprises at least one chemical element or a majority of such element or a percentage of mas of such element larger than or equal to 0, 1, 5, 10, 50, 90, or 99%.

In still some other cases, the composition or at least one constituent of the composition comprises at least one chemical element or a minority of such element or a percentage of mas of such element smaller than or equal to 100, 99, 75, 50, 10, 5, 2, 1 or 0%.

In still some other cases, the chemical element is selected in the group consisting of: Actinium, Aluminum, Americium, Antimony, Argon, Arsenic, Astatine, Barium, Berkelium, Beryllium, Bismuth, Bohrium, Boron, Bromine, Cadmium, Calcium, Californium, Carbon, Cerium, Cesium, Chlorine, Chromium, Cobalt, Copernicium, Copper, Curium, Darmstadtium, Dubnium, Dysprosium, Einsteinium, Erbium, Europium, Fermium, Flerovium, Fluorine, Francium, Gadolinium, Gallium, Germanium, Gold, Hafnium, Hassium, Helium, Holmium, Hydrogen, Indium Iodine, Iridium, Iron, Krypton Lanthanum, Lawrencium, Lead, Lithium, Livermorium, Lutetium, Magnesium, Manganese, Meitnerium, Mendelevium, Mercury, Molybdenum, Moscovium, Neodymium, Neon, Neptunium, Nickel, Nihonium, Niobium, Nitrogen, Nobelium, Oganesson, Osmium, Oxygen, Palladium, Phosphorus, Platinum, Plutonium, Polonium, Potassium, Praseodymium, Promethium, Protactinium, Radium, Radon, Rhenium, Rhodium, Roentgenium, Rubidium, Ruthenium, Rutherfordium, Samarium, Scandium, Seaborgium, Selenium, Silicon, Silver, Sodium, Strontium, Sulfur, Tantalum, Technetium, Tellurium, Tennessine, Terbium, Thallium, Thorium, Thulium, Tin, Titanium, Tungsten, Uranium, Vanadium, Xenon, Ytterbium, Yttrium, Zinc, and Zirconium.

In one embodiment of the invention, the composition or at least one constituent of the composition comprises the at least one nanoparticle or at least one constituent of the composition with the cryoprotectant or protectant compound or protectant compound and optionally a liquid such as water or a gas or a solid or at least one substance that is different from the nanoparticle or at least one constituent of the composition and/or cryoprotectant or protectant compound or protectant compound. Such substance preferentially disperses or suspends the at least one nanoparticle or at least one constituent of the composition preferentially with the cryoprotectant or protectant compound. Such substance and/or cryoprotectant and/or protectant compound preferentially embeds and/or surrounds and/or act(s) as a matrix surrounding the at least one nanoparticle or at least one constituent of the composition and/or cryoprotectant or protectant compound. Such substance preferentially has a different function than the cryoprotectant or protectant compound or protectant compound. For example, the substance could enable the dispersion or re(dispersion), preferentially homogenously, of the at least one nanoparticle or at least one constituent of the composition, preferentially before administration of the composition or at least one constituent of the composition to the body part, whereas the cryoprotectant or protectant compound could protect or maintain at least one property of the nanoparticle or at least one constituent of the composition, preferentially during storage of the composition or at least one constituent of the composition preferentially in a lyophilized or dehydrated or powder form.

In one embodiment of the invention, the at least one property of the composition or of at least one constituent of the composition is selected in the group consisting of: i) the chain arrangement or geometric figure or assembly of the at least two nanoparticles or constituents of the composition, ii) the composition of at least one constituent of the composition, preferentially maintained by maintaining at least 1, 5, 10, 50, 75, 100, 10³ or 10⁵ atom(s) or metal(s) or % of atom(s) or metal(s) or % in mass of atom(s) or metal(s) preferentially in the composition or at least one constituent of the composition, iii) preferentially maintained by maintaining the size of the nanoparticle or at least one constituent of the composition, preferentially by preventing a change in nanoparticle or at least one constituent of the composition size or in size of the at least one constituent of the composition or at least one constituent of the composition, preferentially by more than 1, 5, 10, 100, 10³ or 10⁵ nm, iii) the oxidation state of the nanoparticle or at least one constituent of the composition or of the at least one constituent of the composition or at least one constituent of the composition, iv) the at least one magnetic property, preferentially the ferrimagnetic property, of the nanoparticle or at least one constituent of the composition or of the at least one constituent of the composition or at least one constituent of the composition, v) the at least one center of activity or of free radical capture or production of the nanoparticle or at least one constituent of the composition or of the at least one constituent of the composition or at least one constituent of the composition, and vi) the isotonicity or osmolality of the composition or at least one constituent of the composition or of the at least one constituent of the composition or at least one constituent of the composition,
wherein preferentially the at least one property exists or is measured preferentially in some cases at or below the temperature of 10⁵, 10³, 10², 50, 20, 10, 5, 2, 1, 0, -5, -10, -50, -77, -100, -200 or -273 °C, in some other cases at or above the temperature of 10⁵, 10³, 10², 50, 20, 10, 5, 2, 1, 0, -5, -10, -50, -77, - 100, -200 or -273 °C.

In another embodiment of the invention, the at least one property of the composition or at least one constituent of the composition is maintained, or kept or protected, preferentially by the cryoprotectant or protectant compound or protectant compound, when:
i) preferentially at least two nanoparticle or at least one constituent of the compositions remain arranged in chains or in a geometric figure or in or within an assembly,
ii) preferentially the nanoparticle or at least one constituent of the composition size is maintained or does not vary more than 10⁵, 10³, 50, 20, 10, 5, 2, 1 or 0%,
iii) preferentially the nanoparticle or at least one constituent of the composition is maintained or does not change or induce a change by or of more than 1, 5, 10, 10³, 10⁵ or 10²⁰ atoms,
iv) preferentially, the magnetic property of the nanoparticle or at least one constituent of the composition, preferentially ferrimagnetic one, is maintained or at least one magnetic property such as the coercivity of the nanoparticle or at least one constituent of the composition, magnetization of the nanoparticle or at least one constituent of the composition, or saturating magnetization of nanoparticle or at least one constituent of the composition does not vary by more than: i) 10⁵, 10³, 50, 20, 10, 5, 2, 1 or 0%, ii) 10⁵, 10³, 50, 20, 10, 5, 2, 1 or 0 Oe, or iii) ii) 10⁵, 10³, 50, 20, 10, 5, 2, 1 or 0 emu or emu per gram or per mg of nanoparticle or at least one constituent of the composition.
v) preferentially, the oxidation state of the nanoparticle or at least one constituent of the composition is maintained or does not change or induce a change by or of more than 1, 5, 10, 10³, 10⁵ or 10²⁰ atoms, preferentially oxygen or of an atom that is oxidized or reduced,
vi) preferentially, at least one center of activity or free radical production or capture remains comprised in the nanoparticle or at least one constituent of the composition or composition or at least one constituent of the composition or remains active, i.e. preferentially able to produce or capture free radical preferentially under the application of a radiation,
   and/or
vii) preferentially, the isotonicity, osmolality, and/or at least one other property of the nanoparticle or at least one constituent of the composition does not change or vary by more than 10⁻³, 1, 5, 10, 25, 50, 75 or 100% where this percentage is preferentially equal to (P2-P1)/P1, preferentially measured in absolute terms or values, where P1 and P2 are values of isotonicity, osmolality, and/or at least one other property of the nanoparticle or at least one constituent of the composition or composition or at least one constituent of the composition at a temperature T1 and T2 or at two different states of the nanoparticle or at least one constituent of the composition, respectively.
preferentially for or when the temperature of the composition or at least one constituent of the composition or nanoparticle or at least one constituent of the composition is maintained at or decreased below or at the temperature of 10⁵, 10³, 10², 50, 20, 10, 5, 2, 1, 0, -5, -10, -50, -77, -100, -200 or -273 °C.

In some cases, the percentage of variation of the at least one other property of the nanoparticle or at least one constituent of the composition or composition or at least one constituent of the composition can be equal to (P2-P1)/P1, preferentially measured in absolute terms or values, where P1 and P2 are two different values of at least one other property of the nanoparticle or at least one constituent of the composition or composition or at least one constituent of the composition, preferentially measured when the nanoparticle or at least one constituent of the composition or composition or at least one constituent of the composition is at two different temperature or in two different conditions.

In some cases, the nanoparticle or at least one constituent of the composition is at least one of the following conditions: i) liquid, ii) solid, and iii) gaseous.

In some cases, the property of at least one nanoparticle or at least one constituent of the composition or chain can be: i) the size, diameter, surface or volume, of the nanoparticle or at least one constituent of the composition, ii) at least one magnetic property of the at least one nanoparticle or at least one constituent of the composition, iii) the composition preferentially metallic and/or organic or carbonaceous of the at least one nanoparticle or at least one constituent of the composition, iv) the cohesion of the nanoparticle or at least one constituent of the composition, v) the percentage in mass of at least one metal in the at least one nanoparticle or at least one constituent of the composition or chain, vi) the chain arrangement of the at least one nanoparticle or at least one constituent of the composition, vii) the number of nanoparticle or at least one constituent of the compositions preferentially in the chain or composition, viii) the surface charge of the at least one nanoparticle or at least one constituent of the composition or chain, ix) the percentage of water or humidity or solid or liquid or gaseous material in the at least one nanoparticle or at least one constituent of the composition or chain, x) the oxidation or redox state of the at least one nanoparticle or at least one constituent of the composition, xi) the coercivity or remanent magnetization or magnetization or saturating magnetization of the at least one nanoparticle or at least one constituent of the composition or chain, xiii) the core diameter or size or volume or surface area or ratio surface/volume of the at least one nanoparticle or at least one constituent of the composition, xiii) the coating thickness of the at least one nanoparticle or at least one constituent of the composition, and/or xiv) the isotonicity or osmolality of the at least one nanoparticle or at least one constituent of the composition or chain.

In some cases, the at least one nanoparticle or at least one constituent of the composition and preferentially the cryoprotectant or protectant compound can be surrounded by or embedded in or mixed with water or liquid or solid or gas or matrix, or surfactant or solvent, which preferentially enables or favors or triggers the dispersion or suspension, preferentially within a homogenous manner, of the at least one nanoparticle or at least one constituent of the composition preferentially of or in the composition or at least one constituent of the composition.

The invention also relates to the composition or at least one constituent of the composition according to the invention, wherein the composition or at least one constituent of the composition comprises an organic part and/or an inorganic part, wherein the inorganic part preferentially comprises the core of the nanoparticle or at least one constituent of the composition, wherein the organic part preferentially comprises the coating of the nanoparticle or at least one constituent of the composition and/or the cryoprotectant or protectant compound, and wherein the percentage in mass of the inorganic part is preferentially larger, preferentially a factor ξ, than the percentage in mass of the organic part.

In some cases, the organic part comprises more than 0, 10⁻¹⁰, 1, 5, 10, 35, 50, 75, 80, 90 or 100% in mass or volume of carbon or carbonaceous material.

In some other cases, the inorganic part comprises less than 100, 99, 80, 75, 50, 25, 10, 5, 2, 1 or 0% in mass or volume of carbon or carbonaceous material.

In still some other cases, the organic part comprises more carbon or carbonaceous material than the inorganic part.

In some cases, the factor ξ is equal to I2/O1, where I2 is the percentage in mass of the inorganic part in the composition or at least one constituent of the composition and O1 is the percentage in mass of the organic part in the composition or at least one constituent of the composition, preferentially measured in the absence of or with minimal water or liquid or gas.

In some cases, ξ is larger than or equal to 1, 2, 5, 10, 100, 10³ or 10⁵.

In some other cases, ξ is smaller than or equal to 10⁵, 10³, 100, 10, 5, 2 or 1.

The invention also relates to the composition or at least one constituent of the composition according to the invention, comprising i) and/or ii):
i) the at least one nanoparticle or at least one constituent of the composition comprising a) and/or b):
   a) a core with at least one property selected in the group consisting of: i) a composition or at least one constituent of the composition of at least one metal or metal oxide, preferentially iron oxide, most preferentially maghemite or magnetite, most preferentially essentially maghemite, preferentially with a percentage in mass of more than 0, 1, 50, 90, 99.6 % of iron or zinc or manganese in terms of metallic composition or at least one constituent of the composition, ii) a size larger than 0, 1, 2, 5, 10, 20, 30 or 35 nm, and iii) a percentage in mass of organic material, preferentially of bacterial origin, preferentially non-denatured, that is lower than 100, 75, 50, 20, 5, 2 or 0%,
      and
   b) a surrounding coating, preferentially with a thickness lower than 100, 10 or 5 nm or lower than the diameter of the core of the nanoparticle or at least one constituent of the composition, and
ii) a cryoprotectant or protectant compound or protectant compound, preferentially sorbitol, preferentially surrounding the at least one nanoparticle or at least one constituent of the composition,
   where the percentages in mass of the nanoparticle or at least one constituent of the composition core, nanoparticle or at least one constituent of the composition coating and/or cryoprotectant or protectant compound, preferentially in or of the composition or at least one constituent of the composition, most preferentially comprised in the dried or de-hydrated composition or at least one constituent of the composition, has(have) at least one property selected in the group consisting of:
   a) The percentage in mass of the nanoparticle or at least one constituent of the composition core is larger than the percentages in mass of the nanoparticle or at least one constituent of the composition coating and/or cryoprotectant or protectant compound or protectant compound, preferentially by a factor a₁,
   b) The percentage in mass of carbon or carbonaceous material of the nanoparticle or at least one constituent of the composition core is lower than the percentage(s) in mass of carbon or carbonaceous material of the nanoparticle or at least one constituent of the composition coating and/or cryoprotectant or protectant compound or protectant compound, preferentially by a factor a₂,
   c) The percentage in iron or metal mass of the nanoparticle or at least one constituent of the composition core is larger than the percentage(s) in iron or metal mass of the nanoparticle or at least one constituent of the composition coating and/or cryoprotectant or protectant compound, or protectant compound preferentially by a factor a₃,

wherein preferentially the strength of the bonds or interactions between the coating and core of the nanoparticle or at least one constituent of the composition is stronger than the strength of the bond or interaction between the cryoprotectant or protectant compound and the coating and/or core of the nanoparticle or at least one constituent of the composition or the strength of the bonds or interactions between the coating and core of the nanoparticle or at least one constituent of the composition is preferentially strong while the strength of the bond or interaction between the cryoprotectant or protectant compound and the coating and/or core of the nanoparticle or at least one constituent of the composition is preferentially weak,
and/or wherein preferentially the coating and core form a complex,
and/or wherein preferentially the cryoprotectant or protectant compound and nanoparticle or at least one constituent of the composition don't form a complex.

In some cases, the percentages in mass of the nanoparticle or at least one constituent of the composition, nanoparticle or at least one constituent of the composition core, nanoparticle or at least one constituent of the composition coating and/or cryoprotectant or protectant compound or protectant compound in the composition or at least one constituent of the composition is/are larger than or equal to 0, 10⁻¹⁰, 1, 5, 10, 25, 30, 50, 75, 80, 90, 99.99 or 100%.

In some other cases, the percentages in mass of the nanoparticle or at least one constituent of the composition, nanoparticle or at least one constituent of the composition core, nanoparticle or at least one constituent of the composition coating and/or cryoprotectant or protectant compound or protectant compound in the composition or at least one constituent of the composition is/are smaller than or equal to 100, 99.99, 90, 80, 70, 75, 50, 25, 10, 1, 0.1 or 0%.

In some cases, a₁ = PM1/PM2, where PM1 is the percentage in mass of the nanoparticle or at least one constituent of the composition core and PM2 is the percentages in mass of the nanoparticle or at least one constituent of the composition coating and/or cryoprotectant or protectant compound.

In some other cases, a₂ = PM3/PM4, where PM3 is the percentage in mass of carbon or carbonaceous material of the nanoparticle or at least one constituent of the composition core and PM4 is the percentage in mass of carbon or carbonaceous material of the nanoparticle or at least one constituent of the composition coating and/or cryoprotectant or protectant compound.

In still some other cases, as = PM5/PM6, where PM5 is the percentage in iron or metal mass of the nanoparticle or at least one constituent of the composition core and PM6 is the percentage in iron or metal mass of the nanoparticle or at least one constituent of the composition coating and/or cryoprotectant or protectant compound.

In some cases, a₁, a₂, and/or a₃ is/are larger than or equal to 0, 10⁻¹⁰, 1, 5, 10, 25, 50, 100, 10³ or 10⁵. In some other cases, a₁, a₂, and/or as is/are smaller than or equal to 10⁴⁰, 10⁵⁰, 100, 50, 25, 10, 5, 2, 1 or 0.

In one embodiment of the invention, the strength of the bonds or interactions between the coating and core of the nanoparticle or at least one constituent of the composition is strong when the core and coating of the nanoparticle or at least one constituent of the composition can be attracted or moved preferentially together by a magnet preferentially of strength stronger than 10⁻⁶, 10⁻³, 10⁻¹ or 1 T. In some cases, such bonds or interactions can be designated as bonds or interactions that ensure the cohesion of the nanoparticle or at least one constituent of the composition preferentially by being resistant to or maintained in the presence of a force preferentially a magnetic force preferentially applied by a magnet preferentially of strength larger than 10⁻⁶, 10⁻³, 10⁻¹ or 1 T.

In another embodiment of the invention, the strength of the bond or interactions between the cryoprotectant or protectant compound and the coating and/or core of the nanoparticle or at least one constituent of the composition is weak when the cryoprotectant or protectant compound can't be attracted or moved preferentially together by a magnet preferentially of strength stronger than 10⁻⁶, 10 ³, 10⁻¹ or 1 T while the core and coating of the nanoparticle or at least one constituent of the composition can be attracted or moved preferentially together by a magnet preferentially of strength stronger than 10⁻⁶, 10⁻³, 10⁻¹ or 1 T.

In another embodiment of the invention, the strength of the bonds or interactions between the coating and core of the nanoparticle or at least one constituent of the composition is stronger than the strength of the bond or interaction between the cryoprotectant or protectant compound and the coating and/or core of the nanoparticle or at least one constituent of the composition, when the cryoprotectant or protectant compound can be less or less strongly attracted or moved by a magnet preferentially of strength stronger than 10⁻⁶, 10⁻³, 10⁻¹ or 1 T than the nanoparticle or at least one constituent of the composition.

The invention also relates to the composition or at least one constituent of the composition according to the invention, further comprising a compound, preferentially designated as the other compound, which has at least one property selected in the group consisting of:
A) It is a chemical element, preferentially as listed in the periodic table of Mendeleev,
B) It is selected in the group consisting of: i) Manganese, ii) Magnesium, iii), Potassium, iv) Calcium, v) Zinc, and vi) Sodium,
C) It originates from or is comprised in at least one chemical compound used to fabricate the at least one nanoparticle or at least one constituent of the composition,
D) It originates from or is comprised in at least one medium used to amplify or grow the living organism, preferentially a magnetotactic bacterium, which synthetizes the at least one nanoparticle or at least one constituent of the composition,
E) It is in ionic or charged form, preferentially in such a form that the interaction, preferentially electrostatic one, between the compound and the core or coating of the nanoparticle or at least one constituent of the compositions is favored,
F) It can be removed from the composition or at least one constituent of the composition, for example by using a chelating agent or a solution containing a chelating agent that preferentially binds to such compound,
G) It has a concentration or percentage in mass in the composition or at least one constituent of the composition that is lower than the concentration or percentage in mass of at least one of the following substances comprised in the composition or at least one constituent of the composition: i) the nanoparticle or at least one constituent of the composition, ii) the nanoparticle or at least one constituent of the composition core, iii) the nanoparticle or at least one constituent of the composition coating, and iv) the cryoprotectant or protectant compound,
I) It is comprised either outside of the nanoparticle or at least one constituent of the composition core or at the surface of the nanoparticle or at least one constituent of the composition core,
J) It is not in a crystalline form or does not predominantly contribute to the crystalline form or type of the nanoparticle or at least one constituent of the composition.

The invention also relates to the composition or at least one constituent of the composition according to the invention, wherein the composition or at least one constituent of the composition or at least one constituent of the composition or at least one constituent of the composition is in the form of: i) a powder, ii) a liquid, iii) or a liquid suspension, iv) a solid, and/or v) one or a mixture of liquid, solid and/or gaseous state(s).

The invention also relates to the composition or at least one constituent of the composition according to the invention, wherein the at least one chain of at least two nanoparticle or at least one constituent of the compositions has at least one property selected in the group consisting of:
A) the at least one chain exists during at least one step of fabrication or use of the at least two nanoparticle or at least one constituent of the composition, preferentially selected among: i) amplification of a nanoparticle or at least one constituent of the composition-producing cell, ii) purification or isolation of nanoparticle or at least one constituent of the compositions preferentially from some material or chemical, organic or not, preferentially originating from the at least one nanoparticle or at least one constituent of the composition-cell, iii) coating or formulation or mixture of the nanoparticle or at least one constituent of the composition preferentially with or between at least one or two constituent(s) of the composition or at least one constituent of the composition, and iv) administration or presence of the at least one chain in or with a body part or cell or matrix or medium or water or gel or material or polymer,
B) the at least one chain comprises at least two nanoparticle or at least one constituent of the compositions, wherein the at least one first direction such as a crystallographic first direction or a first direction perpendicular to a nanoparticle or at least one constituent of the composition facet or edge or surface or crystallographic plane or a first direction parallel to a nanoparticle or at least one constituent of the composition diameter of a first nanoparticle or at least one constituent of the composition is aligned with at least one second direction such as a crystallographic second direction or a second direction perpendicular to a nanoparticle or at least one constituent of the composition facet or edge or surface or crystallographic plane or a second direction parallel to a nanoparticle or at least one constituent of the composition diameter of a second nanoparticle or at least one constituent of the composition, wherein the alignment of the first and second directions is preferentially characterized by an angle between the first and second directions that is smaller than 180, 90, 45, 30, 20, 10, 5, 2, 1, 0.1 or 0 °, wherein such angle is preferentially measured or preferentially exists in at least one moment in time and/or one location in space preferentially during the life time of the chain,
C) the at least one chain comprises at least two nanoparticle or at least one constituent of the compositions, which are separated by a distance larger than 10⁻³, 0, 1, 5, 10, 10², 10³, 10⁵ or 10⁹ nm, preferentially when the at least two nanoparticle or at least one constituent of the composition are disassembled or are not linked with each other by some binding material or interaction forces preferentially belonging to the composition or at least one constituent of the composition but can preferentially be reassembled preferentially by adding to or mixing with the at least two nanoparticle or at least one constituent of the compositions some binding material that preferentially re-assemble the at least two nanoparticle or at least one constituent of the compositions,
D) the at least one chain comprises at least two nanoparticle or at least one constituent of the compositions, which are separated by a distance smaller than 10⁹, 10⁶, 10³, 100, 50, 10, 5, 2, 1 or 0 nm, preferentially when the at least two nanoparticle or at least one constituent of the compositions are assembled or are linked with each other by some binding material or interaction forces preferentially belonging to the composition or at least one constituent of the composition,
   and
E) the at least one chain is in the form of: i) a powder, ii) a liquid, iii) a liquid suspension, iv) a solid, and/or v) one or a mixture of liquid, solid and/or gaseous state(s).

The invention also relates to the composition or at least one constituent of the composition according to the invention, wherein the nanoparticle or at least one constituent of the composition comprises at least one center of activity, preferentially of medical activity, preferentially selected in the group consisting of:
A) a thermal center that increases or decreases the heat or the cold, preferentially of the body part,
B) a medical center that increases or enhances the effect of a medical compound such as an immunotherapy, chemotherapy, hormonotherapy, radiotherapy, or surgical medical compound,
   and
C) a center of activity or free radical production or capture, which is preferentially characterized by at least one property selected in the group consisting of:
   A) the nanoparticle core or at least one constituent of the composition comprises a first center of activity or free radical production or capture C_{1FRPC}, wherein C_{1FRPC} is preferentially selected in the group consisting of:
      i) another metal than iron such as Zinc or Aluminum,
         and
      ii) another metal oxide than iron oxide such as Zinc Oxide or Aluminum Oxide.
         and
   B) the nanoparticle coating or at least one constituent of the composition comprises a second center activity or of free radical production or capture C_{2FRPC}.

The invention also relates to the composition or at least one constituent of the composition according to the invention, wherein the nanoparticle or at least one constituent of the composition, preferentially the nanoparticle or at least one constituent of the composition core, is synthesized by a living organism or nanoparticle or at least one constituent of the composition producing cell, preferentially a magnetotactic bacterium.

The invention also relates to the composition or at least one constituent of the composition according to the invention, wherein the center of activity is selected in the group consisting of:
A) a radio-sensitizer or amplificator of radiation, a radio-photosensitizer or amplificator of light radiation, an acoustic sensitizer or amplificator of acoustic radiation or wave, a sonosensitizer or amplificatory of acoustic wave, a particle radiation sensitizer or amplificator of particle radiation, where the particle comprises (or not) a mass, it is a thermal-sensitizer or amplificator of heat or cold or thermal treatment, an amplificator of the medical effect of a compound,
B) an attenuator of radiation, of light radiation, of acoustic radiation or wave, of particle radiation, where the particle comprises (or not) a mass, of heat or cold, of thermal treatment, and/or of the medical effect of a compound,
   and
C) a compound, preferentially of meter or centimeter or millimeter or micrometer or nanometer or sub-nanometer or atomic size, selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or at least one constituent of the composition or nanomaterial, 56) Natural products or compounds, 57) Non-Steroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66) Silicon phthalocyanine (PC4), 67) RLP068, 68) Porfimer sodium, 69) Porfins, 69) Porphyrins, such as 5,10,15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene, 91) ABS-FA, 92) Acrylonitrile Butadiene Styrene, 93) Styrene, 94) Folic acid, 95) AIMP NP, aminoacyl tRNA synthetase complex-interacting multifunctional protein, 96) Au Nanomaterial, 97) gold, 98) Au-MnO nanomaterial, 99) manganese oxide, 100) Antineoplastic drugs, 101) NSAIDs, 102) nonsteroidal anti-inflammatory drug, 103) Artemether, 104) 5-ALA (5-aminolevulinic acid), 105) Acridine, Acridine Orange, 106) Au-doped TiO2, 107) Carbon based nanomaterial, 108) carbon nanotube, 109) Chlorine, 110) Ce6, 111) PTX, Paclitaxel, 112) chemotherapeutic drug or compound, 113) infrared dye or IR783, 114) Curcumin, 115) Cyanine or Cu-Cyanine, 116) DHMS, 117) dimethylsulfure, 118) Docetaxel, 119) chemotherapeutic drug or compound, 119) DOX/Mn-TPPS@RBCS, 120) doxorubicin, 121) manganese, 122) blood cell, 123) red blood cell, cell, 124) polymer, 125) elastomer, 126) Erythosin or Erythosin B, 127) FA or FA-OI or FA-OI NP or folic acid, 128) F3-PLGA@MB/Gd NPs, 129) poly(lactic-co-glycolic acid), 130) gadolinium, 131) Fe-TiO2 or titanium oxide, 132) Fe-VS₂, 133) iron, 134) vanadium disulfide, 135) FMSNs-DOX, 136) silica, 137) HCQ, 138) hydrochloroquine, 139) HP, 140) hematoporphyrin, 141) HMME, 142) hematoporphyrin monomethyl ether, 143) HSYA or Hydroxysafflor yellow A, 144) Hypocrellin, Hypocrellin B, 145) IR780, 146) Levofloxacin, 147) LIP3 or Lithium phosphide, 148) Lithium, 149) Liposome or Liposomal nanomaterial, 150) Lomefoxacin, 151) MG@P NPs, 152) MnP or Manganese peroxidase, 153) MnTTP-HSAs, 154) HSA-wrapped metal-porphyrin complex, 155) albumin, 156) MnWOx, 157) MnWOx-PEG, 158), PEG, 159) metallic or bimetallic or multi-metallic compound preferentially oxide, 160) Mn (III)-HFs, 161) managense, 162) hemoporfin, 163) nano-compound or nanoroad or nanoflower or nanowire or quantum dot, 164) Noble or halogen or Hydrogen or alkali metals or Alkaline earth metals or Triels or Tetrels or Pnicto-gen or Chal-co-gens or metal or gas or liquid or solid preferentially nanomaterial, 165) oxygen indyocyanine preferentially nanoparticle or at least one constituent of the composition, 166) Phthalocyanines, 167) PIO or Pioglitazone, 168) Polymeric nanomaterial, 169) Porphyrin, 170) Pt-doped TiO₂, 171) R837, 172) Rose Bengal, 173) Sparfloxacin, 174) TAPP or 5,10,15,20-tetrakis (4-aminophenyl) porphyrin, 175) TiO₂ or titanium dioxide nanomaterial, 176) TCPP, isomer, or Tris(1-chloro-2-propyl) phosphate 177) TPI or Thermoplastic Polyimide or thermoplastic polymer, 178) TPZ or Tirapazamine, 179) Transition metal oxide, 180) nanoparticle or at least one constituent of the composition or Janus nanoparticle or at least one constituent of the composition, and 181) Xanthones, 182) AQ4N, 183) Apaziquone (E09), 184) Bromodeoxyuridine, 185) Carbogen, 186) Cetuximab, 187) Chemotherapeutic drug or compound, 188) Chlorpromazine, 189) C-reactive peptide, 190) Curcumin, 191) Diamide, 192) Diethylmaeate, 193) Dihydroartemisinin, 194) Docetaxel, 195) ECI301, 196) Etanidazole, 197) Fludarabine, 198) 5-Fluorouracil, 199) Fluorodeoxyuridine, 200) Gadolynium, 201) Gemcitabine, 202) HER-3 ADC, 203) HSP, 204) Hydrogen peroxide, 205) Hydroxyurea, 206) Hyperbaric oxygen, 207) Hyperthermia, 208) Hypoxic cell cytotoxic agent, 209) Irinotecan, 210) lanthanide-doped radiosensitizer-based metal-phenolic network, 211) Lidocaine, 212) Lododeoxyuridine, 213) Metronidazole, 214) misonidazole, 215) etanidazole, 216) nimorazole, 217) N-Ethylmalemide, 218) malmeide, 219) ethylmalmeide, 220) Nanomaterial such as those consisting of or composed of at least partly or fully gold, silver, bismuth, gadolinium, polysiloxane matrix and gadolinium chelates, hafnium, Tantalum, Zinc, Gadolinium,
Germanium, Chromium, Praseodymium, Silicon, iron, platinum, cobalt, manganese, magnesium, iron, Titanium, carbon nanotube, quantum dot, nanoroad, Triflate, or metal oxide, 221) Nelfinavir, 222) Nicotinamide, 223) Nimotuzumab, 224) RNA, or miRNA, or miR-201, or miR-205, or miR-144-5p, or miR-146a-5p, or miR-150, or miR-99a, or miR-139-5p, or miR-320a, 225) Membrane active agent, 226) Mitomycin-C or Mitomycin, 227) Motexafin, 228) NBTXR3, 229) Oligonucleotide, 230) Paclitaxel, 231) Papaverine or Papaverine hydrochloride, 232) Paraxonase-2, 233) Pocaine, 234) Porfiromycin (POR), 235) Protein, 236) Peptide, 237) Radiosensitizing nucleosides or compounds, 238) Resveratrol, 239) RRx-001, 240) SiRNa, 241) Suppressors of sulfhydral groups, 242) SYM004, 243) Texaphyrins, 244) TH-302, and 245) Tirapazamine.

The invention also relates to the composition or at least one constituent of the composition according to the invention combined with at least one nanoparticle or at least one constituent of the composition-producing cell, preferentially a magnetotactic bacterium,
wherein the composition or at least one constituent of the composition preferentially comprises a first cryoprotectant or protectant compound,
wherein the nanoparticle or at least one constituent of the composition-producing cell preferentially comprises a second cryoprotectant or protectant compound,
wherein the first and second cryoprotectant or protectant compounds are preferentially different compounds,
wherein preferentially the composition or at least one constituent of the composition and nanoparticle or at least one constituent of the composition producing cells are used or fabricated or amplified separately or one after the other, i.e. the composition or at least one constituent of the composition is preferentially used or fabricated after the nanoparticle or at least one constituent of the composition-producing cells has been used to fabricate the nanoparticle or at least one constituent of the composition comprised in the composition or at least one constituent of the composition.

In some cases, the first and second cryoprotectant or protectant compounds can be the same or different compounds.

In some other cases, the combination of the composition or at least one constituent of the composition and nanoparticle or at least one constituent of the composition-producing cell is or belongs to a system or combined system or combined product.

The invention also relates to the composition or at least one constituent of the composition according to the invention, wherein the composition or at least one constituent of the composition preferentially comprises an inert part and/or an active part, wherein the inner part preferentially does not comprise at least one center of activity or the active part preferentially comprises at least one center of activity or the active part preferentially comprises more or a larger number of centers of activity than the inner part,
wherein the inner part preferentially ensures the cohesion of the composition or at least one constituent of the composition or preferentially comprises links or bonds or forces or atoms or ions or nanoparticle or at least one constituent of the compositions that maintain the at least one or two constituent(s) of the composition or at least one constituent of the composition assembled together within one volume,
wherein the center of activity is preferentially comprised in the nanoparticle or at least one constituent of the composition or at least one constituent of the composition or at least one constituent of the composition, wherein the center of activity preferentially increases or decreases or amplifies or attenuates the production of heat or cold by the nanoparticle or at least one constituent of the composition or at least one constituent of the composition or at least one constituent of the composition, the medical activity of the nanoparticle or at least one constituent of the composition or at least one constituent of the composition or at least one constituent of the composition, and/or the radiation or strength or power or wavelength or intensity or frequency of the radiation applied on the nanoparticle or at least one constituent of the composition or at least one constituent of the composition or at least one constituent of the composition,
where the center of activity is preferentially selected in the group consisting of:
   A) a thermal center that preferentially increases the thermal activity of the nanoparticle or at least one constituent of the composition or at least one constituent of the composition or at least one constituent of the composition, preferentially heat or the cold, preferentially by at least 0.1 °C, preferentially locally around the nanoparticle or at least one constituent of the composition or at least one constituent of the composition or at least one constituent of the composition or at a distance from the nanoparticle or at least one constituent of the composition or at least one constituent of the composition or at least one constituent of the composition of preferentially less than 1 µm,
   B) a thermal center that preferentially decreases the thermal activity of the nanoparticle or at least one constituent of the composition or at least one constituent of the composition or at least one constituent of the composition, preferentially heat or the cold, preferentially by at least 0.1 °C, preferentially locally around the nanoparticle or at least one constituent of the composition or at least one constituent of the composition or at least one constituent of the composition or at a distance from the nanoparticle or at least one constituent of the composition or at least one constituent of the composition or at least one constituent of the composition preferentially of less than 1 µm,
   C) a medical center that preferentially increases or enhances the effect or activity or strength of a medical compound, preferentially selected in the group consisting of: an enhancer of an immunotherapy, chemotherapy, hormonotherapy, radiotherapy or radio-enhancer, contrast agent, sonosensitizer, and surgical medical compound,
   D) a medical center that preferentially decreases the effect or activity or strength of a medical compound, preferentially selected in the group consisting of: an attenuator of an immunotherapy, chemotherapy, hormonotherapy, radiotherapy or radio-enhancer, contrast agent, sonosensitizer, and surgical medical compound,
   E) a center of radiation amplification that preferentially increases the strength of a radiation applied on the nanoparticle or at least one constituent of the composition or at least one constituent of the composition or at least one constituent of the composition, preferentially by at least 10⁻⁵ or 0.1 Gray or Watt or Candela, preferentially locally around the nanoparticle or at least one constituent of the composition or at least one constituent of the composition or at least one constituent of the composition or at a distance from the nanoparticle or at least one constituent of the composition or at least one constituent of the composition or at least one constituent of the composition preferentially of less than 1 µm,
   F) a center of radiation attenuation that preferentially decreases the strength of a radiation applied on the nanoparticle or at least one constituent of the composition or at least one constituent of the composition or at least one constituent of the composition, preferentially by at least 10⁻⁵ or 0.1 Gray or Watt or Candela, preferentially locally around the nanoparticle or at least one constituent of the composition or at least one constituent of the composition or at least one constituent of the composition or at a distance from the nanoparticle or at least one constituent of the composition or at least one constituent of the composition or at least one constituent of the composition of preferentially less than 1 µm,
      and
   G) a center of activity or free radical production or capture,
      wherein the at least one center of activity is preferentially characterized by at least one property selected in the group consisting of:
         I) the nanoparticle or at least one constituent of the composition core or at least a first constituent of the composition or at least one constituent of the composition preferentially comprises a first center of activity, C_{A1}, preferentially being a first center of activity or free radical production or capture C_{1FRPC}, wherein C_{A1} or C_{1FRPC} is preferentially selected in the group consisting of:
            xi) another metal than iron such as Zinc or Aluminum,
            xii) another metal oxide than iron oxide such as Zinc Oxide or Aluminum Oxide,
            xiii) a compound that is essentially or in majority or at least partly inorganic or metallic, and
         II) the nanoparticle or at least one constituent of the composition coating or second constituent of the composition or at least one constituent of the composition preferentially comprises a second center of activity, C_{A2}, preferentially being a second center of activity or free radical production or capture C_{2FRPC}, preferentially being a compound that is essentially or in majority or at least partly organic or non-metallic,
         III) the cryoprotectant or protectant compound or third constituent of the composition or at least one constituent of the composition preferentially comprises a third center of activity, C_{A3}, preferentially being a center of activity that preferentially protects or maintains or prevents the diminution or increases the activity of C_{A1} and/or C_{A2},
      wherein C_{A1}, C_{A2}, and/or C_{A3} has(have) at least one property selected in the group consisting of:
         i) It(they) is(are) preferentially over time or under storage or under use of the composition or at least one constituent of the composition or under administration of the composition or at least one constituent of the composition to the body part.
         ii) C_{A1}, C_{A2}, and/or C_{A3} is(are) preferentially different compounds,
         iii) C_{A1}, C_{A2}, and/or C_{A3} activity can preferentially be measured by comparing the activity of a body part or medium comprising the composition or at least one constituent of the composition with that of a body part or medium not comprising the composition or at least one constituent of the composition, where the body part or medium comprising and not comprising the composition or at least one constituent of the composition are exposed to similar or the same radiation or thermal variation,
         iv) C_{A1}, C_{A2}, and/or C_{A3} is(are) preferentially separated by a distance of at least 0.1, 1, 5, 10, 100 or 10³ nm,
         v) C_{A1}, C_{A2}, and/or C_{A3} is(are) preferentially different from at least one whole constituent of the composition or at least one constituent of the composition, i.e. preferentially the at least one whole constituent preferentially comprises at least one other substance that is different from a center of activity,
         vi) C_{A1}, C_{A2}, and/or C_{A3} occupies(y) preferentially less than 100 or 99 or 50% of the volume or location of the at least one constituent,
            and
         vii) C_{A1}, C_{A2}, and/or C_{A3} preferentially has(have) a percentage in mass of less than 100 or 99 or 50% relatively to the mass of the at least one constituent,
         viii) C_{A1}, C_{A2}, and/or C_{A3} preferentially release or diffuse, preferentially in an outward direction relatively to at least one constituent of the composition or at least one constituent of the composition, or expel or activate at least one atom, electron, free radical, ion, metal, DNA, RNA, protein, lipid, enzyme, biological or non-biological material, organic or non-organic material, an immune entity from or of or in or at the surface or outside the nanoparticle or at least one constituent of the composition or at least one constituent of the composition or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,
         ix) C_{A1}, C_{A2}, and/or C_{A3} preferentially capture or diffuse, preferentially in an inward direction relatively to at least one constituent of the composition or at least one constituent of the composition, at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition or at least one constituent of the composition or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,
      wherein the at least one constituent of the composition is preferentially selected in the group consisting of: i) the nanoparticle or at least one constituent of the composition coating, ii) the nanoparticle or at least one constituent of the composition core, iii) the cryoprotectant or protectant compound, and iv) the other compound,
      wherein the immune entity, preferentially the first and/or second immune entity(ies), is preferentially selected in the group consisting of: i) DNA preferentially different types of DNA, ii) RNA preferentially different types of RNA, iii) an antigen, ii) an antibody, iii) an immune cell, preferentially of or belonging to the innate and/or adaptative immune system(s), iv) an antigen presenting cell (APC), v) a basophil, vi) a dendritic cell, vii) an eosinophil, viii) a granulocyte, ix) a killing cell, x) a natural killer, xi) a leukocyte, xii) a lymphocyte, xiii) a macrophage, preferentially of M1 and/or M2 type(s), xiii) a mast cell, xiv) a neutrophil, xv) a phagocyte, xvi) a B cell, xvii) a T cell, xviii) a CD8 or CD8⁺ or CD4 or CD4⁺ or Treg or MAIT or Ty6 T lymphocyte or cell, xix) a helper cell preferentially of Th1 or Th2 type, and xx) a gamma delta T cell.

In one embodiment, at least one binding material binds at least two nanoparticle or at least one constituent of the compositions preferentially in chain or geometric figure or assembly.

In some cases, the binding materials can be the same material as the coating and/or have at least one property in common with the coating or at least one constituent of the composition.

In some cases, the binding material can be a different material from the coating, and/or comprise water or a gel or tissue or cellular or cellular component such as cytoplasm and/or have at least one property different from the coating or at least one constituent of the composition.

In one embodiment of the invention, the bonds between: i) the nanoparticle or at least one constituent of the composition different from the cryo-protectant or protectant compound, nanoparticle core, and/or nanoparticle coating and ii) the cryoprotectant or protectant compound are weak bonds, preferentially Van der Walls bonds, preferentially non-covalent or non-metallic bonds.

In one embodiment of the invention, the bonds between: i) the nanoparticle or at least one constituent of the composition, nanoparticle core, and/or nanoparticle coating and ii) the cryoprotectant or protectant compound or protectant compounds are weaker or weaker in strength than the bonds between the coating and the core of the nanoparticle.

In one embodiment of the invention, there exists partly or fully a complexation between the core and the coating of the nanoparticle or at least one constituent of the composition, preferentially within part or the whole lifespan of the composition or at least one constituent of the composition, preferentially during lyophilization or storage of the composition or at least one constituent of the composition, preferentially when the composition or at least one constituent of the composition is in liquid, gas, or solid form, or in one of these sates in a dominant manner.

In another embodiment of the invention, there does not exist partly or fully a complexation between the coating or core of the nanoparticle or at least one constituent of the composition and the cryoprotectant or protectant compound or protectant compound, preferentially within part or the whole lifespan of the composition or at least one constituent of the composition, preferentially during lyophilization or storage of the composition or at least one constituent of the composition, preferentially when the composition or at least one constituent of the composition is in liquid, gas, or solid form, or in one of these sates in a dominant manner.

In one embodiment of the invention, the cryoscopic constant of the composition or at least one constituent of the composition or the Ebullioscopic constant of the composition or at least one constituent of the composition or the depression of freezing point of the solvent or of the composition or at least one constituent of the composition is smaller than 10⁵⁰, 10⁵, 10³, 100, 50, 20, 10, 5, 2, 1, 0 kg × K / mol.

In one embodiment of the invention, the cryoscopic constant of the composition or at least one constituent of the composition, or the Ebullioscopic constant of the composition or at least one constituent of the composition or the depression of freezing point of the solvent or of the composition or at least one constituent of the composition is larger than 10⁻¹⁰, 10⁻⁵, 10⁻³, 10⁻¹, 0, 1, 5, 10, 50 or 100 kg × K / mol.

The invention also relates to a method for storage or preservation of at least one property of at least one nanoparticle or at least one constituent of the composition such as the chain arrangement, preferentially by following at least one of the following step(s):
1. Mixing at least one nanoparticle or at least one constituent of the composition with a cryoprotectant or protectant compound or protectant compound;
2. Lyophilizing or desiccating or dehydrating or applying a temperature or pressure gradient or temperature decrease or oxidation or reduction or inducing a change of state to the composition or at least one constituent of the composition preferentially issued from step 1;
3. Storing or keeping the composition or at least one constituent of the composition preferentially issued from step 2, preferentially for a lapse of time of more than 1 second, 1 day, 1 month or 1 year;
4. (re)suspending or (re)dispersing the composition or at least one constituent of the composition preferentially issued from step 3, preferentially in a liquid such as water or in a solid or in a gas sate, preferentially in such a way that the nanoparticle or at least one constituent of the composition has maintained at least one of its property such as its chain arrangement, preferentially in such a way that the at least one nanoparticle or at least one constituent of the composition can be injected to a body part;
wherein the change of state of the state of the composition or at least one constituent of the composition is preferentially selected in the group consisting of: i) liquid to solid, ii) liquid to gas, iii) solid to liquid, iv) solid to gas, v) gas to liquid, and vi) gas to solid.

In one embodiment of the invention, the cryoprotectant or protectant compound occupies a larger volume than the coating or core or nanoparticle or at least one constituent of the composition different from cryoprotectant or protectant compound, preferentially when the composition or at least one constituent of the composition is in a liquid state or is mixed in a liquid.

In one embodiment of the invention, the distance between center of the nanoparticle or at least one constituent of the composition and the external surface of the nanoparticle or at least one constituent of the composition or coating or the thickness of the coating preferentially without the cryoprotectant or protectant compound is smaller than the thickness or diameter of the volume comprising the cryoprotectant or protectant compound.

In one embodiment of the invention, the volume occupied by the coating or nanoparticle or at least one constituent of the composition preferentially without the cryoprotectant or protectant compound is smaller than the volume occupied by the cryoprotectant or protectant compound.

In one embodiment of the invention, the coating material has at least one different property from the cryoprotectant or protectant compound, preferentially selected among the group consisting of: i) the coating material is not located in the same region or location than the cryoprotectant or protectant compound in the composition, ii) the coating material interacts or complexes more strongly with the nanoparticle core or at least one constituent of the composition different from the cryo-protectant or protectant compound than the cryoprotectant or protectant compound in the composition, and iii) the coating material has a different composition or structure or magnetic property or crystallinity or is different than/from the cryoprotectant or protectant compound or has at least 1, 5, 10, 10³ or 10⁵ atom(s) that differ from that(those) of the cryoprotectant or protectant compound.

In one embodiment, the function of the cryoprotectant or protectant compound in the composition or at least one constituent of the composition is to replace water molecule or maintain the size, composition or at least one constituent of the composition, chain arrangement, or magnetic property of the at least one nanoparticle or at least one constituent of the composition, preferentially during cooling or application of a temperature/pressure gradient to the composition or at least one constituent of the composition, preferentially to avoid damages caused by ice on the coating or nanoparticle or at least one constituent of the composition.

In one embodiment, the composition or at least one constituent of the composition is or is maintained at a temperature larger than -273, -50, -1, 0, 2, 5, 10, 100, 10³ or 10⁵ °C, preferentially for more than 10⁻¹⁰, 0, 1,5, 10, 10³ or 10¹⁰ second(s).

In another embodiment, the composition or at least one constituent of the composition is or is maintained at a temperature smaller than 10⁵, 10³, 100, 10, 5, 2, 1, 0, -10, -50, -100, -250 °C, preferentially for more than 10⁻¹⁰, 0, 1, 5, 10, 10³ or 10¹⁰ second(s).

In one embodiment, the composition or at least one constituent of the composition is or is maintained under a pressure larger than 10⁻⁵⁰, 10⁻¹⁰, 10⁻⁵, 10⁻¹, 0, 1, 5, 10, 10³, 10⁵, 10¹⁰ or 10²⁰ bar, preferentially for more than 10⁻¹⁰, 0, 1, 5, 10, 10³ or 10¹⁰ second(s).

In another embodiment, the composition or at least one constituent of the composition is or is maintained under a pressure smaller than 10⁵⁰, 10⁵⁰, 10, 0, 10⁻³, 10⁻⁵, 10⁻¹⁰ or 10⁻²⁰ bar, preferentially for more than 10⁻¹⁰, 0, 1, 5, 10, 10³ or 10¹⁰ second(s).

In one embodiment of the invention, the presence of the cryoprotectant or protectant compound in the composition or at least one constituent of the composition enables to store the composition or at least one constituent of the composition, preferentially in a powder form, preferentially for more than 10⁻¹⁰, 0, 1, 5, 10, 10³ or 10¹⁰ second(s), preferentially in such a way that the composition or at least one constituent of the composition maintains at least one of its properties.

In one embodiment of the invention, the presence of a nanoparticle core or at least one constituent of the composition, preferentially a metallic one, enhances the effect of the cryoprotectant or protectant compound in the composition or at least one constituent of the composition, preferentially by facilitating the interaction between the coating and the cryoprotectant or protectant compound, preferentially due to a stabilization of the coating on the core.

In one embodiment of the invention, the composition of the at least one constituent of the composition is a mixture of amorphous and crystalline structure, wherein the nanoparticle core or at least one constituent of the composition is preferentially essentially or dominantly crystalline, wherein the nanoparticle coating or at least one other constituent of the composition, cryoprotectant and/or protectant compound is/are preferentially essentially or dominantly amorphous.

In one embodiment of the invention, the cryoprotectant and/or protectant compound protects and/or preserves and/or maintains at least one property of at least one constituent of the composition.

In one embodiment of the invention, at least one constituent of the composition, preferentially amorphous, needs the presence of the cryoprotectant and/or protectant compound to have at least one of its properties protected and/or preserved and/or maintained preferentially following exposure of the composition or at least one constituent of the composition to a temperature and/or pressure gradient(s) and/or radiation, i.e. preferentially without the cryoprotectant and/or protectant compound the at least one property of the at least one constituent of the composition would preferentially be damaged or changed.

In one embodiment of the invention, the at least one constituent of the composition needs the presence of the cryoprotectant and/or protectant compound to have at least one of its properties protected and/or preserved and/or maintained preferentially following exposure of the composition or at least one constituent of the composition to a temperature and/or pressure gradient(s) and/or radiation, i.e. preferentially without the cryoprotectant and/or protectant compound the at least one property of the at least one constituent of the composition would preferentially be damaged or changed.

The invention also relates to a method for the cryo-preservation, size-preservation, composition-preservation, cohesion-preservation, magnetic property-preservation, or preservation of at least one property of the composition or at least one constituent of the composition or nanoparticle by following at least one of the following steps:
1. Preferentially mixing at least one nanoparticle or at least one constituent of the composition with a cryoprotectant or protectant compound;
2. Preferentially lyophilizing or desiccating or dehydrating or applying a temperature or pressure gradient or temperature decrease or oxidation or reduction or radiation inducing a change of state to the composition or at least one constituent of the composition preferentially issued from step 1;
3. Storing or keeping the composition or at least one constituent of the composition preferentially issued from step 2, preferentially for a lapse of time of more than 1 second, 1 day, 1 month or 1 year;
4. (re)suspending or (re)dispersing the composition or at least one constituent of the composition preferentially issued from step 3, preferentially in a liquid such as water or in a solid or in a gas sate, preferentially by or under sonication or radiation application, preferentially under sterile conditions, or preferentially prior to sterilization of the composition or at least one constituent of the composition, preferentially in such a way that the nanoparticle or at least one constituent of the composition has maintained at least one of its property such as its chain arrangement, preferentially in such a way that the at least one nanoparticle or at least one constituent of the composition can be injected to a body part;

In some cases, the preservation of at least one property of the composition or at least one constituent of the composition or nanoparticle can be the variation by less than 10⁵, 10³, 102, 90, 75, 60, 50, 25, 10, 5, 2, 1 or 0% of this property, where this variation is preferentially equal to (P2-P1)/P1, where P1 and P2 are preferentially this property before and after the composition or at least one constituent of the composition or nanoparticle have been subjected to a temperature and/or pressure gradient or cooled down or exposed to radiation or oxidized or reduced.

The invention also relates to the composition or at least one constituent of the composition or kit or ensemble or system or assembly comprising:
i) A first component or constituent consisting of at least one living organism or cell or bacterium or nanoparticle-producing cell or magnetotactic bacterium and a first cryoprotectant or protectant compound, preferentially used to preserve the activity of the living organism such as its ability to divide or produce nanoparticle preferentially under storage;
   and/or
ii) A second component or constituent consisting of at least one nanoparticle or at least one constituent of the composition and a second cryoprotectant or protectant compound, preferentially used to preserve at least one property of at least one nanoparticle or at least one constituent of the composition, preferentially under storage;
wherein the first and second cryoprotectant or protectant compound are preferentially different or have at least one different function.

In some cases, the first and second components or constituents are sold or used separately or at different times or under different conditions.

In some cases, the first component or constituent is necessary or used or serves as starting material for the production or fabrication of the second component.

In some other cases, the first and second components or constituents are sold or used together or at similar times or under similar conditions.

The invention also relates to a method for the cryo-preservation, size-preservation, composition-preservation, cohesion-preservation, magnetic property-preservation, or preservation of at least one property of the composition or at least one constituent of the composition or nanoparticle by following at least one of the following steps:
1. Storing or amplifying a living organism, preferentially a nanoparticle producing cell or magnetotactic bacterium in the presence of a first cryoprotectant or protectant compound;
2. Mixing at least one nanoparticle or at least one constituent of the composition with a second cryoprotectant or protectant compound;
3. Lyophilizing or desiccating or dehydrating or applying a temperature or pressure gradient or temperature decrease or oxidation or reduction or radiation or inducing a change of state to the composition or at least one constituent of the composition preferentially issued from step 2;
4. Storing or keeping the composition or at least one constituent of the composition preferentially issued from step 2, preferentially for a lapse of time of more than 1 second, 1 day, 1 month or 1 year;
5. (re)suspending or (re)dispersing the composition or at least one constituent of the composition preferentially issued from step 4, preferentially in a liquid such as water or in a solid or in a gas sate, preferentially by or under sonication or radiation application, preferentially under sterile conditions, or preferentially prior to sterilization of the composition or at least one constituent of the composition, preferentially in such a way that the nanoparticle or at least one constituent of the composition has maintained at least one of its property such as its chain arrangement, preferentially in such a way that the at least one nanoparticle or at least one constituent of the composition can be injected to a body part;

In some cases, the first cryoprotectant or protectant compound can be used to protect a living organism or can be DMSO or Ethylene glycol or Glycerol or 2-Methyl-2,4-pentanediol (MPD) or Propylene or glycol or Sucrose or Trehalose.

The invention also relates to a method for enabling nanoparticle or at least one constituent of the compositions stored preferentially in powder form with a specific type of assembly or geometric figure preferentially a chain assembly to maintain this type of assembly upon reconstitution preferentially in liquid such as water, where this method preferentially comprises at least one of the following steps of:
- First step of mixing the at least one nanoparticle or at least one constituent of the composition with at least one protectant compound,
- Second step of removing water or at least one compound that is different from the at least one nanoparticle and/or from the at least one protectant compound, preferentially by lyophilizing the composition preferentially originating from step 1,
- Third step of re-suspending the composition or at least one constituent of the composition preferentially originating from step 2, preferentially in a liquid, solid, or gas, most preferentially in water.

The invention also relates to a method of fabrication or cryo-preservation of the composition or at least one constituent of the composition according to the invention and optionally of the at least one cell producing the at least one nanoparticle, which comprises at least one of the following steps:
- **Step** 1 of storing or cryo-preserving the at least one cell producing the at least one nanoparticle preferentially in a medium comprising a first cryoprotectant or protectant compound,
- **Step 2** of amplifying magnetotactic bacteria or nanoparticle -producing cells preferentially in at least one medium, preferentially comprising:
   1) the compounds necessary for the growth of magnetotactic bacteria or nanoparticle or at least one nanoparticle-producing cells and/or the production of magnetosomes or nanoparticle or at least one constituent of the compositions, which are preferentially selected in the group consisting of:
      - a source of carbon preferentially selected from the group consisting of: at least one compound comprising at least one atom of carbon, lactic acid, Na lactate, lactic acid, acetate, glycolate, glucose, pyruvate, succinate, carbon dioxide, glycerol and combinations thereof, at a concentration preferentially comprised between 1 nM and 2 Mol/L;
      - a source of iron preferentially selected from the group consisting of: at least one compound comprising at least one atom of iron, iron citrate, iron quinate, iron chloride, iron sulfate, FeCl₃, and combinations thereof, at a concentration preferentially comprised between 1 nM and 2.10⁻³ Mol/L;
      - a source of nitrogen preferentially selected from the group consisting of: at least one compound comprising at least one atom of nitrogen, nitrate salt, nitrogen gas, ammonium, ammonia, ammonium salt, urea, an amino acid, ammonia gas, and combinations thereof, at a concentration preferentially comprised between 1 nM and 4 Mol/L;
      - a source of oxygen preferentially selected from the group consisting of: at least one compound comprising at least one atom of oxygen, oxygen or air or compressed air, preferentially in the form of a gas, the source of oxygen being in some cases bubbled or introduced to the growth medium, at a gas rate that is preferentially comprised between 5 mL of gas per minute and 50000 mL of gas per minute;
      - a source of phosphate preferentially consisting of at least one compound comprising at least one atom of phosphate, at a concentration preferentially comprised between 1 nM and 2.10⁻¹ Mol/L;
      - a source of potassium preferentially consisting of at least one compound comprising at least one atom of potassium, at a concentration preferentially comprised between 1 nM and 2.10⁻¹ Mol/L;
      - a source of sulfur or sulfate preferentially consisting of at least one compound comprising at least one atom of sulfur or sulfate, at a concentration preferentially comprised between 1 nM and 4.10⁻¹ Mol/L;
      - a source of manganese preferentially consisting of at least one compound comprising at least one atom of manganese, at a concentration preferentially comprised between 1 nM and 4.10⁻¹ Mol/L;
      - a source of vitamin preferentially selected from the group consisting of: at least one compound comprising at least one vitamin, Biotin, Calcium, pantothenate, Folic acid, Inositol, Nicotinic acid, p-Aminobenzoic acid, Pyridoxine HCl, Riboflavin, Thiamine, Thiamine HCL and derivatives thereof and combinations thereof, at a concentration preferentially comprised between 1 nM and 10⁴ Mol/L, and
      - a source of calcium preferentially consisting of at least one compound comprising at least one atom of calcium, at a concentration preferentially comprised between 1 nM and 10⁻¹ Mol/L.
   2) at least one compound preferentially necessary for doping the magnetosomes or nanoparticles with C_{1FRPC} or a center of activity or another metal than iron, preferentially zinc or aluminum, for example a source of zinc, preferentially zinc sulfate or zinc citrate or zinc chlorate or zinc quinate.
      - **Step 3** of extracting or isolating magnetosomes or nanoparticle or at least one constituent of the compositions from magnetotactic bacteria or nanoparticle-producing cells;
      - **Step 4** of purifying the extracted or isolated magnetosomes or nanoparticle or at least one constituent of the compositions preferentially by heating them preferentially to yield magnetosome minerals or nanoparticle or at least one constituent of the compositions comprising a percentage in mass of organic material preferentially originating from magnetotactic bacteria or from the at least one nanoparticle -producing cell that is lower than 100, 50, 20, 10, 5, 2 or 1%,
      - **Step 5** of coating the magnetosome minerals or nanoparticle or at least one constituent of the compositions preferentially with a coating material, preferentially comprising the compound C_{2FRPC} or a center of activity, preferentially by mixing the magnetosome minerals or nanoparticle or at least one constituent of the compositions with the coating material, where the mixing is preferentially realized in at least one of the following conditions:
         under sonication,
         under the application of radiation,
         under temperature variation,
         under pH changes,
         under oxidoreduction potential adjustment,
         preferentially using a ratio between the quantity or mass of magnetosome minerals and the quantity or mas of coating material, preferentially of compound D, that is adjusted or varied or larger than 1,
      - **Step 6** of adding at least one cryoprotectant or protectant compound, preferentially the second cryoprotectant or protectant compound, to the coated magnetosome minerals or nanoparticle preferentially obtained at the end of step 5,
      - **Step 7** of lyophilizing or dehydrating or drying or desiccating the composition or at least one constituent of the composition preferentially obtained at the end of step 6,
      - **Step 8** of re-suspending the lyophilized or dehydrated composition or at least one constituent of the composition preferentially obtained of step 7, preferentially in water,
   wherein the first and second cryoprotectant or protectant compounds when they are present are compounds that are either the same or different compounds.

The invention also relates to a method for storing the composition or at least one constituent of the composition or preserving over time at least one property of the nanoparticle or at least one constituent of the composition according to the invention, or preserving the geometric arrangement or assembly, preferentially chain arrangement of the at least one nanoparticle or at least one constituent of the composition according to the invention, which comprises at least one of the following steps:
- **Step 1:** Choosing or preparing the composition or at least one constituent of the composition in the form of a liquid suspension,
- **Step 2:** Lyophilizing, desiccating, dehydrating the composition or at least one constituent of the composition, or removing water or liquid or ion or atom, preferentially totally or partly or essentially different from iron, from the composition or at least one constituent of the composition,
- **Step3:** Storing the composition or at least one constituent of the composition preferentially essentially or totally or partly in a powder or solid form, preferentially during or for more than 1 second or 3 months,
   and
- **Step 4:** Suspending or resuspending the composition or at least one constituent of the composition, preferentially in water in water or liquid or gas or solid, preferentially under isotonic conditions.

The invention also relates to a method for the fabrication, the cryo-preservation, size-preservation, composition-preservation, cohesion-preservation, magnetic property-preservation, or preservation of the composition of at least one constituent of the composition according to the invention, by following at least one of the following steps:
- **Step 1:** Mixing at least one nanoparticle or at least one constituent of the composition with a cryoprotectant or protectant compound,
- **Step 2:** Lyophilizing or desiccating or dehydrating or applying a temperature or pressure gradient or temperature decrease or oxidation or reduction or radiation inducing a change of state to the composition or at least one constituent of the composition preferentially originating from step 1,
- **Step 3:** Storing or keeping the composition or at least one constituent of the composition or at least one constituent of the composition or at least one constituent of the composition preferentially originating from step 2, preferentially for a lapse of time of more than 1 second, 1 day, 1 month or 1 year,
- **Step 4:** (re)suspending or (re)dispersing the composition or at least one constituent of the composition preferentially originating from step 3, preferentially in a liquid such as water or in a solid or in a gas sate, preferentially by or under sonication or radiation application, preferentially under sterile conditions, or preferentially prior to sterilization of the composition or at least one constituent of the composition, preferentially in such a way that the nanoparticle or at least one constituent of the composition maintains at least one of its property such as its chain arrangement, preferentially in such a way that the at least one nanoparticle or at least one constituent of the composition can be injected to a body part or that the composition or at least one constituent of the composition is isotonic.

The invention also relates to a method for removing at least one compound from the composition or at least one constituent of the composition according to the invention, preferentially the other compound, by following at least one of the following steps:
- **Step 1:** Mixing the composition or at least one constituent of the composition with at least one chelating agent and/or introducing at least one chelating agent in the composition or at least one constituent of the composition,
- **Step 2:** Positioning a magnet near the composition or at least one constituent of the composition to attract the magnetic nanoparticle or at least one constituent of the composition in the region where the magnet is located,
- **Step 3:** Removing the part of the composition or at least one constituent of the composition that has not been attracted by the magnet or that is not magnetic,
- **Step 4:** Re-suspending the magnetic nanoparticle or at least one constituent of the composition or the part of the composition that is magnetic in a liquid, solid or gas, preferentially in a presence of a cryoprotectant or protectant compound,
- **Step 5:** Lyophilizing or desiccating or dehydrating or applying a temperature or pressure gradient or temperature decrease or oxidation or reduction or radiation inducing a change of state to the composition or at least one constituent of the composition, preferentially originating from step 4,
- **Step 6:** Storing or keeping the composition or at least one constituent of the composition preferentially originating from step 5, preferentially for a lapse of time of more than 1 second, 1 day, 1 month or 1 year,
- **Step 7:** (re)suspending or (re)dispersing the composition or at least one constituent of the composition preferentially originating from step 6, preferentially in a liquid such as water or in a solid or in a gas sate, preferentially by or under sonication or radiation application, preferentially under sterile conditions, or preferentially prior to sterilization of the composition or at least one constituent of the composition, preferentially in such a way that the nanoparticle or at least one constituent of the composition maintains at least one of its property such as its chain arrangement, preferentially in such a way that the at least one nanoparticle or at least one constituent of the composition can be injected to a body part or is isotonic,
   wherein the composition or at least one constituent of the composition preferentially comprises a magnetic part and/or a non-magnetic part;
   wherein the magnetic part of the composition is preferentially a part of the composition that can be attracted or isolated or moved or modified partly or fully by a magnet, preferentially of strength larger than the strength of the earth magnetic field or 0, 10⁻⁶, 10⁻³ or 10⁻¹ T,
   wherein the magnetic part of the composition is preferentially a part of the composition that can be attracted or isolated or moved or modified partly or fully by a magnet, preferentially of strength larger than the strength of the earth magnetic field or 10⁻⁶, 10⁻³ or 10⁻¹, T, more importantly than the non-magnetic part.

The invention also relates to a method for the fabrication, storage, preservation, preservation of the geometric arrangement or assembly, preferentially chain arrangement of the at least one nanoparticle or at least one constituent of the composition, cryo-preservation, size-preservation, composition-preservation, cohesion-preservation, magnetic property-preservation, or preservation of at least one property of the composition or at least one constituent of the composition according to the invention, which comprises at least one of the following steps:
- **Step 1:** Choosing or preparing the composition or at least one constituent of the composition, preferentially by mixing at least one nanoparticle or at least one constituent of the composition with a cryoprotectant or protectant compound,
- **Step 2:** Lyophilizing or desiccating or dehydrating or removing water or liquid or ion or atom, preferentially totally or partly or essentially different from iron, from the composition or at least one constituent of the composition, applying a temperature or pressure gradient or temperature decrease or oxidation or reduction or radiation inducing a change of state to the composition or at least one constituent of the composition originating from step 1,
- **Step 3:** Storing or keeping the composition or at least one constituent of the composition preferentially essentially or totally or partly in a powder or solid form, preferentially originating from step 2, preferentially for a lapse of time of more than 1 second, 1 day, 1 month or 1 year,
- **Step 4:** (re)suspending or (re)dispersing the composition or at least one constituent of the composition preferentially originating from step 3, preferentially in a liquid such as water or in a solid or in a gas sate, preferentially by or under sonication or radiation application, preferentially under sterile conditions, or preferentially prior to sterilization of the composition or at least one constituent of the composition preferentially in such a way that the nanoparticle or at least one constituent of the composition maintains at least one of its property such as its chain arrangement, preferentially in such a way that the at least one nanoparticle or at least one constituent of the composition can be injected to a body part or that the composition or at least one constituent of the composition is isotonic.

In one embodiment, the at least one method or composition or at least one constituent of the composition according to the invention is used for the treatment or detection of a disease or for the preparation or storage or activation or preservation of the composition or at least one constituent of the composition or for the administration of the composition or at least one constituent of the composition to a body part. In some cases, the composition can be can have at least one common property with the at least one constituent of the composition.

In some other cases, the at least one constituent of the composition is different from or has at least one different property from the composition.

In one embodiment of the invention, the at least one constituent of the composition, preferentially the nanoparticle coating, consists of or comprises at least one compound selected in the group consisting of: i) at least one acid such as citric acid, oleic acid, polymethacrylic acid, poly(ethyleneoxide)-b-poly(methacrylic acid) acid, polyacrylic (PAA) acid, polylactic acid, poly(ethylene oxide)-blockpoly(glutamic acid), Phosphonic acid, ii) Albumin, iii) a bisphosphonate, iv) Alendronate, v) Alginate, vi) a metal, vii) Au, viii) Al203, ix) Alginate, x) Aluminium, xi) Aluminium hydroxide, xii) Arabinogalactan, xiii) Bentonite, xiv) cellulose, xv) Carboxymethylcellulose, xvi) Chitosan, xvii) Cholseterol, xviii) Citrate, xix) Dextran, xx) Dimercaptosuccinic acid, xxi) Dopamine, xxii) DOPC, xxiii) DTAP, xxiv) DVB, xxv) Ethylcellulose, xxvi) Erythrocyte, xxvii) Fatty acid, xxviii) Ferrite, xxix) Folic acid, xxx) Gelatin Human, xxxi) serum albumin, xxxii) Liposome, xxxiii) MIPS, xxxiv) MnO, xxxv) Mn3O4, xxxxvi) Oleic acid, xxxvii) PEI, xxxviii) PEG, xxxix) PEO-PGA, xl) PLA (poly(lactide acid), xli) PLGA, xlii) Phosphatidylcholine, xliii) Phosphorylcholine, xliv) Pluronic, xlv) Polyacrylamide, xlvi) Polyacrylic acid, xlvii) PAA, xlviii) Polyaniline Polyethylene glycol preferentially with terminal carboxyl groups , xlix) peptides, L) polypeptides, Li) Poly(ethylene oxide), Lii) Poly(vinyl alcohol), Liii) Ploy(N-isopropylacrylamide), 54) Poly(vinylpyrrolidone), 55) Poly(oligoethylene oxide), 56) Poly(N,N-dimethyl ethylamino acrylate), 57) Poly(imine), 58) Poly(acrylic acid), 59) Poly-D-L lactide, 60) Polyalkylcyanoacrylate, 61) Polymer, 62) PAMAM or PDMAEMA or PPEGMA or PolyNIPAAM, 63) Polyacrylic acid, 64) Polydipyrrole/dicarbazole, 65) Poly-L-lysine, 66) Polymethylmethaacrylate, 67) Polymersomes, 68) Polysaccharide, 69) Agarose or alginate or carrageenan or chitosan or dextran or heparin or Gum Arabic or Pullulan or Starch, 70) Polystyrene, 71) PVA, 72) PVP Silica, 73) an amorphous or mesoporous compound, 74) Silane, 75) SiO2, 76) Sodium Oleate, 77) Starch, 78) Styrène, 79) TaOx, 80), ZrO2, 81) Polysacharides, 82) Agarose or alginate or carrageenan or chitosan or dextran or carboxy-methyl-dextran or heparin or Gum Arabic or Pullulan or Starch, 82) Acide, 83) polymethacrylic acid or poly(ethyleneoxide)-b-poly(methacrylic acid) or polyacrylic acid (PAA) or polylactic acid or poly(ethylene oxide)-blockpoly(glutamic acid) or Phosphonic acid or Dimercaptosuccinic acid or Fatty acids or folic acid or PLA (poly(lactide acid) or Polyacrylic acid PAA 84) Polymer, 85) Dextran or Poly(ethylene oxide) or Poly(vinyl alcohol) or Ploy(N-isopropylacrylamide) or Poly(vinylpyrrolidone) or Poly(oligoethylene oxide) or Poly(N,N-dimethyl ethylamino acrylate) or Poly(imine) or Poly(acrylic acid), 86) Carboxylate, 87) inorganic compound, 88) SiO2 or Al2O3 or ZrO2 or ferrite or MnO or Mn3O4 or Au or Bentonite or Carbon preferentially activated, graphitized, 89) organic metals, 90) MIPs or Celulose or DV8 or Ppy or Chitosan or Polyacrylamide or alginate or PEI or surfactants or Phosphates or Silica or Gold or Dextran or PEG or Alginate or Chitosan, 90) or compound with a chemical function such as Alcohol for example PVA (poly(vinyl alcohol), amide, for example Poly(N-isopropylacrylamide), aldehydes, 91) a compound with a type of interactions with avec hydroxyl groups at the surface of the iron oxide (Fe-OH), 92) a compound with electrostatic interactions, preferentially with a difference in charge between at least two constituents of the composition or at least one constituent of the composition, 93) a compound with a hydrophobic, chelating, covalent, interaction or bond, 93) a compound with a type of functional group that can preferentially be attached at the surface of a metal or iron oxide such as -OH, 94) PEG or Dextran or Polyvinylalcool or pluronic or Dopamine or Amine or Cysteine or phosphonic acid or carboxylic acid or trimethoxy or silane, 95) a compound with a -NH2 group, 96) Chitosan or polyethylenimine or Ploy(L-lysine) or PEG with terminal amine groups or Ethylenamine, 97) a compound with a -COOH group, 98) Polyacrylic acid, 99) carboxymethylcellulose, 100) PEG with terminal carboxyl groups, 101) alginate, 102) Polymethacrylic acid, 103) citrate, and 104) iminodiacetic acid or nitrilotriacetic acid or ethylendiamin or tetraacetic acid or diethylenetrianine pentaacetic acid or folic acid or L-cysteine or an amino acid or Thiol or Dimercaptosuccinic acid or a Phosphate compound or pyridoxal phosphate or adenosine diphosphate or nicotinamide adenine dinucleotide phosphate.

The invention also relates to a method for activating the composition or at least one constituent of the composition or at least one center of activity of the composition or at least one constituent of the composition, according to the invention, preferentially by applying a radiation or physico-chemical disturbance on the composition or at least one constituent of the composition preferentially for a certain or sufficiently long time, preferentially for more than 10⁻³, 1, 0, 1, 10, 10³ second(s),
wherein the activation of the composition or at least one constituent of the composition preferentially comprises at least one at least one of the following event(s) or step(s) of:
   i) releasing or diffusing or triggering the release or diffusion, preferentially in an outward direction relatively to at least one constituent of the composition, (of) at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,
   ii) capturing or diffusing or triggering the release or diffusion, preferentially in an inward direction relatively to at least one constituent of the composition, (of) at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,
      and
   iii) activating or triggering the activation (of) at least one atom, electron, free radical, ion, metal, DNA, RNA, protein, lipid, enzyme, biological or non-biological material, organic or non-organic material, an immune entity from or of or in or at the surface or inside the nanoparticle or at least one constituent of the composition, preferentially against or to fight against a disease or preferentially against or to deactivate or kill at least one pathological cell or preferentially to activate a first type of immune entity that deactivates or kills at least one pathological cell or disease or preferentially to deactivate a second type of immune entity that protects at least one pathological cell or disease or tumor or tumor environment,
wherein the at least one constituent of the composition is preferentially selected in the group consisting of: i) the nanoparticle coating, ii) the nanoparticle core, iii) the cryoprotectant or protectant compound, and iv) the other compound,
wherein the immune entity, preferentially the first and/or second immune entity(ies), is preferentially selected in the group consisting of: i) DNA preferentially different types of DNA, ii) RNA preferentially different types of RNA, iii) an antigen, ii) an antibody, iii) an immune cell, preferentially of or belonging to the innate and/or adaptative immune system(s), iv) an antigen presenting cell (APC), v) a basophil, vi) a dendritic cell, vii) an eosinophil, viii) a granulocyte, ix) a killing cell, x) a natural killer, xi) a leukocyte, xii) a lymphocyte, xiii) a macrophage, preferentially of M1 and/or M2 type(s), xiii) a mast cell, xiv) a neutrophil, xv) a phagocyte, xvi) a B cell, xvii) a T cell, xviii) a CD8 or CD8+ or CD4 or CD4+ or Treg or MAIT or Tγδ T lymphocyte or cell, xix) a helper cell preferentially of Th1 or Th2 type, and xx) a gamma delta T cell,
wherein the radiation is preferentially selected from the group consisting of: i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave,
wherein preferentially the physico-chemical disturbance is or is caused by or induces at least one action selected from the group consisting of:
   i) a variation of an environment of at least one constituent of the composition, where the environment of the at least one constituent of the composition is preferentially a liquid, solid, or gaseous medium or at least one substance surrounding or including the at least one constituent of the composition,
   ii) a variation of the environment of the at least one constituent of the composition preferentially selected from the group consisting of: a pH variation of this environment that is preferentially between 10⁻³ and 10 pH units, a variation in temperature of this environment that is preferentially between 10⁻¹³ and 10³ °C, a variation in redox potential of this environment that is preferentially between 0.001 and 100 V, a variation in viscosity of this environment that is preferentially between 10⁻⁹ and 10²⁰ Pa.s, and a variation in the concentration of at least one substance of the environment that is preferentially between 10⁻¹³ and 10¹⁰ mole per liter, micromole per liter, nano-mole per liter, mole per milliliter, micromole per milliliter, nano-mole per milliliter, mole per cubic meter, mole per cubic decimeter, mole per cubic centimeter or mole per cubic millimeter,
   iii) a modification of at least one condition of the at least one constituent of the composition preferentially selected from the group consisting of a pH variation of the at least one constituent of the composition preferentially between 10⁻³ and 10 pH units, a temperature variation preferentially between 10⁻¹³ and 10³ ° C, a variation in standard potential preferentially between 0.001 V and 100 V, an increase or decrease in charge of the at least one constituent of the composition preferentially between 0.001 and 100 Volt, a variation preferentially between 1 and 10¹⁰ atom(s) in the number of atoms comprised in the at least one constituent of the composition,
   iv) a variation of the concentration of at least one substance of an environment of the at least one constituent of the composition preferentially larger than 10⁻¹³ mole per liter, micromole per liter, nano-mole per liter, mole per milliliter, micromole per milliliter, nan-mole per milliliter, mole per cubic meter, mole per cubic decimeter, mole per cubic centimeter, or mole per cubic millimeter,
   v) a variation in chemical composition of at least one substance of an environment of the at least one constituent of the composition of preferentially less than 10¹⁰ mole per liter, micromole per liter, nano-mole per liter, mole per milliliter, micromole per milliliter, nanomole per milliliter, mole per cubic meter, mole per cubic decimeter, mole per cubic centimeter, or mole per cubic millimeter,
   vi) a modification of at least one substance in an environment of the at least one constituent of the composition preferentially selected from the group consisting of a chemical modification, a structural modification, an appearance of at least one substance in the environment, a disappearance of at least one substance from the environment, and combinations thereof,
      and
   vii) a variation of chemical composition or at least one constituent of the composition of preferentially less than 10¹⁰ substances in an environment of the at least one constituent of the composition, where the variation is preferentially selected from the group consisting of a chemical modification, a structural modification, an appearance of at least one substance in the environment or disappearance of at least one substance from the environment, and combinations thereof.

In one embodiment of the invention, the chelating agent or the at least one constituent of the composition is selected in the group consisting of: (i) chelating agents which have one or more carboxyl groups, (ii) chelating agents which have one or more hydroxyl groups, (iii) chelating agents which have one or more amino and/or carboxyl and/or ketone groups, (iv) chelating agents which have one or more phosphonate and/or phosphonic acid groups, (V) chelating agents which have one or more bisphosphonate and/or trisphosphonate and/or tetra phosphonate groups, (vi) chelating agents which have one or more Sulfonate and/or Sulfonic acid groups, and (vii) chelating agents of monodentate or polydentate type or poly chelating agent that may comprise one or a plurality of functional groups, for example among carboxyl, hydroxyl or amino groups, (viii) of polysaccharide type. (ix) the chelating agents with one or more carboxyl groups or 9) ALA (alpha-lipoic acid) or 10) calcein or 11) carboxyfluorescein or deferasirox or dipicolinic acid or DTPA (diethylenetriaminepen taacetic acid) or EDTA (ethylenediaminetetraacetic acid) or folic acid or vitamin B9 or lactic acid or rhodamine B or carboxymethyl dextran or oxalic acid or citric acid or a compound comprising one or more citric and/or citrate functional groups or phenolic acid or 12) a chelating agent comprising one or more acetate and/or acetic functional groups or BAPTA (aminophenoxyethanetetraacetic acid) or CDTA (cyclohexane-1,2-diaminetetraacetic acid) or EDDHMA (ethylenediaminedi(ohydroxy-p-methylphenyl)acetic acid) or CaNa-EDTA or EDTCA (ethylenediaminetetraacetic acid with Cetavlon(R) or ammonium-type surfactant or EDDA or ethylenediamine-N,N'-diacetic acid) or EDDHA or ethylenediamine-N,N'-bis(2-hydroxyphenylacetic acid or EGTA or ethylene glycol bis(B-aminoethyl ether)-N,N,N',N'-tetraacetic acid or HEDTA (N-(2-hydroxyethyl)ethylenediaminetriacetic acid or HEEDTA or hydroxy-2-ethylenediaminetriacetic acid or NTA or nitrile triacetate or 13) a molecule comprising one or more hydroxyl functional groups, such as catechol or derivatives thereof, or else deferiprone or 14) a molecule comprising one or more amino functional groups, such as dopamine and/or deferoxamine or 15) a molecule comprising one or more aminocarboxylic and/or ketone functional groups, such as doxorubicin, caffeine, D-penicil lamine, pyrroloquinoline and HEIDA (hydroxyethylimino N,N-diethanoic acid) or 16) a molecule comprising at least one phosphonate or phosphonic functional group, such as AEPN (2-aminoethylphosphonic acid) or AMP (aminotris(methylenephosphonate)) or ATMP (aminotris(methylenephosphonic acid)), CEPA (2-carboxy ethylphosphonic acid) or DMMP (dimethyl methylphosphonate) or DTPMP (diethylenetriaminepenta(methylenephos phonic acid)) or EDTMP (ethylenediaminetetra (methylenephosphonic acid)) or HEDP (1-hydroxyethylidene 1,1-diphosphonic acid) or HDTMP (hexamethylenediaminetetra(methylenephosphonic acid)) or HPAA (2-hydroxyphosphonocarboxylic acid) or PBTC (phosphonobutanetricarboxylic acid), PMIDA (N-(phosphor nomethyl)iminodiacetic acid) or TDTMP (tetramethylenediamine-tetra(methylenephosphonic acid)) or ADP (adenosine diphosphoric acid) or 1-12-4-(dipyrromethene boron difluoride)butanoyl)aminododecanoyl-2-hydroxy-sn-glycero-3-phosphate or a sodium salt of L-O-phosphatidic acid or a sodium salt of 1-palmitoyl-2-(dipyrromethene boron difluoride)undecanoyl-sn-glycero-3-phospho-L-serine or 17) a molecule containing at least one bisphosphonate, trisphosphonate or tetraphosphonate functional group, such as 1-hydroxymethylene-bis-phosphonic acid, propanetriphosphonic acid, (nitrilotris(methylene))trisphosphonic acid or (phosphinylidynetris(methylene))trisphosphonic, or 18) a molecule comprising one or more Sulfonate or Sulfonic acid functional groups, or else a dimercapto group, such as BPDS (bathophenanthroline disulfonate or 4,7-di(4-phenylsulfonate)-1,10-phenanthroline), DMPS (dimercaptopropane Sulfonate or 2,3-dimercapto-1-propanesulfonic acid), Sulforhodamine 101 or DMSA (dimercaptosuccinic acid) or 19) polydentate ligands, i.e. chelating agents having more than one atom capable of binding to a metal atom, such as hemoglobin, chlorophyll, porphyrins and organic compounds containing pyrrole rings or 20) polymeric compounds, in particular polysaccharide compounds or 21) rhodamine B, ascorbic acid, citric acid, folic acid, erythrosine, hemoglobin, a low-molecular-weight dextran, anthranilic acid, calcein, alendronate, 3-cyclohexy lamino-1-propanesulfonic acid (CAPS) or EDTA.

The invention also relates to the composition or at least one constituent of the composition according to the invention, wherein the composition or at least one constituent of the composition, comprises an organic part and/or an inorganic part, wherein the inorganic part preferentially comprises the core of the nanoparticle or at least one constituent of the composition, wherein the organic part preferentially comprises the coating of the nanoparticle or at least one constituent of the composition and/or the cryoprotectant or protectant compound, and wherein the percentage in mass of the inorganic part is preferentially larger than the percentage in mass of the organic part.

In one embodiment of the invention, a protectant compound is a compound that maintains or prevents the variation of at least one property of the nanoparticle or at least one constituent of the composition or composition or at least one constituent of the composition preferentially by more than 10²⁰, 10¹⁰, 10⁵, 10, 5, 2, 1 0% or preferentially by a factor of more 10²⁰, 10¹⁰, 10⁵, 10, 5, 2, 10, preferentially over time, preferentially over more than 1 second or 1 year, preferentially between the time t₁ and t₂, where the properties of the nanoparticle or at least one constituent of the composition or composition or at least one constituent of the composition at times t₁ and t₂ are preferentially P₁ and P₂.

In some cases, P₂/P₁ and/or t₂/t₁ can be smaller than 10²⁰, 10¹⁰, 10⁵, 10, 5, 2, 10.

In some other cases, P₂/P₁ and/or t₂/t₁ can be larger than 0, 10⁻¹⁰, 0.1, 0, 1, 10, 50, 10³ or 10⁵.

In some cases, the factor α is equal to V₂/V₁, where V₂ is the volume occupied by the cryoprotectant or protectant compound or protectant compound in the composition or at least one constituent of the composition and V₁ is the volume occupied by the at the at least one nanoparticle or at least one constituent of the composition or the at least one chain in the composition or at least one constituent of the composition.

In some cases, α is larger than or equal to 1, 2, 5, 10, 100, 10³ or 10⁵.

In some other cases, α is smaller than or equal to 10⁵, 10³, 100, 10, 5, 2 or 1.

In some other cases, α is larger when the composition or at least one constituent of the composition is partly or fully in a liquid state than when it is partly or fully in a solid or powder form.

In some cases, the percentage in mass of cryoprotectant or protectant compound or protectant compound or nanoparticle or at least one constituent of the composition is larger than or equal to 0, 10¹⁰, 10 ', 1, 5, 10, 50, 75, 99 or 100%.

In some other cases, the percentage in mass of cryoprotectant or protectant compound or at least one constituent of the composition is smaller than or equal to 100, 99.99, 99, 85, 75, 50, 25, 20, 10, 5, 2, 1 or 0%.

In still some other cases, the percentage in mass of the nanoparticle or at least a first constituent of the composition is larger preferentially by a factor β than the percentage in mass of the cryoprotectant or protectant compound or of at least one second constituent in or of the composition, preferentially when the composition or at least one constituent of the composition is in powder or solid form.

In some cases, the factor β is equal to PM₂/PM₁, where PM₂ is the percentage in mass of the at least one nanoparticle or at least a first constituent of the composition and PM₁ is the percentage in mass of the cryoprotectant or protectant compound or at least one second constituent of the composition.

In some cases, β is larger than or equal to 1, 2, 5, 10, 100, 10³ or 10⁵.

In some other cases, β is smaller than or equal to 10⁵, 10³, 100, 10, 5, 2 or 1.

In one embodiment of the invention, the nanoparticle or nanoparticle core or least one constituent of the composition comprises a percentage in mass in at least one metal preferentially iron preferentially in terms of metallic composition that is larger than 0, 1, 5, 10, 50, 75, 90, 99 or 99.9%.

In one embodiment of the invention, the nanoparticle or nanoparticle coating or least one constituent of the composition comprises a percentage in mass in at least one metal preferentially iron preferentially in terms of metallic composition that is smaller than 100, 99.9, 90, 75, 50, 25, 10, 5, 2 or 1%.

In one embodiment of the invention, the percentage(s) in mass of the nanoparticle or protectant compound, or at least one constituent of the composition is(are) larger than or equal to 0, 1, 5, 10, 50, 75, 90, 99 or 99.9%.

In another embodiment of the invention, the percentage(s) in mass of the nanoparticle or protectant compound, or at least one constituent of the composition, is(are) smaller than or equal to 100, 99.9, 90, 75, 50, 25, 10, 5, 2 or 1%.

In one embodiment of the invention, the percentage(s) in mass of the inorganic and/or organic part(s) in the composition or at least one constituent of the composition, preferentially in the dried composition is(are) larger than or equal to 0, 1, 5, 10, 50, 75, 90, 99 or 99.9%.

In another embodiment of the invention, the percentage(s) in mass of the inorganic and/or organic part(s) in the composition or at least one constituent of the composition, preferentially in the dried composition, is(are) smaller than or equal to 100, 99.9, 90, 75, 50, 25, 10, 5, 2 or 1%.

In one embodiment of the invention, the nanoparticle or at least one constituent of the composition, has/have at least one of the following properties selected in the group consisting of:
(a) magnetic, diamagnetic, superparamagnetic, ferromagnetic, ferrimagnetic, and/or paramagnetic behavior(s) or property(ies), preferentially observed under the application of magnetic field of strength preferentially larger than 10⁻⁵⁰, 10⁻⁴⁰, 10⁻²⁰, 10⁻¹⁰, 10⁵, 10⁻² or 10⁻¹ T, preferentially observed at temperatures lower than 10¹⁰, 10⁵, 10³, 10², 10 or 1 K, wherein in some cases, the core can have different magnetic property(ies) from the coating, for example, the core can be ferromagnetic or superparamagnetic while the coating can be diamagnetic or paramagnetic.
(b) a crystalline part or structure comprising at least 1, 2, 5, 10, 50, 100, 10³, 10⁵, 10⁷, 10⁹, 10²⁰ or 10⁵⁰ crystalline plane(s) or crystalline ordered structures, which can preferentially be observed or measured under electron microscopy, wherein in some cases, the core can have a different crystalline structure from the coating, for example, the core can comprise more than 1, 5, 10, 10³ or 10⁵ crystalline plane(s) or crystalline ordered structure(s) while the coating can have less than 10⁵, 10³, 10, 5 or 2 crystalline planes or crystalline ordered structures.
(c) a composition or at least one constituent of the composition made of metal(s) or metal oxide(s), preferentially iron oxide, most preferentially maghemite and/or magnetite, wherein in some cases, the core comprises a different composition or at least one constituent of the composition from the coating, for example, the core comprises more than 1, 5, 10, 25, 50, 75, 90, 95 or 99 percent or percent in mass of iron oxide while the coating comprises less than 99, 95, 90, 75, 50, 10, 5 or 1 percent or percent in mass of iron oxide, wherein this percentage can be the ratio between the quantity, volume, number of atoms, mass of iron oxide comprised in the core and/or coating divided by the total quantity, total volume, total number of atoms, total mass, of all chemical element(s) comprised in the core and/or coating.
(d) single domain, or be magnetically mono-domain,
(e) a magnetic microstructure, which can be characterized by the presence of magnetic field lines, which can be oriented in a preferential direction such as an axis of easy magnetization or a crystallographic direction of the core of the nanoparticle or at least one constituent of the composition(s) such as [111], where such a magnetic microstructure can under certain conditions be observable, in particular by electronic holography,
(f) a size comprised between 1 nm and 10⁵ µm, 1 nm and 10³ µm, 1 nm and 100 µm, 1 nm and 10 µm, 1 nm and 1 µm, 5 nm and 1 µm, 5 and 500 nm, 5 and 250 nm, 5 and 100 nm, 5 and 80 nm, 5 and 60 nm, 10 nm and 1 µm, 10 and 500 nm, 10 and 250 nm, 10 and 100 nm, 10 and 80 nm, 10 and 60 nm, 15 nm and 1 µm, 15 and 500 nm, 15 and 250 nm, 15 and 100 nm, 15 and 80 nm, 15 and 60 nm, 20 nm and 1 µm, 20 and 500 nm, 20 and 250 nm, 20 and 100 nm, 20 and 80 nm, or between 20 et 60 nm,
(g) a size in some cases larger than 0.1, 1, 2, 5, 10, 15, 20, 25, 30, 35 or 40 nm,
(h) a size in some other cases lower than 10¹⁰, 10⁵, 10⁴, 2000, 1000, 500, 400, 300, 200, 150, 120, 100, 95, 90, 80, 75, 70, 65, 60, 55, 50, 45, 40, 35, 30, 25, 20, 15, 10 or 5 nm,
(i) a zeta potential, charge, or surface charge comprised between -10¹⁰ mV and 10¹⁰ mV, -10⁵ mV and 10⁵ mV, -10⁴ mV and 10⁴ mV, -10³ mV, -10² mV and 10² mV, -10 and 10 mV, preferentially at pH comprised between 0 and 14, 1 and 13, 2 and 12, 3 and 11, 4 and 10, 5 and 9, or between 6 and 8.
(j) a zeta potential, charge, or surface charge, which is in some cases larger than -10⁵⁰, -10²⁰, -10¹⁰, -10⁵, -10³, -10, -5, -1, 0, 5, 10, 20, 50, or 100 mV, preferentially at pH larger than 0, 1, 2, 3, 4, 5, 6, 7, 8, 9, 10, 11, 12 or 13,
(k) a zeta potential, charge, or surface charge, which is in some other cases larger than -10⁵⁰, -10²⁰, - 10¹⁰, -10⁵, -10³, -10, -5, -1, 0, 5, 10, 20, 50, or 100 mV, preferentially at pH lower than 14, 13, 12, 11, 10, 9, 8, 7, 6, 5, 4, 3, 2, 1 or 0,
(l) a zeta potential, charge, or surface charge, which is in some other cases lower than 10⁵⁰, 10²⁰, 10¹⁰, 10⁵, 10³, 10, 5, 1, 0, -5, -10, -20, -50, or -100 mV, preferentially at pH larger than 0, 1, 2, 3, 4, 5, 6, 7, 8, 9, 10, 11, 12 or 13,
(m) a zeta potential, charge, or surface charge, which is in some other cases lower than 10⁵⁰, 10²⁰, 10¹⁰, 10⁵, 10³, 10, 5, 1, 0, -5, -10, -20, -50, or -100 mV, preferentially at pH lower than 14, 13, 12, 11, 10, 9, 8, 7, 6, 5, 4, 3, 2, 1 or 0.
(n) an isoelectric point comprised between 0 and 14, 1 and 13, 2 and 12, 3 and 11, 4 and 10, 5 and 9, or between 6 and 8,
(o) in some cases, an isoelectric point in some cases larger than 0, 1, 2, 3, 4, 5, 6, 7, 8, 9, 10, 11, 12 or 13, and/or
(p) in some other cases, an isoelectric point in some other cases lower than 14, 13, 12, 11, 10, 9, 8, 7, 6, 5, 4, 3, 2, 1 or 0,
(q) an isotonicity, preferentially in some cases at pH smaller or larger than 0, 1, 2, 3, 4, 5, 6, 7, 8, 9 10, 11, 12, 13 or 14, preferentially in some other cases at pH comprised between 0 &nd 14 or between 1 and 13 or between 3 and 10,
(r) an interaction strength between two constituents of the composition or at least one constituent of the composition, such as the core and coating, that is sufficiently strong to form a complex comprising these two constituents,
(s) an interaction strength between two constituents of the composition or at least one constituent of the composition, such as the nanoparticle or at least one constituent of the composition and cryoprotectant or protectant compound or protectant compound, that is sufficiently weak to prevent the formation of a complex comprising these two constituents
(t) an interaction or bond or interaction or force between two constituents of the composition or at least one constituent of the composition that is of the type selected in the group consisting of : i) weak, ii) metallic, iii) ionic, iv) covalent, v) London or dispersion, vi) dipole-dipole, vii) hydrogen, viii) nonpolar or polar bond, ix) van der Waals, x) electrostatic, xi) charged, xii) magnetic, xiii) thermal, ix) atomic, x) molecular, xi) nanometric, xii) complexation, and xiii) solid, liquid, gaseous interaction or bond or interaction or force.
   and
(r) a solid, liquid or gaseous form or state.

In some cases, the coating and core form a complex.

In some other cases, the cryoprotectant or protectant compound and nanoparticle or at least one constituent of the composition don't form a complex or form a weaker or less stable complex than the complex formed by the coating and core.

In some cases, the constituent of the composition can be selected in the group consisting of: i) the constituent of the composition, ii) the other constituent of the composition, iii) the first constituent of the composition, and v) the second constituent of the composition.

The description is followed by the following non-limiting example(s)

### Example 1: Method to dope magnetosomes with zinc by growing magnetotactic bacteria MSR-1 in the presence of a source of zinc in 6.5-liter fermenters.

This example describes the experimental protocol used to dope magnetosome with zinc by growing MSR-1 MTB in presence of a source of zinc, resulting in the production of zinc-doped magnetosomes. In this example, MTB are grown in 6.5-liter Applikon fermenters with an ez control system and piloted by the BioXpert software for the regulation of stirring and airflow rates. The composition or at least one constituent of the compositions of the pre-growth medium, growth medium, and fed-batch medium are indicated for 1 liter of medium in tables 1, 2 and 5 for the tested conditions. As a whole, 2 different culture conditions are tested: Condition A, in absence of a source of zinc and Condition B in presence of a source of zinc in the fed-batch medium at a concentration of 452 micromoles. During the first day of the experiment (D1), a first step consists in collecting the tubes of 2 ml containing 1 mL of MSR-1 cellular stock tube from the freezer at -80 °C, in thawing the tubes by letting them at room temperature for 10 minutes. Under a hood, we collected from these tubes 1 ml comprising 5.108 MSR-1 magnetotactic bacteria that we have inserted in 500 mL bottles filled with 250 mL of pre-growth medium. The 500 ml bottles are incubated for 8 days between D1 and D8 in an incubator at 29.5 °C and between D7 and D8 under orbital agitation at 110 rpm. During the eighth day (D8), the second pre-growth step started. For that, 3-liter fermenters are filled with 1L of deionized water and autoclaved. The fermenters are placed under the hood and then filled with 500 mL of filtered pre-growth medium. The 500 mL bottles from the first step of pre-growth, are placed under a hood. 500 mL of the pre-growth medium containing the MSR-1 bacteria were transferred in the 3-liter fermenters. The temperature was maintained at 29.5°C and agitation at 110 rpm in the 3-liter fermenters during 48 hours between D8 and D10. During the tenth day (D10), the growth step started. For that, 6.5-liter fermenters are filled with 3-liter of deionized water and autoclaved. The fermenters were then filled with 500 mL of filtered growth medium. The 3-liter fermenters from the second step of pre-growth, are placed under a hood. About 900mL of pre-growth medium containing the MSR-1 bacteria originating from the second step of pre-growth is then transferred respectively in the 6.5-liter fermenters. Between day D10 and D18, an acidic fed-batch medium comprising a source of iron (condition A) and sources of iron and zinc (condition B) were added to the growth medium to enable the synthesis of magnetosomes (condition A) and zinc-doped magnetosomes (condition B) by MSR-1 bacteria, while maintaining the pH of the growth medium at 6.9. During the growth step, the temperature was maintained at 29.5 °C, the stirring fixed at 150 rpm (rotation per minute) and airflow rates were adjusted to maintain to oxygen concentration above 0.1% and below 1% enabling the growth of MSR-1 magnetotactic bacteria and the synthesis of magnetosomes by these bacteria. The optical densities, measured at 565 nm of the bacterial suspensions, the volume of Fed-batch added to the medium at different days of the pre-growth step (D8 and D10) and growth step (D10, D12, D16, D17, D18) are indicated in table 6.

Following fermentation at D18, MSR-1 MTB are washed and concentrated in deionized water using tangential flow filtration to an OD565 ~ 30 and stored at -80°C for no less than 24 hours. Concentrated MSR-1 MTB from conditions A and B are thawed at 40°C for 2 h and diluted with deionized water to an OD565 ~ 20. MSR-1 MTB are then lysed in 2 M KOH at 80°C for 1 h under agitation (150 rpm). A direct current field is then applied to the suspension for 12 hours to separate magnetosomes from organic material residues. Magnetosomes are washed 2 times with 10 X Phosphate-buffered saline (PBS) then 3 times with deionized water. Magnetosomes are collected in 50 ml-conical tubes and centrifuged at 10°C, 3380 g for 30 min. Tubes containing magnetosomes are stored at -80 °C for 24 hours and lyophilized at -50 °C, 0.003 mbar for 24 hours. Magnetosomes are then purified in a muffle furnace as followed, 50 °C (1 h) - 270 °C (2 h) - 310 °C (2 h) - 420 °C (2 h). Purified magnetosomes (condition A) and purified Zinc-doped magnetosomes (condition B) are thus obtained.

The content of zinc in purified Magnetosomes (condition A) and in purified zinc-doped magnetosomes (condition B) is determined by inductively coupled plasma-mass spectrometer (ICP-MS). Two milligrams of purified Magnetosomes are dissolved in 35-microliter of 37% w/v HCl and 286-microliter of 70% w/v HNO3 at ~ 20 °C for 12 hours. Ultrapure water is added to a volume of 10-milliliter. Analysis is then carried out by ICP-MS. The Zn/Fe ratio in purified magnetosomes (condition A) and purified zinc-doped magnetosomes (condition B) are presented in figure 2. When no source of zinc is brought to the growth medium (condition A), the percentage of zinc with respect to the iron in purified magnetosomes is very low at 0.06 % while with a concentration of zinc sulfate of 30 micromoles in the growth medium the percentage of zinc with respect to the iron in purified zinc-doped magnetosomes (condition B) is 0.74 %.

### Example 3: method to coat purified zinc doped-magnetosomes from MSR-1 MTB with folic acid or citric acid covalently bound with a photosensitizer.

This example describes the experimental protocol used to coat purified zinc-doped magnetosomes originating from MSR-1 MTB with citric acid and a derivative of Protoporphyrin IX.

In the dark and under an inert atmosphere, the corresponding photosensitizer Protoporphyrin IX (= P1, CAS n° 553-12-8) was dissolved in 20-50 mL of chloroform (CAS n° 67-66-3). N- hydroxysuccinimide (3 eq., CAS n° 6066-82-6) and N, N' dicyclohexylcarbodiimide (3 eq., CAS n° 538-75-0) are added to the photosensitizer solution. The mixture was stirred at 40 °C, for 4-24 hours, under argon. Purification of the crude material is performed using a silica gel column with DCM (Dichloromethane, CAS n° 75-09-2). P1-NHS is obtained, after recrystallization in DCM/hexane (CAS n° 110-54-3).

P1-NHS (1éq) and N-Boc-2,2'-(ethylenedioxy)diethylamine (1éq., CAS n°153086-78-3) are dissolved in 30 mL of THF (Tetrahydrofuran, CAS n° 109-99-9). The reaction mixture is stirred at room temperature, under a nitrogen atmosphere and in the dark for 18 hours. The solvent is removed under vacuum. Purification of the crude material is performed using a silica gel column with a solution of ethanol and chloroform and P1-NHBoc is obtained. It is dissolved in 2 mL of TFA (Trifluoroacetic acid, CAS n°76-05-1). The solution is stirred for 2 hours at room temperature, in the dark and under nitrogen. Next, TFA is removed by lyophilization. The residue is solubilized in 10 mL of DCM, and anhydrous potassium carbonate is added until the color changed. After filtration, the organic layer is concentrated. Purification of the crude material is performed using a silica gel column with a solution of DCM and MeOH at different gradients (v/v). P1-NH2 is thus obtained.

In the dark and under an inert atmosphere, folic acid or citric acid (1 eq.) and N, N' dicyclohexylcarbodiimide (1 eq.) is dissolved in 5mL of anhydrous DMSO (Dimethyl sulfoxide, CAS n°67-68-5) and 2 mL of pyridine (CAS n° 110-86-1) and the mixture is stirred for 15 min at room temperature. Compound P1-NH2 (0.9 eq.) is then added, and the reaction mixture is further stirred for 24h at room temperature. The solution is slowly poured into vigorously stirred cold diethyl ether (CAS n°). The precipitate obtained by centrifugation is collected and washed with diethyl ether. The powder is dried under a high vacuum. The residue is purified by HPLC on a C18 preparative column using an acetonitrile/water gradient. P1-folic or P1-citric is thus obtained and presented in figure 3.

The coating process is carried out in the dark and under sterile conditions, using a biosafety hood. A P1-folic or P1-citric solution was prepared at a concentration of 50 mg/ml in ultrapure water, at pH 6 and is filtered with a 0.22 µm polyether sulfone filter for sterilization. Different volumes of this solution are added to the 50 milliliter-glass bottles containing 80 mg of purified zinc doped-magnetosomes (corresponding to 40 mg of Fe) previously dispersed in 16 ml of ultrapure water. The suspension is sonicated for 5 min in a sonicating bath (SB) (Elma, TI-H 20) at 25 Kilohertz at room temperature. Following sonication, the pH of the suspension is adjusted at 6 using NaOH at 1 Mole and the suspension is then sonicated in the SB at 25 Kilohertz, 80°C for 10-30 h for preparing P1-folic purified zinc doped-magnetosomes (ZnM-P1-folic) or P1-citric purified zinc doped-magnetosomes (ZnM-P1-citric). After the sonication, the ZnM-P1-folic and ZnM-P1-citric suspension are centrifuged (3380) at 10°C (Eppendorf, Centrifuge 5810 R) for 45 min to remove excess of P1-citric or P1-folic in the supernatant. ZnM-P1-folic and ZnM-P1-citric are then re-suspended in 20 ml of water and stored in the dark at +4°C.

## Claims

1. Nanoparticle for use in a method, for at least one of the following purposes: i) increasing the production of free radicals ii) amplifying radiation, iii) amplifying the effect of radiation, iv) increasing the destruction of the body part, preferentially pathological or tumor cells or a tumor or cancer or virus or a pathological part of the body part, v) destroying or inactivating at least 1, 1.1, 2, 5 or 10 times more pathological or tumor cells or virus preferentially when the pathological cell or pathological body part is exposed to radiation in the presence of the composition than when the pathological cell or pathological body part is exposed to radiation in the absence of the composition, vi) reducing side effects of radiation or preserving the body part, preferentially non-pathological or healthy cells or healthy body part, preferentially surrounding the pathological body part, vii) preserving or maintaining activated or alive at least 1, 1.1, 2, 5 or 10 times more healthy cells preferentially when the healthy cells are exposed to radiation or subjected to an indirect such as an immune reaction that occurs after the application of radiation on the body part in the presence of the composition than when the healthy cells are exposed to radiation or subjected to an indirect such as an immune reaction that occurs after the application of radiation on the body part in the presence of the composition in the absence of the composition, viii) sonodynamic therapy, ix) photodynamic therapy, x) radiation therapy, xi) a diagnosis, and x) a treatment,
wherein the method preferentially comprises at least one step of::
1) Introducing or reintroducing in a body part to be treated at least one nanoparticle comprising:
- a first center of a first center activity, C_{A1}, such as a first center of free radical production or radiation amplification, C_{1FRP}, comprised in its core,
and
- a second center of activity, C_{A2}, such as a second center of free radical production or radiation amplification, C_{2FRP}, comprised in its coating,
2) Applying an external radiation on the body part comprising the nanoparticle(s) during a time t₁;
3) Not applying the external radiation on the body part comprising the nanoparticle(s) during a time t₂ or applying a radiation of lower intensity, energy, power or power density during the time t₂ than during the time t₁;
4) Re-applying the external radiation on the body part comprising the nanoparticle(s) during a time t₃ or applying an external radiation of larger intensity, energy, power or power density during t₃ than during t₂;
wherein C_{A1} and C_{A2} are different compounds,
wherein C_{A1} and C_{A2} are selected in the group consisting of:
A) a radio-sensitizer or amplifier of radiation, a photosensitizer or amplifier of light radiation, an acoustic sensitizer or amplifier of acoustic radiation or wave, a sonosensitizer or amplificatory of acoustic wave or ultrasound, a particle radiation sensitizer or amplifier of particle radiation, where the particle comprises (or not) a mass, it is a thermal-sensitizer or amplifier of heat or cold or thermal treatment, an amplifier of the medical effect of a compound,
and
B) an attenuator of radiation, of light radiation, of acoustic radiation or wave, of particle radiation, where the particle comprises (or not) a mass, of heat or cold, of thermal treatment, and/or of the medical effect of a compound,
wherein optionally the nanoparticle is comprised in a composition,
wherein optionally the composition comprises the nanoparticle and a protectant compound,
wherein the distance separating C_{A1} and C_{A2} in the composition is preferentially larger than 0.1 or 1 nm,

2. Nanoparticle for use according to claim **1,** wherein the protectant compound is selected in the group consisting of:
i) a cryo-protectant or a compound that protects or maintains at least one property of the composition when it is cooled down, preferentially below 0°C,
ii) a thermo-protectant or a compound that protects or maintains at least one property of the composition when it is exposed to a temperature gradient, preferentially of more than 0.1, 1 or 10°C,
iii) an oxydo-reduction-protectant or a compound that protects or maintains at least one property of the composition when it is exposed to a reduction or an oxidation, preferentially resulting in a different oxidation state of the nanoparticle,
iv) a pressure-protectant or a compound that protects or maintains at least one property of the composition when it is exposed to a pressure or pressure variation, preferentially a pressure larger than 10⁻¹⁰, 10⁻³, 1, 0, 1, 10³ or 10⁵ bar or atm or mbar or Pa,
v) a pH-protectant or a compound that protects or maintains at least one property of the composition when it is exposed to a pH variation, preferentially a pH variation of more than 0, 1, 3, 5, 10 or 14 pH unit(s),
vi) a radiation-protectant or a compound that protects or maintains at least one property of the composition when it is exposed to a radiation, preferentially a radiation selected in the group consisting of: : i) a magnetic or electric or electromagnetic field or wave, a wave a particulate radiation, ii) laser light, iii) light produced by a lamp, iv) light emitted at a single wavelength, v) light emitted at multiple wavelengths, vi) a ionizing radiation, vii) microwave, viii) radiofrequencies, and ix) a sound, an ultrasound an infrasound, or an acoustic wave.
wherein the at least one property of the composition is selected in the group consisting of: i) a chain arrangement of at least two nanoparticles, ii) an activity, iii) the size, iv) the cohesion, v) the magnetic property, vi) the composition of at least one constituent of the composition,
wherein the chain arrangement of the two nanoparticles is maintained when at least two nanoparticles are or remain arranged in changed in the composition,
wherein the activity of at least one constituent of the composition is maintained when it does not decrease or disappear,
wherein the size of at least one constituent of the composition is maintained is maintained when it does not vary by more than 50% or by more than 0.1, 1, 10 or 10⁶ nm,
wherein the cohesion of at least one constituent of the composition is maintained when at least 1, 2, or 3 or all constituents of the composition remain in the composition,
wherein the magnetic property of at least one constituent of the composition is maintained when the coercivity, magnetization, remanent magnetization, saturation magnetization does not vary by more than 50% or by more than 100 mT or 10³ Oe or Oe per mg of constituent(s) or when at least one constituent of the composition does not change from one magnetic state to another magnetic state,
wherein the composition of at least one constituent of the composition is maintained when less than 1, 5, 10, 10⁵ or 10¹⁰ atom(s) preferentially per constituent or 50% of constituents remain in the composition,
wherein the activity of the composition is preferentially selected in the group consisting of: a therapeutic, immunological, pharmacological, chemotherapeutical, metabolic, thermal, vaccine, hyperthermia, cryo-thermal, ablative, prophylactic, and diagnosis activity of at least one constituent of the composition,
wherein the magnetic state is selected in the group consisting of: a diamagnetic, paramagnetic, superparamagnetic, ferromagnetic, and ferrimagnetic state.
wherein the composition is in the form of a powder or a liquid suspension,
wherein the composition is isotonic,
wherein the percentage in mass of protectant compound in the composition is comprised between 0.5 and 50 %,

3. Nanoparticle for use in the method of claim **1** or **2,** wherein C_{A1} and/or C_{A2} is/are comprised in the nanoparticle(s) at a concentration ranging from 1 to 10¹⁰ C_{A1} and/or C_{A2} per nanoparticle, wherein C_{A1} and/or C_{A2} is/are preferentially atom(s), ion(s), nanoparticle(s), nanoelement(s), or assembly(ies) of atom(s), ion(s), nanoparticle(s), nanoelement(s), or assembly(ies), wherein C_{A1} and/or C_{A2} preferentially has(have) a size lower than the size of at least one nanoparticle, of its core, of its coating or of 100, 10 or 1 nm.

4. Nanoparticle for use in the method according to claim **1** or **2,** wherein at least two steps among steps 1), 2), 3) and 4) follow each other, *i.e.* that preferentially: i) step 2 follows step 1), step 2) follows step 3), step 2) follows step 4), step 3) follows step 1), step 3) follows step 2), step 3) follows step 4), step 4) follows step 1), step 4) follows step 2), step 4) follows step 3), step 1) follows step 2), step 1) follows step 3), and/or step 1) follows step 4).

5. Nanoparticle for use in a method according to claim **2,** wherein the nanoparticle, preferentially C_{A1}, comprises:
i) At least compound C and compound B, where compound C is another metal than iron such as Zinc and/or compound B is Oxygen, where the nanoparticle core is optionally composed of ZnO in this case,
ii) At least compound C, compound B, and another compound A, where compound C is another metal than iron such as Zinc, compound B is Oxygen, and compound A is iron, where the core is optionally composed of ZnFe₂O₄ or ZnFeO₃ in this case,
and/or
iii) At least compound C and/or compound D, wherein compound C and/or compound D is/are radioactive compound(s).

6. Nanoparticles for use in the method according to any one of claims **1** to **5,** wherein at least two nanoparticles are organized in a chain.

7. Nanoparticles for use in the method according to any one of claims **1** to **6,** which is **characterized by** at least one of the following properties:
i) t₂ is smaller than t₁ and/or t₃;
ii) the intensity, power, energy, or energy density of the radiation produced by C_{1FRP} and/or C_{2FRP} is smaller than the intensity, power, energy, or energy density of the radiation produced by the external radiation.

8. Nanoparticles for use in the method according to any one of claims **1** to **7,** which is **characterized by** at least one of the following properties:
iii) t₂ is larger than t₁ and/or t₃;
iv) the intensity, power, energy, or energy density of the radiation produced by C_{A1}, C_{1FRP}, C_{A2}, and/or C_{2FRP} is larger than the intensity, power, energy, or energy density of the radiation produced by the external radiation.

9. Nanoparticle for use in a method according to any ones of claims **1** to **8,** wherein the radiation is selected in the group consisting of: i) an undulating radiation, ii) a particulate radiation, iii) an acoustic wave, iv) a heating radiation, v) a non-heating radiation, vi) an ionizing radiation, vii) a non-ionizing radiation, viii) a laser, ix) a light, x) a sound radiation, xi) an ultrasound radiation, xii) a hyper-sound radiation, xiii) an infrasound radiation, xiv) an X-ray radiation, xv) an electromagnetic radiation, xvi) a radiation due to an electric, magnetic, electromagnetic field, a magnetic field, an alternating magnetic field, xvii) a thermal radiation, xviii) a radiation due to or producing by cold, xix) a radiation due to or producing heat, xx) a radiation produced by or associated with zero-mass, non-zero-mass, neutrons, photon protons, electrons, positron, alpha particle, beta, gamma, neutrinos or muon particle presence or emission, xxi) a radiation oscillating at least 1 frequency or wavelength, and xxii) a source producing any of the previously cited radiation(s).

10. Nanoparticle for use in a method according to any of the claims **1** to **9,** wherein the nanoparticle comprises a metallic core that is synthesized by a living organism or nanoparticle producing cells, preferentially a magnetosome or magnetosome mineral, wherein the living organism or nanoparticle producing cells is preferentially a magnetotactic bacterium, wherein the nanoparticle preferentially comprises a coating, preferentially not synthesized by the living organism.

11. Nanoparticle for use in a method according to any of the claims **1** to **10,** wherein C, D, C_{A1} and/or C_{A2} is/are photosensitizer(s), selected in the group consisting of: 1) Acridine, such as Acridine Orange, acridine yellow, 2) ALA (5-Aminolevulinic acid), 3) Aluminum phthalocyanine tetrasulfonate (AlPcS4), 4) Aminolevulinic acid, delta- Aminolevulinic acid, 5) Antihistamines, 6) Azulene, 7) Bavteriochlorin, 8) TOOKAD or TOOKAD Soluble, 9) WST-11, 10) LUZ11, 11) BC19, 12) BC21, 13) porphyrin such as Benzoporphyrin derivative monoacid ring A (BPD-MA), 14) Chlorin such as Chlorin e6, m-tetrahydroxyphenylchlorin 15) Foscan, 16) Verteporfin, 17) benzoporphyrin derivative mono acid ring A, 18) Monoaspartyl chlorin(e6), 19) talaporfin sodium, 20) HPPH, 21) Transition metal compounds, 22) Chlorine e6 green porphrin, 23) Chlorine e6 porphrin, 24) Coal Tar and Derivatives, 25) Contraceptives, Oral and Estrogens, 26) Curcumin, 27) Cyanine, 28) Cysview, 29) Dyes such as synthetic dyes, 30) Phenothiazinium salts, 31) Rose Bengal, 32) Squaraines, 33) BODIPY dyes, 34) Phenalenones, 35) benzophenoxazinium dyes, 36) Erythrosine, 37) Flavins, 38) Foscan, 39) Fotoscan, 40) Fullerenes such as cationic fullerenes, 41) Furocoumarins, 42) HAL (Hexaminolevulinate), 43) Hemoporfin, 44) 2-(1-Hexyloxyethyl)-2-devinyl pyropheophorbide (HPPH), 45) Hypericin, 46) Hypocrellin, 47) ICG (Indocyanine Green), 48) Levulan, 49) MAL -methyl aminolevulinate), 50) Meta-tetra(hydroxyphenyl)chlorin (m-THPC), 51) Metvix, 52) Methylene Blue, 53) Monoterpene, 54) Motexafin lutetium (Lu-Tex), 54) N-aspartyl chlorin e6 (NPe6), 55) Nanoparticle or nanomaterial, 56) Natural products or compounds, 57) NonSteroidal Anti-Inflammatory Drugs, 58) Palladium bacteriopheophorbide (WST09), 59) Phatalocyanin dyes, 60) Phenothiazines, 61) Photochlor, 62) Photofrin, 63) Photosens, 64) Phthalocyanine such as Liposomal ZnPC, 65) Chloroaluminium sulfonated phthalocyanine (CASP), 66) Silicon phthalocyanine (PC4), 67) RLP068, 68) Porfimer sodium, 69) Porfins, 69) Porphyrins, such as 5,10,15,20-Tetrakis(1-methylpyridinium-4-yl) porphyrin tosylate, 70) XF70, 71) Protoporphyrin, 72) ALA-induced protoporphyrin IX, 73) Psoralens, 74) Quantum dots, 75) Quinones, 76) Riboflavin, 77) Rose Bengal, 78) silicon or Silicon phthalocyanine (Pc4), 79) Sulfonamides, 80) Sulfonylureas, 81) Talaporfin or Talaporfin soudium, 82) Temoporfin, 82) Tetrahydropyrroles, 83) Tin ethyl etiopurpurin, 84) Titanium dioxide, 85) Toldudine blue O, 86) Transition metal compounds such as Ruthenium(II), polypyridyl complexes, ruthenium, rhodium, cyclometalated, Rh(II)-Rh(II) bridged dimer compounds, platinum(II), gold(III), 87) Verteporfin, 88) Vulnic based compound such as Aminovulinic, aminovulinic acid, 89) WST11, and 90) Xanthene.

12. Nanoparticle for use in a method according to any of the claims **1** to **11,** wherein C, D, C_{A1} and/or C_{A2} is/are sono-sensitizer(s), selected in the group consisting of: 1) ABS-FA, 2) Acrylonitrile Butadiene Styrene, 3) Styrene, 4) Folic acid, 5) AIMP NP, aminoacyl tRNA synthetase complex-interacting multifunctional protein, 6) Au Nanomaterial, 7) gold, 8) Au-MnO nanomaterial, 9) manganese oxide, 10) Antineoplastic drugs, 11) NSAIDs, 12) nonsteroidal anti-inflammatory drug, 13) Artemether, 14) 5-ALA (5-aminolevulinic acid), 15) Acridine, Acridine Orange, 16) Au-doped TiO2, 17) Carbon based nanomaterial, 18) carbon nanotube, 19) Chlorine, 20) Ce6, 21) PTX, Paclitaxel, 22) chemotherapeutic drug or compound, 23) infrared dye or IR783, 24) Curcumin, 25) Cyanine or Cu-Cyanine, 26) DHMS, 27) dimethylsulfure, 28) Docetaxel, 29) chemotherapeutic drug or compound, 30) DOX/Mn-TPPS@RBCS, 31) doxorubicin, 32) manganese, 33) blood cell, 34) red blood cell, cell, 35) polymer, 36) elastomer, 37) Erythosin or Erythosin B, 38) FA or FA-OI or FA-OI NP or folic acid, 39) F3-PLGA@MB/Gd NPs, 40) poly(lactic-co-glycolic acid), 41) gadolinium, 42) Fe-TiO2 or titanium oxide, 43) Fe-VS₂, 44) iron, 45) vanadium disulfide, 46) FMSNs-DOX, 47) silica, 48) HCQ, 49) hydrochloroquine, 50) HP, 51) hematoporphyrin, 52) HMME, 53) hematoporphyrin monomethyl ether, 54) HSYA or Hydroxysafflor yellow A, 55) Hypocrellin, Hypocrellin B, 56) IR780, 57) Levofloxacin, 58) LIP3 or Lithium phosphide, 59) Lithium, 60) Liposome or Liposomal nanomaterial, 61) Lomefoxacin,, 62) MG@P NPs, 63) MnP or Manganese peroxidase, 64) MnTTP-HSAs, 65) HSA-wrapped metal-porphyrin complex, 66) albumin, 67) MnWOx, 68) MnWOx-PEG, 69), PEG, 70) bimetallic oxide, 71) Mn (III)-HFs, 72) managense, hemoporfin, 73) Nanoroads, 74) Noble metal nanomaterial, 75) OI NP or oxygen indyocyanine, 76) Phthalocyanines, 77) PIO or Pioglitazone, 78) Polymeric nanomaterial, 79) Porphyrin, 80) Pt-doped TiO₂, 81) R837, 82) Rose Bengal, 83) Sparfloxacin,, 84) TAPP or 5,10,15,20-tetrakis (4-aminophenyl) porphyrin, 85) TiO₂ or titanium dioxide nanomaterial, 86) TCPP, isomer, or Tris(1-chloro-2-propyl) phosphate 87) TPI or Thermoplastic Polyimide or thermoplastic polymer, 88) TPZ or Tirapazamine, 89) Transition metal oxide, 90) nanoparticle or Janus nanoparticle, and 91) Xanthones.

13. Nanoparticle for use in a method according to any of the claims **1** to **12,** wherein C, D, C_{A1} and/or C_{A2} is/are radio-sensitizer(s), selected in the group consisting of: : 1) AMG102, 2) AQ4N, 3) Apaziquone (E09), 4) Bromodeoxyuridine, 5) Carbogen, 6) Cetuximab, 7) Chemotherapeutic drug or compound, 8) Chlorpromazine, 9) C-reactive peptide, 10) Curcumin, 11) Diamide, 12) Diethylmaeate,, 13) Dihydroartemisinin, 14) Docetaxel, 15) ECI301, 16) Etanidazole, 17) Fludarabine, 18) 5-Fluorouracil, 19) Fluorodeoxyuridine, 20) Gadolynium, 21) Gemcitabine, 22) HER-3 ADC, 23) HSP, 24) Hydrogen peroxide, 25) Hydroxyurea, 26) Hyperbaric oxygen, 27) Hyperthermia, 28) Hypoxic cell cytotoxic agent, 29) Irinotecan, 30) lanthanide-doped radiosensitizer-based metal-phenolic network, 31) Lidocaine, 32) Lododeoxyuridine, 33) Metronidazole, 34) misonidazole, 35) etanidazole, 36) nimorazole, 37) N-Ethylmalemide, 38) malmeide, 39) ethylmalmeide, 40) Nanomaterial such as those consisting of or composed of at least partly or fully gold , silver, bismuth, gadolinium, polysiloxane matrix and gadolinium chelates, hafnium, Tantalum, Zinc, Gadolinium, Germanium, Chromium, Praseodymium, Silicon, iron, platinum, cobalt, manganese, magnesium, iron, Titanium, carbon nanotube, quantum dot, nanoroad, Triflate, or metal oxide, 41) Nelfinavir, 42) Nicotinamide, 43) Nimotuzumab, 44) RNA, or miRNA, or miR-201, or miR-205, or miR-144-5p, or miR-146a-5p, or miR-150, or miR-99a, or miR-139-5p, or miR-320a, 45) Membrane active agent, 46) Mitomycin-C or Mitomycin, 47) Motexafin, 48) NBTXR3, 49) Oligonucleotide, 50) Paclitaxel, 51) Papaverine or Papaverine hydrochloride, 52) Paraxonase-2, 53) Pocaine, 54) Porfiromycin (POR), 55) Protein, 56) Peptide, 57) Radiosensitizing nucleosides or compounds, 58) Resveratrol, 59) RRx-001, 60) SiRNa, 61) Suppressors of sulfhydral groups, 62) SYM004, 63) Texaphyrins, 64) TH-302, and 65) Tirapazamine.
